# EUROPEAN PATENT SPECIFICATION

(11) **EP 0 856 058 B1**
(45) Date of publication and mention of the grant of the patent: **26.07.2006**
(21) Application number: 96929433.9
(22) Date of filing: 06.09.1996
(51) Int. Cl.: C12N 15/54, C12N 9/12, C12Q 1/48, C12Q 1/68, G01N 33/50, C12N 15/11, C07K 16/40, A61K 48/00, C07K 14/39

(54) **CELL-CYCLE CHECKPOINT GENES**
ZELLZIKLUSKONTROLLPUNKT-SPEZIFISCHE GENE
GENES DE POINT DE CONTROLE A CYCLE CELLULAIRE

(30) Priority: 06.09.1995 GB 9518220
(43) Date of publication of application: 05.08.1998
(73) Proprietor: ICOS Corporation, Bothell WA 98201 (US)
(72) Inventor: CARR, Antony, Michael, Falmer, Brighton BN1 9RR (GB)
(74) Representative: Richardson, Kate
(86) International application number: PCT/GB1996/002197
(87) International publication number: WO 1997/009433

(56) References cited:
- WO-A-97/18323
- PROC. NATL. ACAD. USA, vol. 93, April 1996, pages 2850-2855, XP002023632 CIMPRICH ET AL.: "cDNA cloning and gene mapping of a candidate human cell cycle checkpoint protein"
- DATABASE EMBL Entry HSAAADPCE, Accession number Z21145, 5 April 1995 MRC HUMAN GENOMIC MAPPING PROJECT RESOURCE CENTRE CLINICAL RESEARCH CENTRE: "The UK-HGMP cDNA program" XP002023639 & The UK-HGMP cDNA program. Unpublished.
- DATABASE EMBL Entry HSAAADPDG, Accession number Z21146, 5 April 1995 MRC HUMAN GENOMIC MAPPING PROJECT RESOURCE CENTRE CLINICAL RESEARCH CENTRE: "The UK-HGMP cDNA program" XP002023640 & The UK-HGMP cDNA program. Unpublished.
- GENE, vol. 119, 1992, pages 83-89, XP002023633 SEATON ET AL.: "Isolation and characterization of the Schizosaccharomyces pombe rad3 gene, involved in the DNA damage and DNA synthesis checkpoints" cited in the application
- THE EMBO JOURNAL, vol. 15, no. 23, 2 December 1996, pages 6641-6651, XP002023634 BENTLEY ET AL.: "The Schizosaccharomyces pombe rad3 checkpoint gene"
- GENES & DEVELOPMENT, vol. 10, no. 19, 1 October 1996, pages 2423-2437, XP000616408 KEEGAN ET AL.: "The Atr and Atm protein kinases associate with different sites along meiotically pairing chromosomes"
- THE JOURNAL OF BIOLOGICAL CHEMISTRY, vol. 269, no. 7, 18 February 1994, pages 5241-5248, XP002023636 VLAHOS ET AL.: "A specific inhibitor of Phospatidylinositol 3-Kinase, 2-(4-Morpholinyl)-8-phenyl-4H-1-benzopyran -4-one (LY294002)"
- PROC. NATL. ACAD. SCI. USA, vol. 89, June 1992, pages 44952-4956, XP002023637 JIMENEZ ET AL.: "The rad3+ gene of Schizosaccharomyces pombe is involved in multiple checkpoint functions and in DNA repair" cited in the application
- PROC. NATL. ACAD. SCI. USA, vol. 88, December 1991, pages 10686-10690, XP002023638 VASAVADA ET AL.: "A contingent replication assay for the detection of protein-protein interactions in animal cells"

## Description

The present invention relates to a class of checkpoint genes which control progression through the cell cycle in eukaryotic cells.

### Background to the invention.

Control of the cell cycle is fundamental to the growth and maintenance of eukaryotic organisms, from yeasts to mammals. Eukaryotic cells have evolved control pathways, termed "checkpoints" which ensure that individual steps of the cell cycle are completed before the next step occurs. In response to DNA damage, cell survival is increased both by direct DNA repair mechanisms and by delaying progression through the cell cycle. Depending on the position of the cell within the cycle at the time of irradiation, DNA damage in mammalian cells can prevent (a) passage from G1 into S phase, (b) progression through S phase or (c) passage from G2 into mitosis. Such checkpoints are thought to prevent deleterious events such as replication of damaged DNA and the segregation of fragmented chromosomes during mitosis (Hartwell and Kastan, 1994).

The *rad3* gene of *Schizosaccharomyces pombe* is required for the checkpoints that respond to DNA damage and replication blocks. Rad3 is a member of the lipid kinase subclass of kinases which possess regions having sequence homology to the lipid kinase domain of the p110 subunit of phosphatidylinositol-3 kinase (PI-3 kinase). This subclass also includes the ATM protein defective in ataxia-telangiectasia patients. Cells from ataxia telangiectasia patients (AT cells) have lost the delay to S phase following irradiation and are said to display radio resistant DNA synthesis (Painter and Young, 1989). AT cells irradiated in S phase accumulate in G2 with lethal damage, presumably as a consequence of attempting to replicate damaged DNA. AT cells irradiated during G2 display a different phenotype: they do not arrest mitosis after DNA damage, and progress through mitosis with damaged DNA (Beamish and Lavin, 1994). Mutations at the A-T locus, to which the *ATM* gene has been mapped, thus result in disruption of several checkpoints required for an appropriate response to ionising radiation. Other members of this lipid kinase subclass include: Tellp (Greenwell *et al*. 1985), a gene involved in maintaining proper telomere length in *Saccharomyces cerevisiae;* Esrlp: Meclp and the product of the *Drosophila melanogaster mei-41* checkpoint gene (Hari *et al.* 1995).

### Disclosure of the invention.

We have analyzed the *S*. *pombe rad3* gene and found that it has a full length amino acid sequence of 2386 amino acids, not the 1070 amino acids described by Seaton *et al.* 1992. We have determined that this is the direct homologue of *S. cerevisiae* Esrlp, and that it shares the same overall structure as the ATM gene. The C-terminal region of the *rad3* protein contains a lipid kinase domain, which is required for Rad3 function. We have shown that Rad3 is capable of self association. We have also identified a protein kinase activity associated with Rad3.

Further, we have found a human homologue to *rad3.* This gene, which we have named ATR (ataxia and rad related), displays significantly higher homology to *rad3* than it does to the ATM gene.

The human ATR cDNA sequence is set out as SEQ ID NO 1. The amino acid sequence of the ORF from nucleotides 80 and 8011 is set out as SEQ ID NO 2.

The DNA sequence of the open reading frame (ORF) of *rad3* is shown as SEQ ID NO 3. The 2386 amino acid translation of the gene (nucleotides 585 to 7742 of SEQ ID NO 3) is shown as SEQ ID NO 4.

According to the present invention, there is provided a purified and isolated polynucleotide encoding a human rad3 polypeptide (ATR) having lipid kinase activity, said polynucleotide selected from the group consisting of (a) the polynucleotide set out in SEQ ID NO: 1; and (b) a polynucleotide which selectively hybridises to the complement of the polynucleotide of (a) under medium to high stringency conditions.

According to a further aspect of the present invention, there is provided a purified and isolated *S. pombe* rad3 polypeptide homologue (ATR) comprising the amino acid sequence set out in SEQ ID NO: 4, as well as a polynucleotide encoding said polypeptide.

According to this aspect of the present invention, there is further provided a purified and isolated polynucleotide encoding an *S. pombe* rad3 polypeptide homologue (ATR) having lipid kinase activity, said polynucleotide selected from the group consisting of: (a) the polynucleotide set out in SEQ ID NO: 3; and (b) a polynucleotide which selectively hybridises to the complement of the polynucleotide of (a) under medium to high stringency conditions.

According to the present invention, there is also provided a purified and isolated human or *S. pombe* rad3 polypeptide homologue (ATR) encoded by a polynucleotide of the invention, a vector comprising a polynucleotide of the invention, a host cell transformed or transfected with said vector, and an antibody product capable of selectively binding to a polypeptide of the invention.

Further provided are detecting methods and screening assays, as defined in the appended claims.

The ATR protein of SEQ ID NO 2 is disclosed herein, as well as homologues thereof, polypeptide fragments thereof, and antibodies capable of binding the ATR protein or polypeptide fragments thereof. ATR proteins, homologues and fragments thereof are referred to below as the disclosed polypeptides.

Also disclosed is a polynucleotide in substantially isolated form capable of hybridising selectively to SEQ ID NO 1 or to the complement (ie, opposite strand) thereof. Further disclosed are polynucleotides encoding the disclosed polypeptides.

Such polynucleotides will be referred to as the disclosed polynucleotides. The disclosed polynucleotides includes DNA of Seq.ID Nos 1 and fragments thereof capable of selectively hybridising to this gene.

In a further aspect, recombinant vectors are disclosed carrying a disclosed polynucleotide the invention, including expression vectors, and methods of growing such vectors in a suitable host cell, for example under conditions in which expression of a protein or encoded by a disclosed sequence occurs.

In an additional aspect, kits are disclosed comprising the disclosed polynucleotides, polypeptides or antibodies and methods of using such kits in diagnosing the presence of absence of ATR and its homologues, or variants thereof, including deleterious ATR mutants.

Further disclosed are assays methods for screening candidate substances for use as compounds for inhibiting or activating ATR activity, or the activity of mutated forms of ATR which are deficient in checkpoint activity. Also disclosed are assay methods for screening candidate substances for use as compounds for inhibiting interactions between ATR and other compounds that interact with ATR, including ATR itself.

In a related aspect, a polynucleotide sequence of Seq. ID No. 3 is disclosed in substantially isolated form, and the protein of Seq. ID No. 4 in substantially isolated form, and novel fragments and variants thereof.

### Detailed description of the invention.

### A. Polynucleotides.

Polynucleotides disclosed herein may comprise DNA or RNA. They may also be polynucleotides which include within them synthetic or modified nucleotides. A number-of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or lifespan of the polynucleotides.

Polynucleotides capable of selectively hybridizing to the DNA of Seq. ID No. 1 will be generally at least 70%, preferably at least 80 or 90% and more preferably at least 95% homologous to the corresponding DNA of Seq. ID No. 1 over a region of at least 20, preferably at least 25 or 30, for instance at least 40, 60 or 100 or more contiguous nucleotides.

It is to be understood that skilled persons may, using routine techniques, make nueleotide substitutions that do not affect the polypeptide sequence encoded by the disclosed polynucleotides to reflect the codon usage of any particular host organism in which the disclosed polypeptides are to be expressed.

Any combination of the above mentioned degrees of homology and minimum sizes may be used to define the disclosed polynucleotides, with the more stringent combinations (i.e. higher homology over longer lengths) being preferred. Thus for example a polynucleotide which is at least 80% homologous over 25, preferably 30 nucleotides is disclosed, as is a polynucleotide which is at least 90% homologous over 40 nucleotides.

Polynucleotides disclosed herein may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term disclosed polynucleotides, as used herein.

Polynucleotides such as a DNA polynucleotide and primers may be produced recombinantly, synthetically, or by any means available to those of skill in the art. They may also be cloned by standard techniques.

In general, primers will be produced by synthetic means, involving a step wise manufacture of the desired nucleic acid sequence one nucleatide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15-30 nucleotides) to a region of the ATR gene which it is desired to clone, bringing the primers into contact with mRNA or cDNA obtained from a human cell (e.g. a dividing cell such as a peripheral blood leukocyte), performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Such techniques may be used to obtain all or part of the ATR sequence described herein. Genomic clones containing the ATR gene and its introns and promoter regions may also be obtained in an analogous manner, starting with genomic DNA from a human cell, e.g. a liver cell.

Although in general the techniques mentioned herein are well known in the art, reference may be made in particular to Sambrook *et al.* (Molecular Cloning: A Laboratory Manual, 1989).

Polynucleotides which are not 100% homologous to the sequences disclosed herein but fall within the scope of the present disclosure can be obtained in a number of ways.

Other human allelic variants of the ATR sequence described herein may be obtained for example by probing genomic DNA libraries made from a range of individuals, for example individuals from different populations.

In addition, other animal, particularly mammalian (e.g. mice, rats or rabbits), more particularly primate, homologues of ATR may be obtained and such homologues and fragments thereof in general will be capable of selectively hybridizing to Seq. ID No. 1. Such sequences may be obtained by probing cDNA libraries made from dividing cells or tissues or genomic DNA libraries from other animal species, and probing such libraries with probes comprising all or part of Seq. ID. 1 under conditions of medium to high stringency (for example 0.03M sodium chloride and 0.03M sodium citrate at from about 50°C to about 60°C).

Allelic variants and species homologues may also be obtained using degenerate PCR which will use primers designed to target sequences within the variants and homologues encoding conserved amino acid sequences. Conserved sequences can be predicted from aligning the ATR amino acid sequence with that of *rad3.* The primers will contain one or more degenerate positions and will be used at stringency conditions lower than those used for cloning sequences with single sequence primers against known sequences.

Alternatively, such polynucleotides may be obtained by site directed mutagenesis of the ATR sequences or allelic variants thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides. Further changes may be desirable to represent particular coding changes found in ATR which give rise to mutant ATR genes which have lost the checkpoint function. Probes based on such changes can be used as diagnostic probes to detect such ATR mutants.

Also disclosed are double stranded polynucleotides comprising a disclosed polynucleotide and its complement.

Polynucleotides or primers disclosed herein may carry a revealing label. Suitable labels include radioisotopes such as ³²P or ³⁵S, enzyme labels, or other protein labels such as biotin. Such labels may be added to polynucleotides or primers disclosed herein and may be detected using techniques known per se.

Polynucleotides or primers disclosed herein or fragments thereof labelled or unlabelled may be used by a person skilled in the art in nucleic acid-based tests for detecting or sequencing ATR in the human or animal body.

Such tests for detecting generally comprise bringing a human or animal body sample containing DNA or RNA into contact with a probe comprising a polynucleotide or primer disclosed herein under hybridizing conditions and detecting any duplex formed between the probe and nucleic acid in the sample. Such detection may be achieved using techniques such as PCR or by immobilizing the probe ow a solid support, removing nucleic acid in the sample which is not hybridized to the probe, and then detecting nucleic acid which has hybridized to the probe. Alternatively, the sample nucleic acid may be immobilized on a solid support, and the amount of probe bound to such a support can be detected. Suitable assay methods of this any other formats can be found in for example WO89/03891 and WO90/13667.

Tests for sequencing ATR include bringing a human or animal body sample containing target DNA or RNA into contact with a probe comprising a polynucleotide or primer disclosed herein under hybridizing conditions and determining the sequence by, for example the Sanger dideoxy chain termination method (see Sambrook *et al.*)*.*

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and d7TP. a buffer and ATP. Dideoxynucieotides are used for selective termination.

Tests for detecting or sequencing ATR in the human or animal body may be used to determine ATR sequences within cells in individuals who have, or are suspected to have, an altered ATR gene sequence, for example within cancer cells including leukaemic cells and solid tumours such as breast, ovary, lung, colon, pancreas, testes, liver, brain, muscle and bone tumours.

In addition, the discovery of ATR will allow the role of this gene in hereditary diseases to be investigated, in a manner analogous to the ATM gene. In general, this will involve establishing the status of ATR (e.g using PCR sequence analysis) in cells derived from patients with diseases that may be connected with damage to replicating cells, e.g. familial predisposition to cancer, chromosome breakage or instability phenotype or repair-damage sensitivity phenotype.

The probes disclosed herein may conveniently be packaged in the form of a test kit in a suitable container. In such kits the probe may be bound to a solid support where the assay format for which the kit is designed requires such binding. The kit may also contain suitable reagents for treating the sample to be probed, hybridizing the probe to nucleic acid in the sample, control reagents, instructions, and the like.

Polynucleotides encoding the disclosed polypeptides are described below. Because such polynucleotides win be useful as sequences for recombinant production of the disclosed polypeptides, it is not necessary for them to be selectively hybridizable to the sequence Seq. ID No. 1, although this will generally be desirable. Otherwise, such polynucleotides may be labelled, used, and made as described above if desired. The disclosed polypeptides are described below.

Particularly preferred polynucleotides are those derived from the lipid kinase domain of ATR. its allelic variants and species homologues. The lipid kinase domain is represented by nucleotides 7054 to 8011 of Seq. ID. 1. Polynucleotides which comprise this domain are particularly preferred. The term "lipid kinase domain" refers to a domain which has homology to other known lipid kinases, in particular the p110 subunit of PI-3 kinase, as determined by sequence alignments.

Other preferred polynucleotides are those which comprise nucleotides encoding amino acids 181 to 302 of Seq. ID No. 2 (nucleotides 620 to 985 of Seq. ID No. 1), which is believed to be a leucine zipper region, a putative site of protein-protein interaction, and amino acids 1358 to 1366 (nucleotides 4151 to 4177), which is also conserved.

In an additional aspect, polynucleotides include those of Seq. ID No. 3 and fragments thereof capable of selectively hybridizing to this sequence other than the fragment consisting of nucleotides 2482 to 6599 in which the following changes have been made: Deletion of residues 2499, 2501. 2507 & 2509; insertion of C between 5918/5919.

Particularly preferred fragments include those comprising residues 6826 to 7334 (the lipid kinase domain) and the leucine zipper regions 1476 to 1625 and 2310 to 2357. Additionally, the fragment comprising the conserved region 3891 to 3917 is preferred. Such polypeptides and fragments may be made and used as described above.

### B. Polypeptides.

Polypeptides disclosed herein include polypeptides in substantially isolated form which comprise the sequence set out in Seq ID No. 2.

Polypeptides further include variants of such sequences, including naturally occurring allelic variants and synthetic variants which are substantially homologous to said polypeptides. In this context, substantial homology is regarded as a sequence which has at least 70%, e.g. 80% or 90 % amino acid homology (identity) over 30 amino acids with the sequence of Seq. ID No. 2 except for the lipid kinase domain and C-terminal portion (residues 2326 to 2644) where substantial homology is regarded as at least 80 % homology, preferably 90% homology (identiry) over 50 amino acids.

Polypeptides also include other those encoding ATR homologues from other species including animals such as mammals (e.g. mice, rats or rabbits), especially primates, and variants thereof as defined above.

Polypeptides also include fragments of the above mentioned full length polypeptides and variants thereof, including fragments of the sequence set out in Seq. ID No.

Preferred fragments include those which include an epitope, especially an ephope. Suitable fragments will be at least about 5, e.g. 10, 12, 15 or 20 amino acids in size, Polypeptide fragments of the ATR protein and allelic and species variants thereof may contain one or more (e.g. 2, 3, 5, or 10) substitutions, deletions or insertions, including conserved substitutions.

Conserved substitutions may be made according to the following table indicates conservative substitutions, where amino acids on the same block in the second column and preferably in the same line in the third column may be substituted for each other:

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |
| OTHER | | N Q D E |

Variants of the polypeptides disclosed herein may also comprise polypeptides wherein one or more of the specified (i.e., naturally encoded) amino acids is deleted or replaced or wherein one or more nonspecified amino acids are added: (1) without loss of the kinase activity specific to the disclosed polypeptides; or (2) with disablement of the kinase activity specific to the disclosed polypeptides; or (3) with disablement of the ability to interact with members or regulators of the cell cycle checkpoint pathway.

Epitopes may be determined either by techniques such as peptide scanning techniques as described by Geysen *et* a*l.* Mol. Immunol., 23; 709-715 (1986).

Polypeptides disclosed herein may be in a substantially isolated form. It will be understood that the polypeptide may be mixed with carriers or diluents which will not interfere with the intended purpose of the polypeptide and still be regarded as substantially isolated. A polypeptide disclosed herein may also be in a substantially purified form, in which case it will generally comprise the polypeptide in a preparation in which more than 90%, e.g. 95%, 98% or 99% of the polypeptide in the preparation is a polypeptide disclosed herein. Polypeptides disclosed herein may be modified for example by the addition of Histidine residues to assist their purification or by the addition of a signal sequence to promote their secretion from a cell.

A polypeptide disclosed herein may be labelled with a revealing label. The revealing label may be any suitable label which allows the polypeptide to be detected. Suitable labels include radioisotopes, e.g. ¹²⁵I, enzymes, antibodies, polynucleotides and linkers such as biotin. Labelled polypeptides disclosed herein may be used in diagnostic procedures such as immunoassays in order to determine the amount of the polypeptide in a sample. Polypeptides or labelled polypeptides disclosed herein may also be used in serological or cell mediated immune assays for the detection of immune reactivity to said polypeptides in animals and humans using standard protocols.

A polypeptide or labelled polypeptide disclosed herein or fragment thereof may also be fixed to a solid phase, for example the surface of an immunoassay well or dipstick.

Such labelled, and/or immobilized polypeptides may be packaged into kits in a suitable container along with suitable reagents, controls, instructions and the like.

Such polypeptides and kits may be used in methods of detection of antibodies to the ATR protein or its allelic or species variants by immunoassay.

Immunoassay methods are well known in the art and will generally comprise:
(a) providing a polypeptide comprising an epitope bindable by an antibody against said protein:
(b) incubating a biological sample with said polypeptide under conditions which allow for the formation of an antibody-antigen complex; and
(c) determining whether antibody-antigen complex comprising said polypeptide is formed.

Polypeptides disclosed herein may be made by synthetic means (e.g. as described by Geysen *et al.*) or recombinantly, as described below.

Particularly preferred polypeptides include those spanning or within the lipid kinase domain, namely from amino acids 2326 to 2644 of Seq. ID. 2. or sequences substantially homologous thereto. Fragments as defined above from this region are particularly preferred. The polypeptides and fragments thereof may contain amino acid alterations as defined above, including substitutions at one or more of positions 2475, 2480 and 2494, which correspond to the positions of the *rad3* substitutions described in the examples below. Preferred substitutions include D2475A, N2480K and D2494E.

Polypeptides disclosed herein may be used in *in vitro* or *in vivo* cell culture systems to study the role of ATR as a checkpoint gene. For example, truncated or modified (e.g. modified in the lipid kinase domain) ATRs may be introduced into a cell to disrupt the normal checkpoint functions which occur in the cell.

The polypeptides disclosed herein may be introduced into the cell by *in situ* expression of the polypeptide from a recombinant expression vector (see below). The expression vector optionally carries an inducible promoter to control the expression of the polypeptide.

The use of mammalian host cells is expected to provide for such post-translational modifications (e.g., myristolation, glycosylation, truncation, lapidation and tyrosine, serine or threonine phosphorylation) as may be needed to confer optimal biological activity on recombinant expression products.

Such cell culture systems in which the disclosed polypeptides are expressed may be used in assay systems to identify candidate substances which interfere or enhance checkpoint functions in the cell (see below).

In an additional aspect, polypeptides disclosed herein include the protein of Seq. ID No. 4 and fragments thereof from the region other than the fragment consisting of amino acids 713 to 1778. Particularly preferred fragments include those comprising residues 2082 to 2386 (the lipid kinase domain) and the leucine zipper regions 298 to 347 and 576 to 591. Additionally, the fragment comprising the conserved region 1103 to 1111 is preferred. Such polypeptides and fragments may be made and used as described above.

Also disclosed are polypeptides substantially homologous to the protein of Seq. ID No. 4, and fragments thereof. In this context, substantial homology is regarded as a sequence which has at least 70%. e.g. 80% or 90% amino acid homology (identity) over 30 amino acids with the sequence of Seq. ID No. 4 except for the lipid kinase domain and C-terminal portion (residues 2082 to 2386) where substantial homology is regarded as at least 80%, preferably at least 90 % homology (identity) over 50 amino acids.

### C. Vectors.

The disclosed polynucleotides can be incorporated into a recombinant replicable vector. The vector may be used to replicate the nucleic acid in a compatible host cell. Thus in a further embodiment, there is disclosed a method of making the disclosed polynucleotides by introducing a polynucleotide disclosed herein into a replicable vector, introducing the vector into a compatible host cell, and growing the host cell under conditions which bring about replication of the vector. The vector may be recovered from the host cell. Suitable host cells are described below in connection with expression vectors.

### D. Expression Vectors.

Preferably, a disclosed polynucleotide in a vector is operably linked to a control sequence which is capable of providing for the expression of the coding sequence by the host cell, i.e. the vector is an expression vector.

The term "operably linked" refers to a juxtaposition wherein the components described are in a relationship permitting them to function in their intended manner. A control sequence "operably linked" to a coding sequence is ligated in such a way that expression of the coding sequence is achieved under condition compatible with the control sequences.

Such vectors may be transformed into a suitable host cell as described above to provide for expression of a polypeptide disclosed herein. Thus, in a further aspect is disclosed a process for preparing the disclosed polypeptides, which process comprises cultivating a host cell transformed or transfected with an expression vector as described above under conditions to provide for expression by the vector of a coding sequence encoding the polypeptides, and recovering the expressed polypeptides.

The vectors may be for example, plasmid, virus or phage vectors provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neoaiycin resistance gene for a mammalian vector. Vectors may be used *in vitro,* for example for the production of RNA or used to transfect or transform a host cell. The vector may also be adapted to be used *in vivo,* for example in a method of gene therapy.

In a further embodiment, host cells are disclosed transformed or transfected with the vectors for the replication and expression of polynucleotides disclosed herein. The cells will be chosen to be compatible with the said vector and may for example be bacterial, yeast, insect or mammalian.

Polynucleotides disclosed herein may also be inserted into the vectors described above in an antisense orientation in order to provide for the production of antisense RNA. Antisense RNA or other antisense polynucleotides may also be produced by synthetic means. Such antisense polynucleotides may be used in a method of controlling the levels of ATR or its variants, or species homologues.

Promoters and other expression regulation signals may be selected to be compatible with the host cell for which the expression vector is designed. For example, yeast promoters include S. cerevisiae GAL4 and ADH promoters, S. pombe nmtl and adh promoter. Mammalian promoters include the metallothionein promoter which is can be included in response to heavy metals such as cadmium. Viral promoters such as the SV40 large T antigen promoter or adenovirus promoters may also be used. All these promoters are readily available in the art.

### E. Antibodies.

Also disclosed are monoclonal or polyclonal antibodies to the disclosed polypeptides or fragments thereof. A process for the production of the disclosed monoclonal or polyclonal antibodies to the disclosed polypeptides is also disclosed. Monoclonal antibodies may be prepared by conventional hybridoma technology using the polypeptides disclosed herein or peptide fragments thereof, as immunogens. Polyclonal antibodies may also be prepared by conventional means which comprise inoculating a host animal, for example a rat or a rabbit, with a disclosed polypeptide or peptide fragment thereof and recovering, immune serum.

In order that such antibodies may be made, polypeptides disclosed herein or fragments thereof are disclosed haptenised to another polypeptide for use as immunogens in animals or humans.

Preferred antibodies will be capable of selectively binding the human ATR protein, that is with an affinity at least 10 fold, preferably at least 100 fold that of the *rad3* protein. Such antibodies can be obtained by routine experimentation, e.g. selecting regions of ATR. protein with sequences different from the corresponding regions of *rad3,* making peptides comprising such sequences and using such peptides as immunogens. Following production of antibodies the binding of said antibodies may be determined. Preferred antibodies include those capable of selectively binding the lipid kinase domain (as defined above) of the human ATR protein. In addition, antibodies which are capable of binding the human and yeast (S. pombe) lipid kinase domains with similar affinity, but not to the domains of the ATM family of proteins are also disclosed. Such antibodies may be raised against peptides from the lipid kinase domains which correspond to regions found to be identical, or substantially identical, in the yeast and human genes.

For the purposes herein, the term "antibody", unless specified to the contrary, includes fragments of whole antibodies which retain their binding activity for a tumour target antigen. Such fragments include Fv, F(ab') and F(ab')₂ fragments, as well as single chain antibodies. Furthermore, the antibodies and fragments thereof may be humanised antibodies, eg. as described in EP-A-239400.

Antibodies may be used in method of detecting disclosed polypeptides present in biological samples by a method which comprises:
(a) providing an antibody disclosed herein;
(b) incubating a biological sample with said antibody under conditions which allow for the formation of an antibody-antigen complex; and
(c) determining whether antibody-antigen complex comprising said antibody is formed.

Suitable samples include extracts from dividing cells, e.g leukocytes or cancer cells including leukaemic cells and solid tumours such as breast, ovary, lung, colon, pancreas, testes, liver brain, muscle and bone tumours.

Antibodies disclosed herein may be bound to a solid support and/or packaged into kits in a suitable container along with suitable reagents, controls, instructions and the like.

### F. Assays.

Abrogating cell cycle checkpoints is a potential strategy for developing or designing drugs, for anti-cancer therapy, both as a novel treatment as such and as part of a combination therapy to enhance the specific toxicity of current chemotherapeutic agents. For example alkylating agents such as nitrogen mustards are used a chemotherapeutic agents which damage DNA in rapidly dividing cells, leading to cell death. The toxicity of such agents may be lessened by DNA repair and checkpoint mechanisms. Abrogating such mechanisms will thus enhance the effectiveness of therapeutic compounds designed to damage DNA. Abrogation of the ATR checkpoint will be especially useful where tumour cells have lost other checkpoint or damage response genes, since these other genes may be able to complement the loss of ATR function in non tumour cells, leading to an even greater enhancement in the effectiveness of the chemotherapeutic agent.

The lipid kinase activity of ATR is a target for developing anti-cancer compounds, since the results presented in the following examples indicate that the kinase domain is required for ATR function. Thus an assay method is disclosed for screening candidate substances for anti-cancer therapy which comprises:
(a) providing a polypeptide disclosed herein which retains lipid kinase activity and a substrate for said kinase, under conditions and with reagents such that the kinase activity will act upon the substrate;
(b) bringing said polypeptide and substrate into contact with a candidate substance;
(c) measuring the degree of decrease in the kinase activity of the polypeptide: and
(d) selecting a candidate substance which provides a decrease in activity.

The assay may be carried out *in vitro,* for example in the wells of a microtitre dish. Such a format may be readily adapted for automation, allowing large numbers of candidate substances to be screened.

The substrate may be a protein or lipid substrate of natural or synthetic origin upon which the disclosed polypeptide will act. Usually, the disclosed polypeptide will phosphorylate the substrate.

Any suitable format for the assay may be used by those of skill in the art of throughput assays. Typically, the disclosed polypeptide which retains lipid kinase activity will be bound to a solid support in the presence of a substrate and cellular and other components which are usually required for activity. Labelled phosphate and a candidate substance will be added to the mixture simultaneously or sequentially in either order. After a suitable reaction time (usually a few minutes but in any event enough for phosphorylation of the substrate in the absence of candidate substance to occur) the amount of free phosphate is determined, e.g. by precipitation of phosphate. Candidate substances which inhibit kinase activity will inhibit the incorporation of free phosphate into the substrate and thus where free phosphate is found this is indicative of inhibition.

Other assay formats may be used by those skilled in the art.

The candidate substances may be used in an initial screen in batches of for example 10 compounds per reaction, and the compounds of those batches which show inhibition tested individually.

Suitable candidate substances include peptides, especially of from about 5 to 20 amino acids in size, based on the sequence of the kinase domain, or variants of such peptides in which one or more residues have been substituted as described above. Peptides from panels of peptides comprising random sequences or sequences which have been varied consistently to provide a maximally diverse panel of peptides may be used. Further candidate substances include kinase inhibitors which are small molecules such as cyclosporin-like and staurosporin-like compounds, or other compounds commercially available in panels of small moleeule inhibitors.

Candidate substances which show activity in *in vitro* screens such as the above can then be tested in *in vivo* systems, such as yeast or mammalian cells which will be exposed to the inhibitor and tested for checkpoint activity.

We have also shown that Rad3 possesses protein kinase activity. Target substrates of Rad3 protein kinase activity may be identified by incorporating test compounds in assays for kinase activity. Rad3 protein is resuspended in kinase buffer and incubated either in the presence of absence of the test compound (e.g., casein, histone H1, or appropriate substrate peptide). Moles of phosphate transferred by the kinase to the test compound are measured by autoradiography or scintillation counting. Transfer of phosphate to the test compound is indicative that the test compound is a substrate of the kinase.

Agents that modulate Rad3/ATR lipid kinase or Rad 3 protein kinase activity may be identified by incubating a test compound and Rad3/ATR immunopurified from cells naturally expressing Rad3/ATR, with Rad3/ATR obtained from recombinant procaryotic or eukaryotic cells expressing the enzyme, or with purified Rad3/ATR, and then determining the effect of the test compound on Rad3/ATR activity. The activity of the Rad3/ATR lipid kinase or Rad3 protein kinase domains can be measured by determining the moles of ³²P-phosphate transferred by the kinase from gamma-³²-P-ATP to either itself (autophosphorylation) or to an exogenous substrate such as a lipid or protein. The amount of phosphate incorporated into the substrate is measured by scintillation counting or autoradiography. An increase in the moles of phosphate transferred to the substrate in the presence of the test compound compared to the moles of phosphate transferred to the substrate in the absence of the test compound indicates that the test compound is an activator of said kinase activity. Conversely, a decrease in the moles of phosphate transferred to the substrate in presence of the test compound compared to the moles of phosphate transferred to the substrate in the absence of the test compound indicates that the modulator is an inhibitor of said kinase activity.

In a presently preferred assay, a Rad3/ATR antibody linked to agarose beads is incubated with a cell lysate prepared from host cells expressing Rad3/ATR. The beads are washed to remove proteins binding nonspecifically to the beads and the beads are then resuspended in a kinase buffer (such as 25 mM K-HEPES pH 7.7. 50 mM potassium chloride,' 10 mM magnesium chloride. 0.1% Nonidet-P-40, 20% glycerol. 1 mM DTT). The reaction is initiated by the addition of 100 µM gamma-³²P-ATP (4 Ci/mM) and an exogenous substrate such as lipid or peptide, and the reaction is carried out at 30°C for 10 minutes. The activity of the kinase is measured by determining the moles of ³²P-phosphate transferred either to the kinase itself or the added substrate. In a preferred embodiment the host cells lack endogenous Rad3/ATR kinase activity. The selectivity of a compound that modulates the lipid kinase activity of Rad3/ATR can be evaluated by comparing its activity on Rad3/ATR to its activity on, for example, other known phosphatidylinositol-3 (PI-3)-related kinases. The combination of the recombinant Rad3/ATR products disclosed herein with other recombinant PI-3-related kinase products in a series of independent assays provides a system for developing selective modulators of Rad3/ATR kinase activity. Similarly, the selectivity of a compound that modulates the protein kinase activity of Rad3 may be determined with reference to other protein kinases, for example the DNA dependent protein kinase or ATM.

In addition, the demonstration that the *rad* mutant *rad.D2249E* (see Examples) can act as a dominant negative mutant indicates involvement in one or more protein complexes, and such complexes themselves can be targeted for therapeutic intervention. We have shown; for example, that Rad3 can both self associate and associate with ATR. It is therefore likely that *Rad*/ATR function as multimeric molecules. Mutant yeast rad or human ATR genes, or derivatives thereof which also lack *radl*ATR activity may be introduced into cells to act as dominant negative mutants. Thus for example if expression of a dominant negative mutant (e.g. ATR D2475A. N2480K or D2494E) in a tumour cell leads to enhanced radiation sensitivity this indicates that the native ATR is still functioning and thus a target for therapeutic agents.

Interacting proteins including components of multimeric protein complexes involving Rad3 or ATR may be identified by the following assays.

A first assay contemplated is a two-hybrid screen. The two-hybrid system was developed in yeast (Chien *et al.* (1991)) and is based on functional *in vivo* reconstitution of a transcription factor which activates a reporter gene. Specifically, a polymmicotide encoding a protein that interacts with Rad3/ATR is isolated by: transforming or transfecting appropriate host cells with a DNA construct comprising a reporter gene under the control of a promoter regulated by a transcription factor having DNA a binding domain and an activating domain: expressing in the host cells a first hybrid DNA sequence encoding a first fusion of part or all of Rad3/ATR and either the DNA binding domain or the activating domain of the transcription factor: expressing in the host cell a library of second hybrid DNA sequences encoding second fusion of part or all putative Rad3/ATR binding proteins and the DNA binding domain or activating domain of the transcription factor which is not incorporated in the first fusion; detecting binding of an Rad3/ATR interacting protein to Rad3/ATR in a particular host cell by detecting the production of reporter gene product in the host cell; and isolating second hybrid DNA sequences encoding the interacting protein from the particular host cell. Presently preferred for use in the assay are a lera promoter to drive expression of the reporter gene, the *lac*Z reporter gene, a transcription factor comprising the *lexA* DNA binding domain and the GAL4 transactivation domain, and yeast host cells.

Other assays for identifying proteins that interact with Rad3 or ATR may involve immobilising Rad3/ATR or a test protein, detectably labelling the nonimmobilised binding panner, incubating the binding partners together and determining the amount of label bound. Bound label indicates that the test protein interacts with Rad3/ATR.

Another type of assay for identifying Rad3 or ATR interacting proteins involves immobilising Rad3/ATR or a fragment thereof on a solid support coated (or impregnated with) a fluorescent agent, labelling a test protein with a compound capable of exciting the fluorescent agent, contacting the immobilised Rad3/ATR with the labelled test protein, detecting light emission by the fluorescent agent, and identifying interacting proteins as test proteins which result in the emission of light by the fluorescent agent. Alternatively, the putative interacting protein may be immobilised and Rad3/ATR may be labelled in the assay.

Compounds that modulate interaction between Rad3/ATR and other cellular components may be used in methods of treating cancer. For example, if a particular form of cancer results from a mutation in a gene other than ATR such as the p53 gene, an agent which inhibits the transcription or the enzymatic activity of ATR and thus the G₂ cell cycle checkpoint may be used to render cancerous cells more susceptible to chemotherapy or radiation therapy. The therapeutic value of such an agent lies in the fact that current radiation therapy or chemotherapy in most cases does nothing to overcome the ability of the p53 mutant cancerous cell to sense and correct the DNA damage imposed as a result of the treatment. As a result, a cancer cell can simply repair the DNA damage. Modulating agents disclosed herein may therefore be chemotherapy and radiation adjuvants or may be directly active as chemotherapy drugs themselves.

Assays for identifying compounds that modulate interaction of Rad3/ATR with other proteins may involve: transforming or transfecting appropriate host cells with a DNA construct comprising a reporter gene under the control of a promoter regulated by a transcription factor having a DNA-binding domain and an activating domain; expressing in the host cells a first hybrid DNA sequence encoding a first fusion of part or all of Rad3/ATR and the DNA binding domain or the activating domain of the transcription factor; expressing in the host cells a second hybrid DNA sequence encoding part or all of a protein that interacts with Rad3/ATR and the DNA binding domain or activating domain of the transcription factor which is not incorporated in the first fusion; evaluating the effect of a test compound on the interaction between Rad3/ATR and the interacting protein by detecting binding of the interacting protein to Rad3/ATR in a particular host cell by measuring the production of reporter gene product in the host cell in the presence or absence of the test compound: and identifying modulating compounds as those test compounds altering production of the reported gene product in comparison to production of the reporter gene product in the absence of the modulating compound. Presently preferred for use in the assay are a *lexA* promoter to drive expression of the reporter gene, the *lacZ* reporter gene, a transcription factor comprising the *lexA* DNA domain and the GAL4 transactivation domain, and yeast host cells.

Another type of assay for identifying compounds that modulate the interaction between Rad3/ATR and an interacting protein involves immobilising Rad3/ATR or a natural Rad3/ATR interacting protein, detectably labelling the nonimmobilised binding partner, incubating the binding partners together and determining the effect of a test compound on the amount of label bound wherein a reduction in the label bound in the present of the test compound compared to the amount of label bound in the absence of the test compound indicates that the test agent is an inhibitor of Rad3/ATR interaction with the protein. Conversely, an increase in the bound in the presence of the test compared to the amount label bound in the absence of the compared indicates that the putative modulator is an activator of Rad3/ATR interaction with the protein.

Yet another method contemplated herein for identifying compounds that modulate the binding between Rad3/ATR and an interacting protein involves immobilising Rad3/ATR or a fragment thereof on a solid support coated (or impregnated with) a fluorescent agent labelling the interacting protein with a compound capable of exciting the fluorescent agent, contacting the immobilised Rad3/ATR with the labelled interacting protein in the presence and absence of a test compound, detecting light emission by the fluorescent agent, and identifying modulating compounds as those test compounds that affect the emission of light by the fluorescent agent in comparison to the emission of light by the fluorescent agent in the absence of the test compound. Alternatively, the Rad3/ATR interacting protein may be immobilised and Rad3/ATR may be labelled in the assay.

We have shown that Rad3 interacts with ATR. Therefore the above-mentioned assays may also be used to identify compounds that modulate the interaction between Rad3 and ATR where the interacting protein described in the assay methods is either Rad3 or ATR.

We have also shown that Rad3 can bind to itself, strongly suggesting that ATR can also bind to itself. Therefore the above-mentioned assays may also be used to identify compounds that modulate Rad3-Rad3 interactions and ATR-ATR interactions.

Such compounds could be used therapeutically to disrupt ATR-ATR interactions and increase the sensitivity of tumour cells to chemotherapy and/or radiotherapy. Thus disclosed herein is an assay method for screening candidate substances for anti-cancer therapy which comprises:
(a)
   (i) incubating a polypeptide disclosed herein with another polypeptide disclosed herein, which may be the same as or different to the first polypeptide, under conditions which allow the first polypeptide to bind to the second polypeptide to form a complex;
   (ii) bringing the complex thus formed into contact with a candidate substance:
   or
(a) incubating a polypeptide disclosed herein with another polypeptide disclosed herein, which may be the same as or different to the first polypeptide, under conditions which allow the first polypeptide to bind to the second polypeptide to form a complex and in the presence of a candidate substance;
   and
(b) determining whether the candidate substance inhibits binding of the first polypeptide to the second polypeptide and
(c) selecting a candidate substance which inhibits binding of the first polypeptide to the second polypeptide.

Preferably the first and second polypeptide may be distinguished from each other. For example, the first polypeptide and the second polypeptide may both be ATR, or may both be Rad3, or one may be ATR and one may be Rad3 or derivatives of either ATR or Rad3 which retain binding activity. When both polypeptides are ATR or Rad3, preferably two distinguishable forms of ATR/Rad3 would be used in these assays. They may be distinguished by, for example, labelling either of the polypeptides. Examples of labels include radioactive labels, epitope tags or other polypeptide tags such as glutathione -S-transferase. For example, one form of Rad3 may have one form of epitope tag, and the other form would have a different epitope, tag, allowing them to be distinguished immunologically such that binding of one to the other, can be ascertained quantitively or qualitatively. In a preferred method, the first polypeptide may be immobilised, for example to agarose beads or a solid support, and the second polypeptide may be in free solution. Binding is then determined using methods described above and well-known to skilled persons.

Also comprehended herein are antibody products (e.g., monoclonal and polyclonal antibodies, single chain antibodies, chimeric antibodies, CDR-grafted antibodies and the like) and other binding proteins (such as those identified in the assays above) which are specific for the Rad3 protein kinase domain or the Rad3/ATR lipid kinase domains. Binding proteins can be developed using isolated natural or recombinant enzymes. The binding proteins are useful, in turn, for purifying recombinant and naturally occurring enzymes and identifying cells producing such enzymes. Assays for the detection and quantification of proteins in cells and in fluids may involve a single antibody substance or multiple antibody substances in a "sandwich" assay format. The binding proteins are also manifestly useful in modulating (i.e., blocking, inhibiting, or stimulating) enzyme/substrate or enzyme/regulator interactions.

Modulators of Rad3/ATR may affect its kinase activity, its localisation in the cell, and/or its imecaction with members of the cell cycle checkpoint pathway. Selective modutaidrs may include, for example, polypeptides or peptides which specifically bind to Rad3/ATR or Rad3/ATR. nucleic acid, and/or other non-peptide compounds (e.g., isolated or synthetic organic molecules) which specifically react with Rad3/ATR or Rad3/ATR nucleic acid Mutant forms of Rad3/ATR which affect the enzymatic activity or cellular localisation of wild-type Rad3/ATR are also contemplated.

Furthermore, combinatorial libraries, peptide and peptide mimetics, defined chemical entities, ligonucleotides, and natural product libraries may be screened for activity as modulators of Rad3/ATR kinase activity and Rad3/ATR interactions in assays such as those described above.

### F. Therapeutic uses

Modulators of Rad3/ATR activity, including inhibitors of their lipid kinase and protein kinase activities, may be used in anti-cancer therapy. In particular, they may be used to increase the susceptibility of cancer cells to chemotherapy and/or radiotherapy by virrue of their ability to disrupt the cell cycle regulatory functions of Rad3/ATR.

Thus, disclosed herein is the use of compounds that modulate Rad3/ATR activity, identified by the screening assays described above, in a method of treatment of cancer. In one embodiment, said compounds are capable of inhibiting rad3/ATR lipid kinase and/or Rad3 protein kinase activity. In another embodiment, said compounds are capable of inhibiting interactions between ATR and itself and/or between ATR and other interacting proteins which may, for example, normally form part of a multimeric protein complex.

It is to be understood that the term "compound" in this context also refers to the candidate substances selected in the above-described assays.

Typically the compounds are formulated for clinical administration by mixing them with a pharmaceutically acceptable carrier or diluent. For example they can be formulated for topical, parenteral, intravenous, intramuscular, subcutaneous, intraocular or transdermal administration. Preferably, the compound is used in an injectable form. Direct injection into the pateient's tumour is advantageous because it makes it possible to concentrate the therapeutic effect at the level of the affected tissues. It may therefore be mixed with any vehicle which is pharmaceutically acceptable for an injectable formulation, preferably for a direct injection at the site to be treated. The pharmaceutically carrier or diluent may be, for example, sterile or isotonic solutions.

The dose of compound used may be adjusted according to various parameters, especially according to the compound used, the age, weight and condition of the patient to be treated, the mode of administration used, pathology of the tumour and the required clinical regimen. As a guide, the amount of compound administered by injection is suitably from 0.01 mg/kg to 30 mg/kg, preferably from 0.1 mg/kg to 10 mg/kg.

The routes of administration and dosages described are intended only as a guide since a skilled practitioner will be able to determine readily the optimum route of administration and dosage for any particular patient and condition.

Compounds to be administered may include polypeptides or nucleic acids. The nucleic acids may encode polypeptides or they may encode antisense constructs that inhibit expression of a cellular gene. Nucleic acids may be administered by, for example, lipofection or by viral vectors. For example, the nucleic acid may form part of a viral vector such as an adenovirus. When viral vectors are used, in general the dose administered is between 10⁴ and 10¹⁴ pfu/ml, preferably 10⁶ to 10¹⁰ pfu/ml. The term pfu ("plaque forming unit") corresponds to the infectivity of a virus solution and is determined by infecting an appropriate cell culture and measuring, generally after 48 hours, the number of plaques of infected cells. The techniques for determining the pfu titre of a viral solution are well documented in the literature.

Any cancer types may be treated by these methods, for example leukaemias, and solid tumours such as breat, ovary, lung, colon, pancreas, testes, liver, brain, muscle and bone tumour. Preferably, the tumour has normal ATR function.

### Description of the Drawings.

### Figure 1

*The relationship between ATR, rad3, mei-41, MEC1, TEL1 and ATM*
A. Overall structures of ATR, Rad3. Mei-41, Mec1p, Tel1p and ATM.
   Legend: open square - Rad3 domain; hatched boxes - kinase domain
B. Dendrogram based on sequence alignments generated by the Clustal method (PAM250) using DNAstar software. *rad3*/*ESR1*/*mei-41*/*ATR* are more closely related to each other than to *ATM* and *TEL1.* Sequences of *rad3* and *ATM* are available in the EMBL database.

The following examples illustrate the invention.

### Example 1

*The rad3 gene of S. pombe* is one of six genes absolutely required for the DNA structure checkpoints in *S. pombe* (Al-Khodairy and Carr, 1992; Al-Khodairy *et al.* 1994). A sequence representing part of the *rad3* gene was reported by Seaton *et al.* (1992). In attempting to clarify the intron/exon structure of this gene we identified sequencing anomalies at both the 5' and 3' ends. We have sequenced the complete gene (see Experimental Procedures) and find that *rad3* is capable of encoding a product of 2386 amino acids. The C-tertninal region contains the consensus sequences typical of a sub-class of kinases known as lipid kinases, the founder member of which is the p110 catalytic subunit of PI3 kinase (Hiles *et al.* 1992).

A truncated *rad3* clone lacking the amino terminus and the kinase region has been reported to complement the *rad3::pR3H1.0* gene disruption mutant of *rad3* (Jimenez *et al.* 1992). This disruption mutant does not remove the potential kinase domain. To clarify the role of this domain, we have created a null mutant by gene replacement. This mutant has amino acids 1477-2271 of *rad3.* including the kinase consensus domain, replaced by ura4⁺. This strain, *rad3.d,* has identical checkpoint defects and radiation/hydroxyurea sensitivides to the *rad3.136* mutant (Nasim and Smith, 1975) and the original *rad3::pR3H1.0* disruption mutant (Jimenez *et al.* 1992: Seaton *et al.* 1992) (data not shown). We have created three separate point mutants in the putative kinase domain of *rad3* and used these in gene replacement experiments to construct strains with defined kinase null mutations. All three strains, *rad3.D2230A, rad3.N2235K* and *rad3.D2249E* have phenotypes identical to the *rad3.d* null mutant (data not shown), suggesting that the kinase activity is required for Rad3 function. In the light of our findings, one interpretation of the results of Seaton *et al.* (1992) and Jimenez *et al.* (1992) is that the partial clone may show intragenic complementation between the plasmid borne truncated gene and a genomic partial deletion which retains kinase function. Such an interpretation would be consistent with Rad3 acting as a dimer or multimer.

When the kinase null allele *rad3.D2249E* was moderately over-expressed in wild type cells under control of a modified *nmt1* promoter (Maundrell, 1990), it caused extreme radiation sensitivity, assayed by UV strip tests, and acted as a dominant negative mutant (data not shown). When the same kinase null construct was expressed at a higher level, it inhibited growth (data not shown). Examination of the cells indicates that division continued very slowly, and at a smaller cell size wild type cells and cells containing empty vector divide at approximately 15 microns, while *rad3* and *rad3.D2249E* over-expressing cells divide at approximately 11.2 microns (data not shown). In *S. pombe,* this usually indicates an advancement of mitosis.

### The human rad3 homolog, ATR

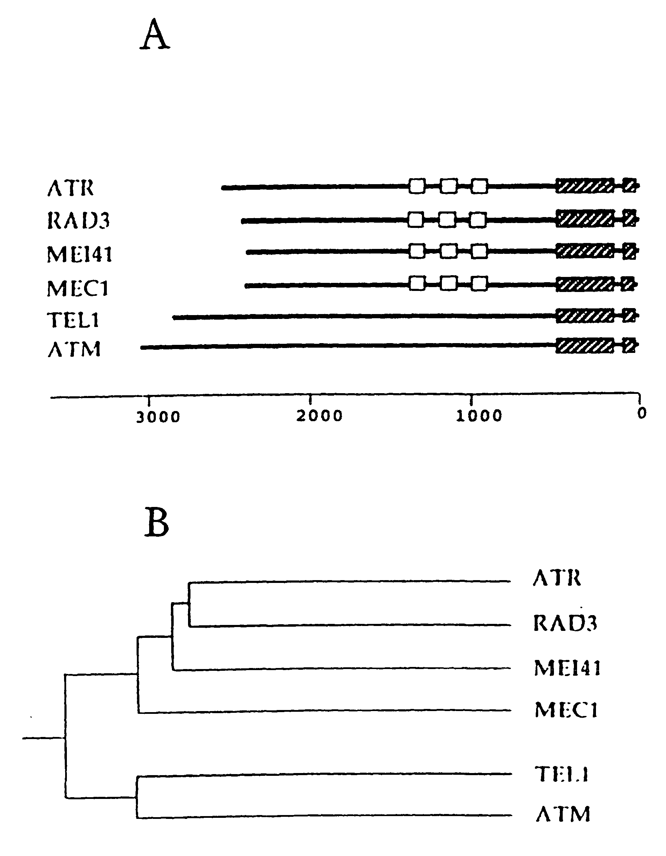

To identify a human form of *rad3,* a combination of methods was applied. Through these approaches, we have cloned the entire coding region of a human gene (see materials and methods), which we have named *ATR* (ataxia and rad related). *ATR* is capable of encoding a 2644 amino acid protein which is much more closely related to the products of *S. pombe rad3, S. cerevisiae ESR1* (Kato and Ogawa, 1994) and *D. melanogaster mei-41* genes (Hari *et al.* 1995) than to the human ATM and *S. cerevisiae* Tell proteins (Savitsky *et al.* 1995; Greenwell *et al.* 1995) and is likely to be the true homolog of *rad3. ESR1* is allelic to the *mec1lsad3* checkpoint mutants (Allen *et al.* 1994; Weinert *et al.* 1994) which have an equivalent phenotype to *rad3. ATR* is less closely related to the human checkpoint gene *ATM,* containing C-terminal putative lipid kinase domain and having a similar overall structure. Sequence alignments demonstrate clearly that the *rad3*/*ESR1(MEC1*/*SAD3)*/*mei-41IATR* genes are more closely related to each other than any are to *ATM* or *TEL1,* and that *ATM* is more homologous to *TEL1* (Figure 1).

The *ATM* gene is expressed in a wide variety of tissues (Savitsky *et. al.* 1995). In *S. cerevisiae, ESR1* shows low level expression in mitotic cells but is rapidly induced during meiosis I (Kato and Ogawa, 1994). Using Northern blot analysis, we have demonstrated that *ATR* is also weakly expressed in many tissues but that it is more highly expressed in testis (data not shown). Given that ATR, Rad3 and Esrlp proteins are more highly related to each other than to ATM, the higher *ATR* expression in testis is consistent with the observation that Esrlp has a role in meiotic recombination (Kato and Ogawa, 1994). Using FISH and PCR analysis, we have mapped *ATR* to chromosome 3q22 - 3q25 (data not shown). This region is not associated with known cancer prone syndromes.

In order to further investigate the possibility that Rad3 acts as a multimer, we have created two separate tagged constructs of full length *rad3* in pREP based inducible vectors. In one, Rad3 is translated with two myc epitope tags at the N terminus, while in the other these are substituted for a triple HA epitope tag. When both constructs are expressed together in wild type cells, it is possible to co-precipitate the HA tagged Rad3 with the myc specific antibody, and the myc tagged Rad3 with the HA specific antibody (data not shown). This demonstrates that, *in vivo,* the Rad3 protein is capable of self association and is fully consistent with the complementation data of Jimenez *et al.* (1992).

Although the *ATR* gene could not complement the phenotype of the *rad3* mutants, we have investigated the ability of ATR to form a protein complex with *S. pombe* Rad3 by expressing both ATR and myc-tagged *S. pombe* Rad3 in the same yeast cells. Using an anti-ATR antibody (which does not precipitate *S. pombe* Rad3, see materials and methods) we are able to co-precipitate the yeast protein. We were also able to precipitate the human ATR protein with myc-specific antibodies that recognise the *S. pombe* Rad3 (data not shown). These data suggest the human and yeast proteins can form a heteromeric-complex, which supports the contention, based on the sequence similarity, of a close functional relationship between these homologues.

### Rad3 proteins have associated kinase activity

Since mutagensis experiments suggest that the kinase activity of the Rad3 proteins *in vivo* appears to be essential for their function, we have investigated this activity further. Using *S. pombe rad3::ura4* cells expressing HA tagged *S. pombe* Rad3, we have been able to detect a significant protein kinase activity which precipitates with HA-specific antibodies only when Rad3 is induced and which is not changed following irradiation (data not shown). This activity, which is specific to Rad3 or co-precipitating kinase, appears to reflect phosphorylation of Rad3 itself, since the major band above 200kD that is phosphorylated can be detected by Western analysis with anti-HA antibody (data not shown). Attempts to identify convenient *in vitro* substrates such as myelin basic protein, RP-A and several purified *S. pombe* checkpoint proteins have so far proved unsuccessful. When the IP *in vitro* kinase assay is performed with cells over-expressing a "kinase-null" D2249E version of Rad3, the associated kinase activity precipitated by HA-specific antibody is significantly reduced (data not shown). There are several possible explanations for this. The measured kinase activity could reflect Rad3 activity directly. In this case the residual activity seen with the kinase dead Rad3 could reflect the fact that it is not unknown for the equivalent D to E mutation in other protein kinases to produce a biologically inert protein with residual *in vitro* biochemical activity. Alternatively the kinase activity which phosphorylates Rad3 may be due to associated proteins, and these may interact less effectively with the D2249E mutant protein.

### Discussion

The checkpoint pathways controlling cell cycle progression following DNA damage or interference in the individual events which comprise the cycle are of considerable importance in maintaining genetic stability and can be considered as pathways which suppress tumorgenesis. Several tumour suppressor genes are intimately involved in subsets of the checkpoint pathways (reviewed in Hartwell and Kastan, 1994), particularly those affecting the transition from G1 into S phase and commitment to the cell cycle. The convergence of the two yeast model systems for checkpoints clearly indicates that the genes involved in these pathways are conserved. Our work extends this conservation to metazoan cells, and clarifies the relationship between *rad3. ESR1 (MEC1*/*SAD3*), *mei-41* and the *ATM* gene.

In this work we demonstrate that the correct sequence of the *rad3* gene places its product in the family of protein/lipid kinases related to ATM. Over-expression of kinase-defective *rad3* mutant in *S. pombe* causes a dominant negative phenotype, which suggests that Rad3 is acting as a member of a protein complex whose integrity is necessary for checkpoint function. This is consistent with the observation that *rad1, rad9, rad17, rad26* and *hus1* deletion mutants all have phenotypes indistinguishable from *rad3.d* (Sheldrick and Carr, 1993). Unexpectedly, unlike the remaining checkpoint *rad* genes, high level over-expression of either wild type or mutant *rad3* alleles inhibits cell growth and causes mitosis to occur at a reduced cell size, indicative of premature entry into mitosis. This "semi wee" phenotype is not observed in the null mutant, and may indicate interference in a second pathway whose function overlaps with that of Rad3 and acts to inhibit mitosis. A candidate for such a pathway is the *ATM*/*TEL1* pathway which has been shown to have some overlapping functions with the *ESRI(MEC1*/*SAD1)* pathway (Morrow et al., 1995).

The structure of ATM is most closely related to the Tellp, which is involved in maintaining telomere length (Greenwell et al., 1995). However, ATM function also appears related to that of the Rad3/Esr1p/mei-41 products. Following the initial discovery of the *ATM* gene and its sequence relationship to the *rad3*/*ESR1* genes and to *TEL1.* it was not clear whether, as in many cases in yeast, the gene had duplicated and diverged in yeast, or whether the two yeast proteins defined conserved sub-families of closely related genes. The significant finding of this work is the identification of a human gene, *ATR,* which is more closely related to *rad3*/*ESR1*/*mei-41.* This defines two structurally distinct checkpoint related subfamilies of protein/lipid kinases that are conserved throughout eukaryotic evolution. Although the proteins in these two subfamilies may have some overlapping functions, they probably control different processes. For example: the *rad3* sub-family in yeast control all the G1 and G2 DNA damage checkpoints in response to both uv and ionising radiation, and the S phase checkpoint which prevents mitosis following inhibition of replication (Al-Khodairy and Carr, 1992; Allen et al., 1994; Weinert et al., 1994). In contrast, A-T cells have abnormal responses to a narrow range of DNA damaging agents including ionising radiation, bleomycin and neocarzinostatin, which produce strand breaks in DNA as a consequence of radical attack. The response to uv and most chemical carcinogens is normal, as is the response to the inhibition of DNA synthesis. It is possible that some or all of the remaining DNA damage checkpoints and the S phase checkpoint are controlled by ATR.

### Experimental procedures

### Strains, plasmids and media

Standard genetic techniques, growth conditions and media for *S. pombe* are described in Gutz *et al.* (1974). *S. pombe* strain sp011 (ura4.D18, leu1.32 ade6.704 h⁻) has been described previously (Murray *et al.* 1992). Plasmid pSUB41 was a gift from S. Subramani (Seaton *et al.* 1992).

### Cloning of S. pombe rad3

A 4.0 kb *Kpn*1 fragment was excised from pSUB41 and sequenced in both directions to obtain the 5' *rad3* sequence. The 3' clone was identified from a genomic library (Barbet *et al.* 1992) by colony hybridisation using a 1 kb 3' probe derived from the published *rad3* sequence, and sequenced in both directions. In this way, the sequence of the entire *rad3* gene was assembled.

### Null and "kinase dead" rad3 mutants

A construct of *rad3,* in which the 794 amino acids between aa 1477 and aa2271 (including the kinase domain) were replaced with a *ura4*+ gene, was created using the methodology described in Barbet *et al.* (1992). A linear fragment of this was used to transform sp011 to uracil prototropy and single copy integration at the *rad3* locus was checked by Southern blotting. To create the site specific kinase null mutations, a C-terminal 3.01 kb *Bam*HI-*Sal*I fragment of *rad3* was mutated with either (A: GTTTTCGCCATGGCGCGCTCCCAAACCCAA, B: TTCATCAAACAATATCTTTTCGCCATGGCG, or C: CAAAAAGACAGTTGAATTCGACATGGATAG) in order to introduce either the D2230A, N2235K or D2249E mutations into the kinase domain. Analogous changes have previously been used in the analysis of PI3 kinase *VPS34* of *S. cerevisiae* (Schu *et al.* 1993). These fragments were then used to transform the *rad3.d* null mutant and gene replacements selected by their ability to grow on FOA containing media (Grimm *et al.* 1988). All strains were checked by Southern blotting. Full length expression constructs of *rad3.D12230A* were created in pREP1 and pREP41 (Maundrell, 1990) by standard subcloning following introduction of an *Nde*I site at the ATG and deletion of three internal *Nde*I sites.

### UV radiation sensitivity strip tests

Expression from REP1 (high) and REP41 (intermediate) was induced by the absence of thiamine for 18 hours prior to plating. Plates were irradiated with a gradient of uv doses down the plate from 0 to 300 Jm⁻² according to the settings on a Stratagene Stratalinker.

### Cloning and expression of ATR

To isolate an appropriate probe for identifying cDNAs corresponding to a human *rad3* homologue, degenerate oligonucleotides were designed against the amino acids LGLGDRH (5' oligo; oDH18) and HVDF[D/N]C (3' oligo; oDH-16) of Rad3/Esr1p. Inosine was incorporated at positions of four-fold degeneracy, and primers were tailed with *Bam*HI (oDH18) and *Eco*RI (oDH16) to facilitate cloning. DNA sequence analysis of the ~100 bp PCR product obtained from amplification of peripheral blood leukocyte cDNA demonstrated significant similarity to *MEC1*/*rad3.* This sequence was used to synthesize a non-degenerate primer (oDH-23; GACGCAGAATTCACCAGTCAAAGAATCAAAGAG) for PCR with an additional degenerate primer (oDH17) designed against the amino acid sequence KFPP[I/V][L/F]Y[Q/E]WF of Rad3/Esr1p. The 174 bp product of this reaction was used directly to screen a macrophage cDNA library. Four positive clones were isolated (the largest approximately 3 kb).

In parallel, database searches with full length *S. pombe rad3* derived from the EMBL database a human cDNA clone, HSAAADPDG, as a potential homologue of *rad3,* if a single frameshift was allowed for in the 233 bp sequence. This 233 bp sequence is contained within a 1.6 kb clone obtained from Dr. N. Affara, Human Molecular Genetics Research Group, Cambridge University, UK. The entire clone (1.6 kb) was sequenced and lies within the cDNA clones identified by degenerate PCR and library screens. To identify the whole gene, RACE PCR experiments were performed on cDNA derived from placental and thymus mRNA using the instructions provided with a Clontech Marathon Kit. Gene specific primers were derived from the cDNA clones. From these experiments, a 8239 bp cDNA sequence was assembled with an internal ORF of 2644 amino acids, a 79 bp 5' noncoding region, a 194 bp 3' noncoding region and a poly A⁺ tail. Parts of the sequence were determined solely by PCR. To avoid errors, clones from a minimum of 3 independent PCR reactions were sequenced in both directions.

The 233 bp sequence corresponds to the sequence of nucleotides 6809 to 7042 (234 nt in total) of Seq. ID No. 1 except for a single base deletion at position 6942. This sequence encodes amino acids 2244 to 2320 of Seq. ID No. 2.

The sequence of the "1.6 kb" insert corresponds to nucleotides 5725 to 7104 (1353 nt) of Seq. ID No. 1, and encodes amino acids 1892 to 2340 of Seq. ID No. 2.

Northern blot hybridisation: a 1.3 kb PCR product was amplified in the presence of³²P-dCTP using primers 279-3 (TGGATGATGACAGCTGTGTC) and 279-6 (TGTAGTCGCTGCTCAATGTC). A nylon membrane containing 2 µg of size-fractionated polyA+ RNA from a variety of human tissue sources (Clontech Laboratories) was probed as recommended by the manufacturer except that the final wash was performed at 55°C rather than 50°C to minimize the possibility of cross-hybridisation to related sequences.

### Mapping ATR.

We mapped the ATR gene to chromosome 3 by a combination of fluorescent in situ hybridisation and polymerase chain reaction (PCR) based assays. FISH analysis using a cDNA clone identified the ATR gene on chromosome 3, at approximately position q22-23. PCR analysis also identified ATR on chromosome 3. Two primers (oATR23: GACGCAGAATTCACCAGTCAAAGAATCAAAGAG and oATR26: TGGTTTCTGAGAACATTCCCTGA) which amplify a 257 bp fragment of the ATR gene were used on DNA derived from human/rodent somatic cell hybrids containing various human chromosome panels available from the NIGMS Human Genetic Mutant Cell Repository (Drwinga *et al.* 1993). PCR with the same primers was used to sub-localise ATR to a specific region on chromosome 3. The templates for these amplifications consisted of DNA samples from patients with truncations along chromosome 3 (Leach *et al.* 1994).

### Immunoprecipitation (IP) and kinase assays with Rad3

The *S. pombe rad3* and human *ATR* genes were cloned into pREP41 expression vector for complementation studies. To tag the proteins, versions of these vectors containing in-frame N terminal tag sequences, either a double myc or a triple HA tag, were used (Griffiths *et al.* 1995). Tagged proteins were expressed by growing in media without thiamine (Maundrell. 1990). Yeast cells lysed in lysis buffer (25 mM Tris.C 1 pH 7.5. 60 mM B-glycerophosphate, 0.1 mM Na₃VO₄, 1% Triton X-100, 50 mM MaCl, 2 mM EDTA. 50 mM NaF, 1 mM phenylmethylsulfonyl fluoride [PMSF], 5 µg/ml leupeptin, 5 µg/ml aprotinin, 1 mM DTT) by the addition of glass beads followed by treatment in a dismembrinator for 2 minutes. For IP's 300µg total protein extract was incubated on ice with the appropriate antibody for 30 min and the immune complexes precipitated by mixing with Protein G beads for a further 30 min at 4°C. For kinase assays, the immune complexes were washed 4 timed with Lysis buffer, once with Kinase Buffer (25 mM Hepes pH7.7; 50 mM KCl; 10 mM MgCl₂; 0.1% NP-40; 2% glycerol; 1 mM DTT), and incubated in Kinase Buffer with 10 µM ATP [50 Ci/mmol]) for 15 minutes at 30°C. The reactions were stopped with 20 ul 2X SDS sample buffer prior to separation on 6% polyacrylamide gels. Rad3 IP's contained several phosphorylated products, including one which comigrated with Rad3 protein itself on Western analysis.

### References

Al-Khodairy, F., and. Carr, A.M. (1992). DNA repair mutants defining G2 checkpoint pathways in *Schizosaccharomyces pombe.* EMBO J. 11, 1343-1350.

Al-Khodairy, F., Fotou, E., Sheldrick, K.S., Griffiths, D.J.F., Lehmann, A.R. and Carr, A.M. (1994). Identification and characterization of new elements involved in checkpoints and feedback controls in fission yeast. Mol. Biol. Cell *5*, 147-160.

Alien, J.B., Zhou, Z., Siede, W., Friedberg, E.C. and Elledge, SJ. (1994) The SAD1/RAD53 protein kinase controls multiple checkpoints and DNA damage-induced transcription in yeast. Genes Dev. *8*. 2416-2428.

Barbet, N.C., Muriel, W.J., and Carr, A.M. (1992) Versatile shuttle vectors and genomic libraries for use with *Schizosaccharomyces pombe.* Gene *114,* 59-66.

Beamish, H. and Lavin, M.F. (1994) Radiosensitivity in ataxia-tetangiectasia: anomalies in radiation-induced cell cycle delay. Int. J. Radiat. Biol. *65*, 175-184.

Carr, A.M. and Hoekstra, M.F. (1995) The Cellular Responses to DNA Damage. Trends in Cell Biology *5*, 32 - 40.

Chien et al., (1991) Proc. Natl. Acad Sci USA *88*, 9578-9582

Deng, C., Zhang, P., Harper, J.W., Elledge, S.J. and Leder, P.J. (1995) Mice lacking p21^{CIP1/WAF1} undergo normal development, but are defective in G1 checkpoint control. Cell, (in press).

Drwinga, H.L., Tojia, L.H., Kim, C.H., Greene, A.E., and Mulovor, R.A. (1993). NIGMS Human/Rodent Somatic Cell Hybrid Mapping Panels 1 and 2. Genomics 16:311-314.

El-Deiry, W.S., Tokino, T., Velculescu, V.E., Levy, D.B., Parson, R, Trent, J.M., Lin. D., Mercer, W.E., Kinzler, K.W. and Vogeistein, B. (1993) WAF1, a potential mediator of p53 tumour suppression. Cell *75*, 817-825

Enoch, T., Carr, A.M. and Nurse, P. (1992). Fission yeast genes involved in coupling mitosis to completion of DNA-replication. Genes Dev. *6*, 2035-2046.

Greenwell, P.W., Kronmal, S.L., Porter, S.E., Gassenhuber, J., Obermaier, B. and Petes, T.D. (1995) TEL1, a gene involved in controlling telomere length in *Saccharomyces cerevisiae,* is homologous to the human ataxia telangiectasia (ATM) gene. Cell *submitted.*

Grimm, C., Kholi, J. Murray, J.M. and Maundrell, K. (1988) Genetic engineering of *Schizosaccharomyces pombe*: a system for gene disruption and replacement using the *ura4* gene as a selectable marker. Mol. Gen. Genet. *215*, 81-86.

Gutz, H., Heslot. H. Leupold, U. and Loprieno, N. (1974). In "Handbook of Genetics", King R. C., Ed., Plenum Press, New York, Vol. 1, 395-446.

Hari, K.L., Santerre. A., Sekelsky, J.J., McKim, K.S., Boyd, J.B. and Hawley. R.S. (1995) The mei-41 gene of Drosophila melanogaster is functionally homologous to the human ataxia telangiec

Harper, J.W., Adami, G., Wei, N., Keyomarsi, K. and Elledge, S.J. (1993) The 21 kD Cdk interacting protein Cip1 is a potent inhibitor of G1 cyclin dependent kinases. Cell 75, 805-816.

Hartwell, L.H., and Kastan, M.B. (1994). Cell cycle control and Cancer. Science *266,* 1821-1828.

Hiles, I.D., Otsu, M., Volinia, S., Fry, M.J., Gout, I., Dhand, R., Panayotou, G., Ruiz-Larrea, F., Thompson, A., Totty, N.F., Hsuan, J.J., Courtneidge, S.A., Parker, P.J. and Waterfield M.D. (1992) Phosphatidylinositol 3-kinase: structure and expression of the 110kd catalytic subunit. Cell *70*, 419-429.

Jimenez, G., Yucel, J., Rowley, R. and Subramani S. (1992) The rad3+ gene of Schizosaccharomyces pombe is involved in multiple checkpoint functions and in DNA repair. Proc Natl. Acad. Sci. USA *87*, 4952-4956

Kato, R. and Ogawa, H. (1994) An essential gene, ESR1, is required for mitotic cell growth, DNA repair and Meiotic recombination in *Saccharomyces cerevisiae.* Nucleic Acids Res. *22*, 3104-3112.

Lamb. J.R, Petit-Frere, C., Broughton, B.C., Lehmann, A.R. and Green, M.H.L. (1989) Inhibition of DNA replication by ionizing radiation is mediated by a trans acting factor. Int. J. Radial. Biol. *56*, 125-130.

Leach, RJ., Chinn, R., Reus, B.E., Hayes, S., Schantz, L., Dubois, B., Overhauser, J., Ballabio, A., Drabkin, H., Lewis, B.T., Mendgen, G., and Naylor, S.L. (1994) Regional Localisation of 188 Sequence Tagged Sites on a Somatic Cell Hybrid Mapping Panel for Human Chromosome 3 Genomics *24*, 549-556

Maundrell, K. (1990). *nmtl* of fission yeast. A highly transcribed gene completely repressed by thiamine. J. Biol. Chem. *265*, 10857-10864.

Morrow, D.M., Tagle, D.A., Shiloh. Y., Collins F.S. and Hieter, P. (1995) *HAT1*/*TEL1,* a *Saccharomyces cerevisiae* homologue of the human gene mutated in ataxia-telangiectasia, is functionally related to the yeast checkpoint gene *MEC1*/*ESR1.* Cell *submitted.*

Murray, J.M., Doe, C. Schenk, P. Carr, A.M.. Lehmann, A.R and Watts, F.Z. (1992) Cloning and characterization of the *S. pombe rad15* gene, a homologue to the *S. cerevisiae RAD3* and human *ERCC2* genes Nucleic Acids Res. *20,* 2673-2678.

Nasim, A. and Smith, B.P. (1975) Genetic control of radiation sensitivity in *Schizosaccharomyces pombe.* Genetics *79*, 573-582.

Painter, R.B. and Young, B.R. (1980) Radiosensitivity in ataxia-telangiectasia: A new explanation. Proc. Natl. Acad. Sci. USA. 77, 7315-7317

Rowley, R., Subramani, S. and Young, P.G. (1992). Checkpoint controls in *Schizosaccharomyces pombe, rad1.* EMBO J. *11,* 1335-1342.

Savitsky, K., Bar-Shira, A., Gilad, S., Rotman, G., Ziv, Y., Vanagaite L., Tagle, D.A., Smith, S., Uziel, T., Sfez, S., Ashkenazi, M., Pecker, I., Frydman, M., Hamik, R., Patanjali, S.R., Simmons, A., Clines, G.A., Sartiel, A., Gatti, R.A., Chessa, L., Sanal, O., Lavine, M.F., Jaspers, N.G.J., Taylor, M.R., Arlett, C.F., Miki, T., Weissman, S.M., Lovett, M., Collins, F.S. and Shiloh, Y. (1995). A single ataxia telangiectasia gene with a product similar to PI-3 kinase. Science *286*, 1749-1753.

Seaton, B.L., Yucel, J., Sunnerhagen P. and Subramani, S. (1992). Isolation and characterisation of the *Schizosaccharomyces pombe rad3* gene which is involved in the DNA damage and DNA synthesis checkpoints. Gene *119,* 83-89.

Schu, P.V., Takegawa, K., Fry, M.J., Stack, J.H., Waterfield, M.D. and Emr, S.D. (1993) Phosphatidylinositol 3-kinase encoded by yeast VPS34 gene essential for protein sorting. Science *260,* 88-91.

Sheldrick. K.S. and Carr, A.M. (1993). Feedback controls and G2 checkpoints, fission yeast as a model system. BioEssays *15*, 775-782.

Walworth, N., Davey, S. and Beach, D. (1993). Fission yeast *chk1* protein kinase links the *rad* checkpoint pathway to *cdc2.* Nature *363*, 368-371.

Weinert, T.A., and Hartwell, L.H. (1988). The *RAD9* gene controls the cell cycle response to DNA damage in *Saccharomyces cerevisiae.* Science *241*, 317-322.

Weinert, T.A., Kiser, G.L. Hartwell, L.H. (1994). Mitotic checkpoint genes in budding yeast and the dependence of mitosis on DNA replication and repair. Genes Dev. *8*, 652-665.

### Sequence Information.

Sequence ID No. 1: ATR seq
Sequence ID No. 2: ATR protein
Sequence ID No. 3: rad3.seq *In italics. sequenced by Seaton et al.*
   *In Bold are those bases deleted in Seaton et al. (2499. 22501. 2507. 2509)*
   *Underlined are the two bases either side of a single C insert (5918*/*5919) in Seaton et al. (i.e. the incorrect base not shown, but the one residue either side is)*
Sequence ID No. 4: rad3 protein

## Claims

1. A purified and isolated polynucleotide encoding a human rad3 polypeptide (ATR) having lipid kinase activity, said polynucleotide selected from the group consisting of:
(a) the polynucleotide set out in SEQ ID NO:1; and
(b) a polynucleotide which selectively hybridises to the complement of the polynucleotide of (a) under medium to high stringency conditions.

2. A polynucleotide according to Claim 1 which is a DNA.

3. A polynucleotide according to Claim 2 which is a genomic DNA.

4. A polynucleotide according to Claim 2 which is a cDNA.

5. A purified and isolated human rad3 polypeptide homologue (ATR) which is encoded by the polynucleotide according to any one of Claims 1 to 4, or a biologically active fragment thereof having lipid kinase activity.

6. A vector comprising the polynucleotide according to any one of Claims 1 to 4.

7. A vector according to Claim 6, wherein the polynucleotide is operably linked to a control sequence.

8. A vector according to Claim 6 or 7, which is a plasmid, virus or phage vector.

9. An *ex vivo* host cell transformed or transfected with the vector of any one of Claims 6 to 8.

10. A host cell according to Claim 9, which is a bacterial, yeast, insect or mammalian host cell.

11. A process for preparing a polypeptide according to Claim 5, comprising cultivating a host cell according to Claim 9 or 10 under conditions to provide for expression of the polypeptide, and recovering the expressed polypeptide.

12. An antibody product capable of selectively binding to the polypeptide of Claim 5.

13. An antibody product according to Claim 12 which is a monoclonal antibody.

14. An antibody product according to Claim 12 which is a polyclonal antibody.

15. A method for detecting the presence of a polynucleotide according to Claim 1 in a human or animal body sample comprising the steps of:
(a) contacting a human or animal body sample containing DNA or RNA with a probe comprising a polynucleotide according to Claim 1 under hybridising conditions; and
(b) detecting in the sample any duplex formed between the probe and nucleic acid.

16. A method of detecting a polypeptide according to Claim 5 in a biological sample comprising the steps of:
(a) contacting the biological sample with an antibody according to Claims 13 or 14 under conditions which allow for the formation of an antibody-antigen complex; and
(b) determining whether antibody-antigen complex comprising said antibody is formed.

17. An assay for screening candidate substances as anti-cancer agents comprising the steps of:
(a) contacting an ATR polypeptide according to Claim 5 and a substrate for lipid kinase activity in the presence and absence of a candidate substance; and
(b) identifying said candidate substance as an anti-cancer agent when decreased lipid kinase activity on the substrate is detected in the presence of the candidate substance compared to lipid kinase activity in the absence of the candidate substance.

18. An assay for screening candidate substances for an anti-cancer agent comprising the steps of:
(a) contacting a first polypeptide according to Claim 5 with a second polypeptide according to Claim 5 under conditions which permit complex formation between the first and second polypeptides and in the presence and absence of a candidate substance; said second polypeptide being the same as or different from the first polypeptide; and
(b) identifying the candidate substance as an anti-cancer agent wherein decreased complex formation between the first polypeptide and the second polypeptide is detected in the presence of the candidate substance compared to complex formation in the absence of the candidate substance.

19. The method according to Claim 18, wherein said first polypeptide can be distinguished from said second polypeptide.

20. A purified and isolated *S.pombe* rad3 polypeptide homologue (ATR) comprising the amino acid sequence set out in SEQ ID NO:4.

21. A purified and isolated polynucleotide encoding the *S.pombe* rad3 polypeptide homologue (ATR) of Claim 20.

22. A purified and isolated polynucleotide encoding an *S.pombe* polypeptide homologue (ATR) having lipid kinase activity, said polynucleotide selected from the group consisting of:
(a) the polynucleotide set out in SEQ ID NO:3; and
(b) a polynucleotide which selectively hybridises to the complement of the polynucleotide of (a) under medium to high stringency conditions.

23. A polynucleotide according to Claim 21 or 22 which is a DNA.

24. A polynucleotide according to Claim 23 which is a genomic DNA.

25. A polynucleotide according to Claim 23 which is a cDNA.

26. A purified isolated *S.pombe* rad3 polypeptide homologue (ATR) which is encoded by the polynucleotide according to any one of Claims 22 to 25, or a biologically active fragment thereof having lipid kinase activity.

27. A vector comprising the polynucleotide according to any one of Claims 21 to 25.

28. A vector according to Claim 27, wherein the polynucleotide is operably linked to a control sequence.

29. A vector according to Claim 27 or 28, which is a plasmid, virus or phage vector.

30. An *ex vivo* host cell transformed or transfected with the vector of any one of Claims 27 to 29.

31. A host cell according to Claim 30, which is a bacterial, yeast, insect or mammalian host cell.

32. A process for preparing a polypeptide according to claim 20 or 26, comprising cultivating a host cell transformed or transfected with a vector according to any one of Claims 27 to 29 under conditions to provide for expression of the polypeptide, and recovering the expressed polypeptide.

33. An antibody product capable of selectively binding to the lipid kinase domain of the polypeptide of Claims 20 or 26.

34. An antibody product according to Claim 33 which is a monoclonal antibody.

35. An antibody product according to Claim 33 which is a polyclonal antibody.

36. A method for detecting the presence of a polynucleotide according to Claim 21 or 22 in a human or animal body sample comprising the steps of:
(a) contacting a human or animal body sample containing DNA or RNA with a probe comprising a polynucleotide according to Claim 21 or 22, under hybriding conditions; and
(b) detecting in the sample any duplex formation between the probe and nucleic acid.

37. A method of detecting a polypeptide according to Claim 20 or 26 in a biological sample comprising the steps of:
(a) contacting the biological sample with an antibody according to Claim 34 or 35 under conditions which allow for the formation of an antibody-antigen complex; and
(b) determining whether antibody-antigen complex comprising said antibody is formed.

38. An assay for screening candidate substances as anti-cancer agents comprising the steps of:
(a) contacting an ATR polypeptide according to Claim 20 or 26 and a substrate for lipid kinase activity in the presence and absence of a candidate substance; and
(b) identifying said candidate substance as an anti-cancer agent when decreased lipid kinase activity on the substrate is detected in the presence of the candidate substance compared to lipid kinase activity in the absence of the candidate substance.

39. An assay for screening candidate substances for an anti-cancer agent comprising the steps of:
(a) contacting a first polypeptide according to Claim 20 or 26 with a second polypeptide according to Claim 20 or 26 under conditions which permit complex formation between the first and second polypeptides and in the presence and absence of a candidate substance; said second polypeptide being the same as or different from the first polypeptide; and
(b) identifying the candidate substance as an anti-cancer agent wherein decreased complex formation between the first polypeptide and the second polypeptide is detected in the presence of the candidate substance compared to complex formation in the absence of the candidate substance.

40. The assay according to Claim 39, wherein said first polypeptide can be distinguished from said second polypeptide.

## Patentansprüche

1. Gereinigtes und isoliertes Polynukleotid, das für ein humanes rad3-Polypeptid (ATR) kodiert, das eine Lipidkinase-Aktivität aufweist, wobei das Polynukleotid ausgewählt ist aus der Gruppe bestehend aus:
(a) das Polynukleotid, das in SEQ ID NR. 1 dargelegt wird; und
(b) ein Polynukleotid, das selektiv an das Komplement des Polynukleotids von (a) unter Bedingungen mittlerer bis hoher Stringenz hybridisiert.

2. Polynukleotid nach Anspruch 1, das eine DNA ist.

3. Polynukleotid nach Anspruch 2, das eine genomische DNA ist.

4. Polynukleotid nach Anspruch 2, das eine cDNA ist.

5. Gereinigtes und isoliertes humanes rad3-Polypeptidhomolog (ATR), das durch das Polynukleotid nach einem der Ansprüche 1 bis 4 kodiert wird, oder ein biologisch wirksames Fragment davon, das eine Lipidkinasae-Aktivität aufweist.

6. Vektor, umfassend das Polynukleotid nach einem der Ansprüche 1 bis 4.

7. Vektor nach Anspruch 6, wobei das Polynukleotid mit einer Kontrollsequenz funktionsfähig verknüpft ist.

8. Vektor nach Anspruch 6 oder 7, der ein Plasmid, Virus oder Phagenvektor ist.

9. *Ex vivo* Wirtszelle, die mit dem Vektor nach einem der Ansprüche 6 bis 8 transformiert oder transfiziert wird.

10. Wirtzelle nach Anspruch 9, die eine bakterielle, Hefe-, Insekten- oder Säugetierwirtszelle ist.

11. Verfahren zur Herstellung eines Polypeptids nach Anspruch 5, umfassend Kultivieren einer Wirtzelle nach Anspruch 9 oder 10 unter Bedingungen, die die Expression des Polypeptids bereitstellen und Aufreinigung des exprimierten Polypeptids.

12. Antikörperprodukt, das geeignet ist, um selektiv an das Polypeptid nach Anspruch 5 zu binden.

13. Antikörperprodukt nach Anspruch 12, das ein monoklonaler Antikörper ist.

14. Antikörperprodukt nach Anspruch 12, das ein polyklonaler Antikörper ist.

15. Verfahren zum Nachweis der Anwesenheit eines Polynukleotids nach Anspruch 1 in einer menschlichen oder tierischen Körperprobe, umfassend die Schritte von:
(a) in Kontakt bringen einer menschlichen oder tierischen Körperprobe, die DNA oder RNA enthält, mit einer Sonde, die ein Polynukleotid nach Anspruch 1 umfasst unter hybridisierenden Bedingungen; und
(b) Nachweisen jeglicher Duplex, die zwischen der Sonde und der Nukleinsäure gebildet wird in der Probe.

16. Verfahren zum Nachweis eines Polypeptids nach Anspruch 5 in einer biologischen Probe, umfassend die Schritte von
(a) in Kontakt bringen der biologischen Probe mit einem Antikörper nach Anspruch 13 oder 14 unter Bedingungen, die die Bildung eines Antikörper-Antigenkomplexes erlauben; und
(b) Feststellen, ob der Antikörper-Antigenkomplex, der den Antikörper umfaßt, gebildet wird.

17. Test zum Screenen von Kandidatsubstanzen als Antikrebsmittel, umfassend die Schritte von:
(a) in Kontakt bringen eines ATR-Polypeptids nach Anspruch 5 und eines Substrats für Lipidkinase-Aktivität in Anwesenheit und Abwesenheit einer Kandidatensubstanz; und
(b) Identifizieren der Kandidatensubstanz als Antikrebsmittel, wenn bei Anwesenheit der Kandidatensubstanz herabgesetzte Lipidkinase-Aktivität des Substrats im Vergleich zur Lipidkinase-Aktivität bei Abwesenheit der Kandidatensubstanz nachgewiesen wird.

18. Test zum Screenen von Kandidatensubstanzen als Antikrebsmittel, umfassend die Schritte von:
(a) in Kontakt bringen eines ersten Polypeptids nach Anspruch 5 mit einem zweiten Polypeptid nach Anspruch 5 unter Bedingungen, die eine Komplexbildung zwischen dem ersten und zweiten Polypeptid erlauben und bei Anwesenheit und Abwesenheit einer Kandidatensubstanz; wobei das zweite Polypeptid gleich oder unterschiedlich von dem ersten Polypeptid ist; und
(b) Identifizieren der Kandidatensubstanz als Antikrebsmittel, wobei im Vergleich zur Komplexbildung bei Abwesenheit der Kandidatensubstanz herabgesetzte Komplexbildung zwischen dem ersten Polypeptid und dem zweiten Polypeptid bei Anwesenheit der Kandidatensubstanz nachgewiesen wird.

19. Test nach Anspruch 18, wobei das erste Polypeptid vom zweiten Polypeptid unterschieden werden kann.

20. Gereinigtes und isoliertes *S.pombe* rad3-Polypeptidhomolog (ATR), umfassend die Aminosäuresequenz, die in SEQ ID NR. 4 dargelegt wird.

21. Gereinigtes und isoliertes Polynukleotid, das für das *S.pombe* rad3-Polypeptidhomolog (ATR) nach Anspruch 20 kodiert.

22. Gereinigtes und isoliertes Polynukleotid, das für ein *S.pombe* Polypeptidhomolog (ATR) kodiert, das eine Lipidkinase-Aktivität aufweist, wobei das Polynukleotid ausgewählt ist aus der Gruppe bestehend aus:
(a) dem Polynukleotid, das in SEQ ID NR. 3 dargelegt wird; und
(b) einem Polynukleotid, das selektiv an das Komplement des Polynukleotids aus (a) Bedingungen unter mittlerer bis hoher Stringez hybridisiert.

23. Polynukleotid nach Anspruch 21 oder 22, das eine DNA ist.

24. Polynukleotid nach Anspruch 23, das eine genomische DNA ist.

25. Polynukleotid nach Anspruch 23, das eine cDNA ist.

26. Gereinigtes isoliertes *S.pombe* rad3-Polypeptidhomolog (ATR), das durch das Polynukleotid nach einem der Ansprüche 22 bis 25 kodiert wird oder ein biologisch wirksames Fragment davon, das eine Lipidkinase-Aktivität aufweist.

27. Vektor, umfassend das Polynukleotid nach einem der Ansprüche 21 bis 25.

28. Vektor nach Anspruch 27, wobei das Polynukleotid mit einer Kontrollsequenz funktionsfähig verknüpft ist.

29. Vektor nach Anspruch 27 oder 28, der ein Plasmid-, Virus- oder Phagenvektor ist.

30. *Ex vivo* Wirtszelle, die mit dem Vektor nach einem der Ansprüche 27 bis 29 transformiert oder transfiziert ist.

31. Wirtszelle nach Anspruch 30, die eine bakterielle, Hefe-, Insekten- oder Säugetierwirtszelle ist.

32. Verfahren zum Herstellen eines Polypeptids nach Anspruch 20 oder 26, umfassend Kultivieren einer Wirtszelle, die mit einem Vektor nach einem der Ansprüche 27 bis 29 unter Bedingungen transformiert oder transfiziert wird, unter Bedingungen, die die Expression des Polypeptids bereitstellen und Aufreinigung des exprimierten Polypeptids.

33. Antikörperprodukt, das geeignet ist, an die Lipidkinase-Domäne des Polypeptids nach Anspruch 20 oder 26 selektiv zu binden.

34. Antikörperprodukt nach Anspruch 33, das ein monoklonaler Antikörper ist.

35. Antikörperprodukt nach Anspruch 33, das ein polyklonaler Antikörper ist.

36. Verfahren zum Nachweis der Anwesenheit eines Polynukleotids nach Anspruch 21 oder 22 in einer menschlichen oder tierischen Körperprobe, umfassend die Schritte von:
(a) in Kontakt bringen einer menschlichen oder tierischen Körperprobe, die DNA oder RNA enthält, mit einer Sonde, umfassend ein Polynukleotid nach Anspruch 21 oder 22 unter hybridisierenden Bedingungen; und
(b) Nachweisen in der Probe jeglicher Duplexbildung zwischen der Sonde und der Nukleinsäure.

37. Verfahren zum Nachweisen eines Polypeptids nach Anspruch 20 oder 26 in einer biologischen Probe, umfassend die Schritte von:
(a) in Kontakt bringen der biologischen Probe mit einem Antikörper nach Anspruch 34 oder 35 unter Bedingungen, die die Bildung eines Antikörper-Antigenkomplexes erlauben; und
(b) Feststellen, ob Antikörper-Antigenkomplex, umfassend den Antikörper, gebildet wird.

38. Test zum Screenen von Kandidatsubstanzen als Antikrebsmittel, umfassend die Schritte von:
(a) in Kontakt bringen eines ATR-Polypeptids nach Anspruch 20 oder 26 und eines Substrats für Lipidkinase-Aktivität in Anwesenheit und Abwesenheit einer Kandidatensubstanz; und
(b) Identifizieren der Kandidatensubstanz als Antikrebsmittel, wenn bei Anwesenheit der Kandidatensubstanz herabgesetzte Lipidkinase-Aktivität des Substrats nachgewiesen wird, im Vergleich zu der Lipidkinaseaktivität bei Abwesenheit der Kandidatensubstanz.

39. Test zum Screenen von Kandidatensubstanzen als Antikrebsmittel, umfassend die Schritte von:
(a) in Kontakt bringen eines ersten Polypeptids nach Anspruch 20 oder 26 mit einem zweiten Polypeptid nach Anspruch 20 oder 26 unter Bedingungen, die eine Komplexbildung zwischen dem ersten und zweiten Polypeptid erlauben und bei Anwesenheit und Abwesenheit einer Kandidatensubstanz; wobei das zweite Polypeptid gleich oder unterschiedlich von dem ersten Polypeptid ist; und
(b) Identifizieren der Kandidatensubstanz als Antikrebsmittel, wobei im Vergleich zur Komplexbildung bei Abwesenheit der Kandidatensubstanz herabgesetzte Komplexbildung zwischen dem ersten Polypeptid und dem zweiten Polypeptid bei Anwesenheit der Kandidatensubstanz nachgewiesen wird.

40. Test nach Anspruch 39, wobei das erste Polypeptid vom zweiten Polypeptid unterschieden werden kann.

## Revendications

1. Polynucléotide purifié et isolé codant un polypeptide rad3 humain (ATR) ayant une activité de lipide kinase, ledit polynucléotide étant choisi parmi le groupe constitué :
(a) du polynucléotide indiqué dans la SEQ ID N° 1 ; et
(b) d'un polynucléotide qui s'hybride sélectivement au complément du polynucléotide de (a) sous des conditions de stringence moyennes à fortes.

2. Polynucléotide selon la revendication 1 qui est un ADN.

3. Polynucléotide selon la revendication 2 qui est un ADN génomique.

4. Polynucléotide selon la revendication 2 qui est un ADNc.

5. Homologue du polypeptide rad3 humain purifié et isolé (ATR) qui est codé par le polynucléotide selon une quelconque des revendications 1 à 4, ou un fragment biologiquement actif de celui-ci ayant une activité de lipide kinase.

6. Vecteur comprenant le polynucléotide selon une quelconque des revendications 1 à 4.

7. Vecteur selon la revendication 6, dans lequel le polynucléotide est lié fonctionnellement à une séquence contrôle.

8. Vecteur selon la revendication 6 ou 7, qui est un plasmide, un virus ou un phage.

9. Cellule hôte ex vivo transformée ou transfectée par le vecteur d'une quelconque des revendications 6 à 8.

10. Cellule hôte selon la revendication 9, qui est une cellule hôte bactérienne, de levure, d'insecte ou de mammifère.

11. Processus pour préparer un polypeptide selon la revendication 5, consistant à mettre en culture une cellule hôte selon la revendication 9 ou 10 sous des conditions pour fournir l'expression du polypeptide, et à récupérer le polypeptide exprimé.

12. Produit anticorps capable de se lier sélectivement au polypeptide de la revendication 5.

13. Produit anticorps selon la revendication 12 qui est un anticorps monoclonal.

14. Produit anticorps selon la revendication 12 qui est un anticorps polyclonal.

15. Procédé pour détecter la présence d'un polynucléotide selon la revendication 1 dans un échantillon corporel humain ou animal comprenant les étapes consistant à :
(a) mettre en contact un échantillon corporel humain ou animal contenant de l'ADN ou de l'ARN avec une sonde comprenant un polynucléotide selon la revendication 1 sous des conditions d'hybridation ; et
(b) détecter dans l'échantillon un quelconque duplex formé entre la sonde et l'acide nucléique.

16. Procédé pour détecter un polypeptide selon la revendication 5 dans un échantillon biologique comprenant les étapes consistant à :
(a) mettre en contact l'échantillon biologique avec un anticorps selon les revendications 13 ou 14 dans des conditions qui permettent la formation d'un complexe anticorps-antigène ; et
(b) déterminer si le complexe anticorps-antigène comprenant ledit anticorps est formé.

17. Test pour cribler des substances candidates en tant qu'agents anti-cancéreux comprenant les étapes consistant à :
(a) mettre en contact un polypeptide ATR selon la revendication 5 et un substrat pour l'activité de lipide kinase en présence et en l'absence d'une substance candidate ; et
(b) identifier ladite substance candidate en tant qu'agent anti-cancéreux quand l'activité de lipide kinase diminuée sur le substrat est détectée en présence de la substance candidate comparativement à l'activité de lipide kinase en l'absence de la substance candidate.

18. Test pour cribler des substances candidates pour un agent anti-cancéreux comprenant les étapes consistant à :
(a) mettre en contact un premier polypeptide selon la revendication 5 avec un second polypeptide selon la revendication 5 dans des conditions qui permettent la formation d'un complexe entre le premier et le second polypeptides et en présence et en l'absence d'une substance candidate, ledit second polypeptide étant le même que le premier polypeptide ou étant différent de celui-ci ; et
(b) identifier la substance candidate en tant qu'agent anti-cancéreux dans lequel la formation du complexe diminuée entre le premier polypeptide et le second polypeptide est détectée en présence de la substance candidate comparativement à la formation du complexe en l'absence de la substance candidate.

19. Procédé selon la revendication 18, dans lequel ledit premier polypeptide peut être distingué dudit second polypeptide.

20. Homologue du polypeptide rad3 de S.pombe purifié et isolé (ATR) comprenant la séquence d'acides aminés indiquée dans la SEQ ID N° 4.

21. Polynucléotide purifié et isolé codant l'homologue du polypeptide rad3 de S.pombe (ATR) de la revendication 20.

22. Polynucléotide purifié et isolé codant un homologue du polypeptide de S.pombe (ATR) ayant une activité de lipide kinase, ledit polynucléotide étant choisi parmi le groupe constitué :
(a) du polynucléotide indiqué dans la SEQ ID N° 3 ; et
(b) d'un polynucléotide qui s'hybride sélectivement au complément du polynucléotide de (a) sous des conditions de stringence moyennes à fortes.

23. Polynucléotide selon la revendication 21 ou 22 qui est un ADN.

24. Polynucléotide selon la revendication 23 qui est un ADN génomique.

25. Polynucléotide selon la revendication 23 qui est un ADNc.

26. Homologue du polypeptide rad3 de S.pombe purifié et isolé (ATR) qui est codé par le polynucléotide selon une quelconque des revendications 22 à 25, ou un fragment biologiquement actif de celui-ci ayant une activité de lipide kinase.

27. Vecteur comprenant le polynucléotide selon une quelconque des revendications 21 à 25.

28. Vecteur selon la revendication 27, dans lequel le polynucléotide est lié de façon fonctionnelle à une séquence contrôle.

29. Vecteur selon la revendication 27 ou 28, qui est un plasmide, un virus ou un phage.

30. Cellule hôte ex vivo transformée ou transfectée avec le vecteur d'une quelconque des revendications 27 à 29.

31. Cellule hôte selon la revendication 30, qui est une cellule hôte bactérienne, de levure, d'insecte ou de mammifère.

32. Processus pour préparer un polypeptide selon la revendication 20 ou 26, consistant à mettre en culture une cellule hôte transformée ou transfectée avec un vecteur selon une quelconque des revendications 27 à 29 sous des conditions pour fournir l'expression du polypeptide, et à récupérer le polypeptide exprimé.

33. Produit anticorps capable de se lier sélectivement au domaine de lipide kinase du polypeptide des revendications 20 ou 26.

34. Produit anticorps selon la revendication 33 qui est un anticorps monoclonal.

35. Produit anticorps selon la revendication 33 qui est un anticorps polyclonal.

36. Procédé pour détecter la présence d'un polynucléotide selon la revendication 21 ou 22 dans un échantillon corporel humain ou animal comprenant les étapes consistant à :
(a) mettre en contact un échantillon corporel humain ou animal contenant de l'ADN ou de l'ARN avec une sonde comprenant un polynucléotide selon la revendication 21 ou 22, dans des conditions d'hybridation ; et
(b) détecter dans l'échantillon une quelconque formation de duplex entre la sonde et l'acide nucléique.

37. Procédé pour détecter un polypeptide selon la revendication 20 ou 26 dans un échantillon biologique comprenant les étapes consistant à :
(a) mettre en contact l'échantillon biologique avec un anticorps selon la revendication 34 ou 35 dans des conditions qui permettent la formation d'un complexe anticorps-antigène ; et
(b) déterminer si le complexe anticorps-antigène comprenant ledit anticorps est formé.

38. Test pour cribler des substances candidates en tant qu'agents anti-cancéreux comprenant les étapes consistant à :
(a) mettre en contact un polypeptide ATR selon la revendication 20 ou 26 et un substrat pour l'activité de lipide kinase en présence et en l'absence d'une substance candidate ; et
(b) identifier ladite substance candidate en tant qu'agent anti-cancéreux quand l'activité de lipide kinase diminuée sur le substrat est détectée en présence de la substance candidate comparativement à l'activité de lipide kinase en l'absence de la substance candidate.

39. Test pour cribler des substances candidates pour un agent anti-cancéreux comprenant les étapes consistant à :
(a) mettre en contact un premier polypeptide selon la revendication 20 ou 26 avec un second polypeptide selon la revendication 20 ou 26 dans des conditions qui permettent la formation d'un complexe entre le premier et le second polypeptides et en présence et en l'absence d'une substance candidate, ledit second polypeptide étant le même que le premier polypeptide ou étant différent de celui-ci; et
(b) identifier la substance candidate en tant qu'agent anti-cancéreux dans lequel la formation du complexe diminuée entre le premier polypeptide et le second polypeptide est détectée en présence de la substance candidate comparativement à la formation du complexe en l'absence de la substance candidate.

40. Test selon la revendication 39, dans lequel ledit premier polypeptide peut être distingué dudit second polypeptide.


### SEQUENCE LISTING

(1) GENERAL INFORMATION:
   (i) APPLICANT:
      (A) NAME: Medical Research Council
      (B) STREET: 20 Park Crescent
      (C) CITY: London
      (E) COUNTRY: UK
      (F) POSTAL CODE (ZIP): W1N 4AL
   (ii) TITLE OF INVENTION: CHECKPOINT GENE
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 8239 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: H. sapiens
   (ix) FEATURE:
      (A) NAME/KEY: CDS
      (B) LOCATION:80..8014
   (xi) SEQUENCE DESCRIPTION: SEQ ID NO: 1:
   GCGCTCTTCC GGCAGCGGTA CGTTTGGAGA CGCCGGGAAC CCGCGTTGGC GTGGTTGACT        60
   AGTGCCTCGC AGCCTCAGC ATG GGG GAA CAT GGC CTG GAG CTG GCT TCC ATG        112
                        Met Gly Glu His Gly Leu Glu Leu Ala Ser Met
                         1               5                  10
   ATC CCC GCC CTG CGG GAG CTG GGC AGT GCC ACA CCA GAG GAA TAT AAT         160
   Ile Pro Ala Leu Arg Glu Leu Gly Ser Ala Thr Pro Glu Glu Tyr Asn
               15                  20                  25
   ACA GTT GTA CAG AAG CCA AGA CAA ATT CTG TGT CAA TTC ATT GAC CGG         208
   Thr Val Val Gln Lys Pro Arg Gln Ile Leu Cys Gln Phe Ile Asp Arg
           30                  35                  40
   ATA CTT ACA GAT GTA AAT GTT GTT GCT GTA GAA CTT GTA AAG AAA ACT         256
   Ile Leu Thr Asp Val Asn Val Val Ala Val Glu Leu Val Lys Lys Thr
       45                  50                  55
   GAC TCT CAG CCA ACC TCC GTG ATG TTG CTT GAT TTC ATC CAG CAT ATC 304
   Asp Ser Gln Pro Thr Ser Val Met Leu Leu Asp Phe Ile Gln His Ile
   60 65 70 75
   ATG AAA TCC TCC CCA CTT ATG TTT GTA AAT GTG AGT GGA AGC CAT GAG 352
   Met Lys Ser Ser Pro Leu Met Phe Val Asn Val Ser Gly Ser His Glu
   80 85 90
   CGC AAA GGC AGT TGT ATT GAA TTC AGT AAT TGG ATC ATA ACG AGA CTT 400
   Arg Lys Gly Ser Cys Ile Glu Phe Ser Asn Trp Ile Ile Thr Arg Leu
   95 100 105
   CTG CGG ATT GCA GCA ACT CCC TCC TGT CAT TTG TTA CAC AAG AAA ATC 448
   Leu Arg Ile Ala Ala Thr Pro Ser Cys His Leu Leu His Lys Lys Ile
   110 115 120
   TGT GAA GTC ATC TGT TCA TTA TTA TTT CTT TTT AAA AGC AAG AGT CCT 496
   Cys Glu Val Ile Cys Ser Leu Leu Phe Leu Phe Lys Ser Lys Ser Pro
   125 130 135
   GCT ATT TTT GGG GTA CTC ACA AAA GAA TTA TTA CAA CTT TTT GAA GAC 544
   Ala Ile Phe Gly Val Leu Thr Lys Glu Leu Leu Gln Leu Phe Glu Asp
   140 145 150 155
   TTG GTT TAC CTC CAT AGA AGA AAT GTG ATG GGT CAT GCT GTG GAA TGG 592
   Leu Val Tyr Leu His Arg Arg Asn Val Met Gly His Ala Val Glu Trp
   160 165 170
   CCA GTG GTC ATG AGC CGA TTT TTA AGT CAA TTA GAT GAA CAC ATG GGA 640
   Pro Val Val Met Ser Arg Phe Leu Ser Gln Leu Asp Glu His Met Gly
   175 180 185
   TAT TTA CAA TCA GCT CCT TTG CAG TTG ATG AGT ATG CAA AAT TTA GAA 688
   Tyr Leu Gln Ser Ala Pro Leu Gln Leu Met Ser Met Gln Asn Leu Glu
   190 195 200
   TTT ATT GAA GTC ACT TTA TTA ATG GTT CTT ACT CGT ATT ATT GCA ATT 736
   Phe Ile Glu Val Thr Leu Leu Met Val Leu Thr Arg Ile Ile Ala Ile
   205 210 215
   GTG TTT TTT AGA AGG CAA GAA CTC TTA CTT TGG CAG ATA GGT TGT GTT 784
   Val Phe Phe Arg Arg Gln Glu Leu Leu Leu Trp Gln Ile Gly Cys Val
   220 225 230 235
   CTG CTA GAG TAT GGT AGT CCA AAA ATT AAA TCC CTA GCA ATT AGC TTT 832
   Leu Leu Glu Tyr Gly Ser Pro Lys Ile Lys Ser Leu Ala Ile Ser Phe
   240 245 250
   TTA ACA GAA CTT TTT CAG CTT GGA GGA CTA CCA GCA CAA CCA GCT AGC 880
   Leu Thr Glu Leu Phe Gln Leu Gly Gly Leu Pro Ala Gln Pro Ala Ser
   255 260 265
   ACT TTT TTC AGC TCA TTT TTG GAA TTA TTA AAA CAC CTT GTA GAA ATG 928
   Thr Phe Phe Ser Ser Phe Leu Glu Leu Leu Lys His Leu Val Glu Met
   270 275 280
   GAT ACT GAC CAA TTG AAA CTC TAT GAA GAG CCA TTA TCA AAG CTG ATA 976
   Asp Thr Asp Gln Leu Lys Leu Tyr Glu Glu Pro Leu Ser Lys Leu Ile
   285 290 295
   AAG ACA CTA TTT CCC TTT GAA GCA GAA GCT TAT AGA AAT ATT GAA CCT 1024
   Lys Thr Leu Phe Pro Phe Glu Ala Glu Ala Tyr Arg Asn Ile Glu Pro
   300 305 310 315
   GTC TAT TTA AAT ATG CTG CTG GAA AAA CTC TGT GTC ATG TTT GAA GAC 1072
   Val Tyr Leu Asn Met Leu Leu Glu Lys Leu Cys Val Met Phe Glu Asp
   320 325 330
   GGT GTG CTC ATG CGG CTT AAG TCT GAT TTG CTA AAA GCA GCT TTG TGC 1120
   Gly Val Leu Met Arg Leu Lys Ser Asp Leu Leu Lys Ala Ala Leu Cys
   335 340 345
   CAT TTA CTG CAG TAT TTC CTT AAA TTT GTG CCA GCT GGG TAT GAA TCT 1168
   His Leu Leu Gln Tyr Phe Leu Lys Phe Val Pro Ala Gly Tyr Glu Ser
   350 355 360
   GCT TTA CAA GTC AGG AAG GTC TAT GTG AGA AAT ATT TGT AAA GCT CTT 1216
   Ala Leu Gln Val Arg Lys Val Tyr Val Arg Asn Ile Cys Lys Ala Leu
   365 370 375
   TTG GAT GTG CTT GGA ATT GAG GTA GAT GCA GAG TAC TTG TTG GGC CCA 1264
   Leu Asp Val Leu Gly Ile Glu Val Asp Ala Glu Tyr Leu Leu Gly Pro
   380 385 390 395
   CTT TAT GCA GCT TTG AAA ATG GAA AGT ATG GAA ATC ATT GAG GAG ATT 1312
   Leu Tyr Ala Ala Leu Lys Met Glu Ser Met Glu Ile Ile Glu Glu Ile
   400 405 410
   CAA TGC CAA ACT CAA CAG GAA AAC CTC AGC AGT AAT AGT GAT GGA ATA 1360
   Gln Cys Gln Thr Gln Gln Glu Asn Leu Ser Ser Asn Ser Asp Gly Ile
   415 420 425
   TCA CCC AAA AGG CGT CGT CTC AGC TCG TCT CTA AAC CCT TCT AAA AGA 1408
   Ser Pro Lys Arg Arg Arg Leu Ser Ser Ser Leu Asn Pro Ser Lys Arg
   430 435 440
   GCA CCA AAA CAG ACT GAG GAA ATT AAA CAT GTG GAC ATG AAC CAA AAG 1456
   Ala Pro Lys Gln Thr Glu Glu Ile Lys His Val Asp Met Asn Gln Lys
   445 450 455
   AGC ATA TTA TGG AGT GCA CTG AAA CAG AAA GCT GAA TCC CTT CAG ATT 1504
   Ser Ile Leu Trp Ser Ala Leu Lys Gln Lys Ala Glu Ser Leu Gln Ile
   460 465 470 475
   TCC CTT GAA TAC AGT GGC CTA AAG AAT CCT GTT ATT GAG ATG TTA GAA 1552
   Ser Leu Glu Tyr Ser Gly Leu Lys Asn Pro Val Ile Glu Met Leu Glu
   480 485 490
   GGA ATT GCT GTT GTC TTA CAA CTG ACT GCT CTG TGT ACT GTT CAT TGT 1600
   Gly Ile Ala Val Val Leu Gln Leu Thr Ala Leu Cys Thr Val His Cys
   495 500 505
   TCT CAT CAA AAC ATG AAC TGC CGT ACT TTC AAG GAC TGT CAA CAT AAA 1648
   Ser His Gln Asn Met Asn Cys Arg Thr Phe Lys Asp Cys Gln His Lys
   510 515 520
   TCC AAG AAG AAA CCT TCT GTA GTG ATA ACT TGG ATG TCA TTG GAT TTT 1696
   Ser Lys Lys Lys Pro Ser Val Val Ile Thr Trp Met Ser Leu Asp Phe
   525 530 535
   TAC ACA AAA GTG CTT AAG AGC TGT AGA AGT TTG TTA GAA TCT GTT CAG 1744
   Tyr Thr Lys Val Leu Lys Ser Cys Arg Ser Leu Leu Glu Ser Val Gln
   540 545 550 555
   AAA CTG GAC CTG GAG GCA ACC ATT GAT AAG GTG GTG AAA ATT TAT GAT 1792
   Lys Leu Asp Leu Glu Ala Thr Ile Asp Lys Val Val Lys Ile Tyr Asp
   560 565 570
   GCT TTG ATT TAT ATG CAA GTA AAC AGT TCA TTT GAA GAT CAT ATC CTG 1840
   Ala Leu Ile Tyr Met Gln Val Asn Ser Ser Phe Glu Asp His Ile Leu
   575 580 585
   GAA GAT TTA TGT GGT ATG CTC TCA CTT CCA TGG ATT TAT TCC CAT TCT 1888
   Glu Asp Leu Cys Gly Met Leu Ser Leu Pro Trp Ile Tyr Ser His Ser
   590 595 600
   GAT GAT GGC TGT TTA AAG TTG ACC ACA TTT GCC GCT AAT CTT CTA ACA 1936
   Asp Asp Gly Cys Leu Lys Leu Thr Thr Phe Ala Ala Asn Leu Leu Thr
   605 610 615
   TTA AGC TGT AGG ATT TCA GAT AGC TAT TCA CCA CAG GCA CAA TCA CGA 1984
   Leu Ser Cys Arg Ile Ser Asp Ser Tyr Ser Pro Gln Ala Gln Ser Arg
   620 625 630 635
   TGT GTG TTT CTT CTG ACT CTG TTT CCA AGA AGA ATA TTC CTT GAG TGG 2032
   Cys Val Phe Leu Leu Thr Leu Phe Pro Arg Arg Ile Phe Leu Glu Trp
   640 645 650
   AGA ACA GCA GTT TAC AAC TGG GCC CTG CAG AGC TCC CAT GAA GTA ATC 2080
   Arg Thr Ala Val Tyr Asn Trp Ala Leu Gln Ser Ser His Glu Val Ile
   655 660 665
   CGG GCT AGT TGT GTT AGT GGA TTT TTT ATC TTA TTG CAG CAG CAG AAT 2128
   Arg Ala Ser Cys Val Ser Gly Phe Phe Ile Leu Leu Gln Gln Gln Asn
   670 675 680
   TCT TGT AAC AGA GTT CCC AAG ATT CTT ATA GAT AAA GTC AAA GAT GAT 2176
   Ser Cys Asn Arg Val Pro Lys Ile Leu Ile Asp Lys Val Lys Asp Asp
   685 690 695
   TCT GAC ATT GTC AAG AAA GAA TTT GCT TCT ATA CTT GGT CAA CTT GTC 2224
   Ser Asp Ile Val Lys Lys Glu Phe Ala Ser Ile Leu Gly Gln Leu Val
   700 705 710 715
   TGT ACT CTT CAC GGC ATG TTT TAT CTG ACA AGT TCT TTA ACA GAA CCT 2272
   Cys Thr Leu His Gly Met Phe Tyr Leu Thr Ser Ser Leu Thr Glu Pro
   720 725 730
   TTC TCT GAA CAC GGA CAT GTG GAC CTC TTC TGT AGG AAC TTG AAA GCC 2320
   Phe Ser Glu His Gly His Val Asp Leu Phe Cys Arg Asn Leu Lys Ala
   735 740 745
   ACT TCT CAA CAT GAA TGT TCA TCT TCT CAA CTA AAA GCT TCT GTC TGC 2368
   Thr Ser Gln His Glu Cys Ser Ser Ser Gln Leu Lys Ala Ser Val Cys
   750 755 760
   AAG CCA TTC CTT TTC CTA CTG AAA AAA AAA ATA CCT AGT CCA GTA AAA 2416
   Lys Pro Phe Leu Phe Leu Leu Lys Lys Lys Ile Pro Ser Pro Val Lys
   765 770 775
   CTT GCT TTC ATA GAT AAT CTA CAT CAT CTT TGT AAG CAT CTT GAT TTT 2464
   Leu Ala Phe Ile Asp Asn Leu His His Leu Cys Lys His Leu Asp Phe
   780 785 790 795
   AGA GAA GAT GAA ACA GAT GTA AAA GCA GTT CTT GGA ACT TTA TTA AAT 2512
   Arg Glu Asp Glu Thr Asp Val Lys Ala Val Leu Gly Thr Leu Leu Asn
   800 805 810
   TTA ATG GAA GAT CCA GAC AAA GAT GTT AGA GTG GCT TTT AGT GGA AAT 2560
   Leu Met Glu Asp Pro Asp Lys Asp Val Arg Val Ala Phe Ser Gly Asn
   815 820 825
   ATC AAG CAC ATA TTG GAA TCC TTG GAC TCT GAA GAT GGA TTT ATA AAG 2608
   Ile Lys His Ile Leu Glu Ser Leu Asp Ser Glu Asp Gly Phe Ile Lys
   830 835 840
   GAG CTT TTT GTC TTA AGA ATG AAG GAA GCA TAT ACA CAT GCC CAA ATA 2656
   Glu Leu Phe Val Leu Arg Met Lys Glu Ala Tyr Thr His Ala Gln Ile
   845 850 855
   TCA AGA AAT AAT GAG CTG AAG GAT ACC TTG ATT CTT ACA ACA GGG GAT 2704
   Ser Arg Asn Asn Glu Leu Lys Asp Thr Leu Ile Leu Thr Thr Gly Asp
   860 865 870 875
   ATT GGA AGG GCC GCA AAA GGA GAT TTG GTA CCA TTT GCA CTC TTA CAC 2752
   Ile Gly Arg Ala Ala Lys Gly Asp Leu Val Pro Phe Ala Leu Leu His
   880 885 890
   TTA TTG CAT TGT TTG TTA TCC AAG TCA GCA TCT GTC TCT GGA GCA GCA 2800
   Leu Leu His Cys Leu Leu Ser Lys Ser Ala Ser Val Ser Gly Ala Ala
   895 900 905
   TAC ACA GAA ATT AGA GCT CTG GTT GCA GCT AAA AGT GTT AAA CTG CAA 2848
   Tyr Thr Glu Ile Arg Ala Leu Val Ala Ala Lys Ser Val Lys Leu Gln
   910 915 920
   AGT TTT TTC AGC CAG TAT AAG AAA CCC ATC TGT CAG TTT TTG GTA GAA 2896
   Ser Phe Phe Ser Gln Tyr Lys Lys Pro Ile Cys Gln Phe Leu Val Glu
   925 930 935
   TCC CTT CAC TCT AGT CAG ATG ACA GCA CTT CCG AAT ACT CCA TGC CAG 2944
   Ser Leu His Ser Ser Gln Met Thr Ala Leu Pro Asn Thr Pro Cys Gln
   940 945 950 955
   AAT GCT GAC GTG CGA AAA CAA GAT GTG GCT CAC CAG AGA GAA ATG GCT 2992
   Asn Ala Asp Val Arg Lys Gln Asp Val Ala His Gln Arg Glu Met Ala
   960 965 970
   TTA AAT ACG TTG TCT GAA ATT GCC AAC GTT TTC GAC TTT CCT GAT CTT 3040
   Leu Asn Thr Leu Ser Glu Ile Ala Asn Val Phe Asp Phe Pro Asp Leu
   975 980 985
   AAT CGT TTT CTT ACT AGG ACA TTA CAA GTT CTA CTA CCT GAT CTT GCT 3088
   Asn Arg Phe Leu Thr Arg Thr Leu Gln Val Leu Leu Pro Asp Leu Ala
   990 995 1000
   GCC AAA GCA AGC CCT GCA GCT TCT GCT CTC ATT CGA ACT TTA GGA AAA 3136
   Ala Lys Ala Ser Pro Ala Ala Ser Ala Leu Ile Arg Thr Leu Gly Lys
   1005 1010 1015
   CAA TTA AAT GTC AAT CGT AGA GAG ATT TTA ATA AAC AAC TTC AAA TAT 3184
   Gln Leu Asn Val Asn Arg Arg Glu Ile Leu Ile Asn Asn Phe Lys Tyr
   1020 1025 1030 1035
   ATT TTT TCT CAT TTG GTC TGT TCT TGT TCC AAA GAT GAA TTA GAA CGT 3232
   Ile Phe Ser His Leu Val Cys Ser Cys Ser Lys Asp Glu Leu Glu Arg
   1040 1045 1050
   GCC CTT CAT TAT CTG AAG AAT GAA ACA GAA ATT GAA CTG GGG AGC CTG 3280
   Ala Leu His Tyr Leu Lys Asn Glu Thr Glu Ile Glu Leu Gly Ser Leu
   1055 1060 1065
   TTG AGA CAA GAT TTC CAA GGA TTG CAT AAT GAA TTA TTG CTG CGT ATT 3328
   Leu Arg Gln Asp Phe Gln Gly Leu His Asn Glu Leu Leu Leu Arg Ile
   1070 1075 1080
   GGA GAA CAC TAT CAA CAG GTT TTT AAT GGT TTG TCA ATA CTT GCC TCA 3376
   Gly Glu His Tyr Gln Gln Val Phe Asn Gly Leu Ser Ile Leu Ala Ser
   1085 1090 1095
   TTT GCA TCC AGT GAT GAT CCA TAT CAG GGC CCG AGA GAT ATC ATA TCA 3424
   Phe Ala Ser Ser Asp Asp Pro Tyr Gln Gly Pro Arg Asp Ile Ile Ser
   1100 1105 1110 1115
   CCT GAA CTG ATG GCT GAT TAT TTA CAA CCC AAA TTG TTG GGC ATT TTG 3472
   Pro Glu Leu Met Ala Asp Tyr Leu Gln Pro Lys Leu Leu Gly Ile Leu
   1120 1125 1130
   GCT TTT TTT AAC ATG CAG TTA CTG AGC TCT AGT GTT GGC ATT GAA GAT 3520
   Ala Phe Phe Asn Met Gln Leu Leu Ser Ser Ser Val Gly Ile Glu Asp
   1135 1140 1145
   AAG AAA ATG GCC TTG AAC AGT TTG ATG TCT TTG ATG AAG TTA ATG GGA 3568
   Lys Lys Met Ala Leu Asn Ser Leu Met Ser Leu Met Lys Leu Met Gly
   1150 1155 1160
   CCC AAA CAT GTC AGT TCT GTG AGG GTG AAG ATG ATG ACC ACA CTG AGA 3616
   Pro Lys His Val Ser Ser Val Arg Val Lys Met Met Thr Thr Leu Arg
   1165 1170 1175
   ACT GGC CTT CGA TTC AAG GAT GAT TTT CCT GAA TTG TGT TGC AGA GCT 3664
   Thr Gly Leu Arg Phe Lys Asp Asp Phe Pro Glu Leu Cys Cys Arg Ala
   1180 1185 1190 1195
   TGG GAC TGC TTT GTT CGC TGC CTG GAT CAT GCT TGT CTG GGC TCC CTT 3712
   Trp Asp Cys Phe Val Arg Cys Leu Asp His Ala Cys Leu Gly Ser Leu
   1200 1205 1210
   CTC AGT CAT GTA ATA GTA GCT TTG TTA CCT CTT ATA CAC ATC CAG CCT 3760
   Leu Ser His Val Ile Val Ala Leu Leu Pro Leu Ile His Ile Gln Pro
   1215 1220 1225
   AAA GAA ACT GCA GCT ATC TTC CAC TAC CTC ATA ATT GAA AAC AGG GAT 3808
   Lys Glu Thr Ala Ala Ile Phe His Tyr Leu Ile Ile Glu Asn Arg Asp
   1230 1235 1240
   GCT GTG CAA GAT TTT CTT CAT GAA ATA TAT TTT TTA CCT GAT CAT CCA 3856
   Ala Val Gln Asp Phe Leu His Glu Ile Tyr Phe Leu Pro Asp His Pro
   1245 1250 1255
   GAA TTA AAA AAG ATA AAA GCC GTT CTC CAG GAA TAC AGA AAG GAG ACC 3904
   Glu Leu Lys Lys Ile Lys Ala Val Leu Gln Glu Tyr Arg Lys Glu Thr
   1260 1265 1270 1275
   TCT GAG AGC ACT GAT CTT CAG ACA ACT CTT CAG CTC TCT ATG AAG GCC 3952
   Ser Glu Ser Thr Asp Leu Gln Thr Thr Leu Gln Leu Ser Met Lys Ala
   1280 1285 1290
   ATT CAA CAT GAA AAT GTC GAT GTT CGT ATT CAT GCT CTT ACA AGC TTG 4000
   Ile Gln His Glu Asn Val Asp Val Arg Ile His Ala Leu Thr Ser Leu
   1295 1300 1305
   AAG GAA ACC TTG TAT AAA AAT CAG GAA AAA CTG ATA AAG TAT GCA ACA 4048
   Lys Glu Thr Leu Tyr Lys Asn Gln Glu Lys Leu Ile Lys Tyr Ala Thr
   1310 1315 1320
   GAC AGT GAA ACA GTA GAA CCT ATT ATC TCA CAG TTG GTG ACA GTG CTT 4096
   Asp Ser Glu Thr Val Glu Pro Ile Ile Ser Gln Leu Val Thr Val Leu
   1325 1330 1335
   TTG AAA GGT TGC CAA GAT GCA AAC TCT CAA GCT CGG TTG CTC TGT GGG 4144
   Leu Lys Gly Cys Gln Asp Ala Asn Ser Gln Ala Arg Leu Leu Cys Gly
   1340 1345 1350 1355
   GAA TGT TTA GGG GAA TTG GGG GCG ATA GAT CCA GGT CGA TTA GAT TTC 4192
   Glu Cys Leu Gly Glu Leu Gly Ala Ile Asp Pro Gly Arg Leu Asp Phe
   1360 1365 1370
   TCA ACA ACT GAA ACT CAA GGA AAA GAT TTT ACA TTT GTG ACT GGA GTA 4240
   Ser Thr Thr Glu Thr Gln Gly Lys Asp Phe Thr Phe Val Thr Gly Val
   1375 1380 1385
   GAA GAT TCA AGC TTT GCC TAT GGA TTA TTG ATG GAG CTA ACA AGA GCT 4288
   Glu Asp Ser Ser Phe Ala Tyr Gly Leu Leu Met Glu Leu Thr Arg Ala
   1390 1395 1400
   TAC CTT GCG TAT GCT GAT AAT AGC CGA GCT CAA GAT TCA GCT GCC TAT 4336
   Tyr Leu Ala Tyr Ala Asp Asn Ser Arg Ala Gln Asp Ser Ala Ala Tyr
   1405 1410 1415
   GCC ATT CAG GAG TTG CTT TCT ATT TAT GAC TGT AGA GAG ATG GAG ACC 4384
   Ala Ile Gln Glu Leu Leu Ser Ile Tyr Asp Cys Arg Glu Met Glu Thr
   1420 1425 1430 1435
   AAC GGC CCA GGT CAC CAA TTG TGG AGG AGA TTT CCT GAG CAT GTT CGG 4432
   Asn Gly Pro Gly His Gln Leu Trp Arg Arg Phe Pro Glu His Val Arg
   1440 1445 1450
   GAA ATA CTA GAA CCT CAT CTA AAT ACC AGA TAC AAG AGT TCT CAG AAG 4480
   Glu Ile Leu Glu Pro His Leu Asn Thr Arg Tyr Lys Ser Ser Gln Lys
   1455 1460 1465
   TCA ACC GAT TGG TCT GGA GTA AAG AAG CCA ATT TAC TTA AGT AAA TTG 4528
   Ser Thr Asp Trp Ser Gly Val Lys Lys Pro Ile Tyr Leu Ser Lys Leu
   1470 1475 1480
   GGT AGT AAC TTT GCA GAA TGG TCA GCA TCT TGG GCA GGT TAT CTT ATT 4576
   Gly Ser Asn Phe Ala Glu Trp Ser Ala Ser Trp Ala Gly Tyr Leu Ile
   1485 1490 1495
   ACA AAG GTT CGA CAT GAT CTT GCC AGT AAA ATT TTC ACC TGC TGT AGC       4624
   Thr Lys Val Arg His Asp Leu Ala Ser Lys Ile Phe Thr Cys Cys Ser
   1500                1505                1510                1515
   ATT ATG ATG AAG CAT GAT TTC AAA GTG ACC ATC TAT CTT CTT CCA CAT       4672
   Ile Met Met Lys His Asp Phe Lys Val Thr Ile Tyr Leu Leu Pro His
                   1520                1525                1530
   ATT CTG GTG TAT GTC TTA CTG GGT TGT AAT CAA GAA GAT CAG CAG GAG       4720
   Ile Leu Val Tyr Val Leu Leu Gly Cys Asn Gln Glu Asp Gln Gln Glu
               1535                1540                1545
   GTT TAT GCA GAA ATT ATG GCA GTT CTA AAG CAT GAC GAT CAG CAT ACC       4768
   Val Tyr Ala Glu Ile Met Ala Val Leu Lys His Asp Asp Gln His Thr
           1550                1555                1560
   ATA AAT ACC CAA GAC ATT GCA TCT GAT CTG TGT CAA CTC AGT ACA CAG       4816
   Ile Asn Thr Gln Asp Ile Ala Ser Asp Leu Cys Gln Leu Ser Thr Gln
       1565                1570                1575
   ACT GTG TTC TCC ATG CTT GAC CAT CTC ACA CAG TGG GCA AGG CAC AAA       4864
   Thr Val Phe Ser Met Leu Asp His Leu Thr Gln Trp Ala Arg His Lys
   1580                1585                1590                1595
   TTT CAG GCA CTG AAA GCT GAG AAA TGT CCA CAC AGC AAA TCA AAC AGA       4912
   Phe Gln Ala Leu Lys Ala Glu Lys Cys Pro His Ser Lys Ser Asn Arg
                   1600                1605                1610
   AAT AAG GTA GAC TCA ATG GTA TCT ACT GTG GAT TAT GAA GAC TAT CAG       4960
   Asn Lys Val Asp Ser Met Val Ser Thr Val Asp Tyr Glu Asp Tyr Gln
               1615                1620                1625
   AGT GTA ACC CGT TTT CTA GAC CTC ATA CCC CAG GAT ACT CTG GCA GTA       5008
   Ser Val Thr Arg Phe Leu Asp Leu Ile Pro Gln Asp Thr Leu Ala Val
           1630                1635                1640
   GCT TCC TTT CGC TCC AAA GCA TAC ACA CGA GCT GTA ATG CAC TTT GAA       5056
   Ala Ser Phe Arg Ser Lys Ala Tyr Thr Arg Ala Val Met His Phe Glu
       1645                1650                1655
   TCA TTT ATT ACA GAA AAG AAG CAA AAT ATT CAG GAA CAT CTT GGA TTT       5104
   Ser Phe Ile Thr Glu Lys Lys Gln Asn Ile Gln Glu His Leu Gly Phe
   1660                1665                1670                1675
   TTA CAG AAA TTG TAT GCT GCT ATG CAT GAA CCT GAT GGA GTG GCC GGA       5152
   Leu Gln Lys Leu Tyr Ala Ala Met His Glu Pro Asp Gly Val Ala Gly
                   1680                1685                1690
   GTC AGT GCA ATT AGA AAG GCA GAA CCA TCT CTA AAA GAA CAG ATC CTT       5200
   Val Ser Ala Ile Arg Lys Ala Glu Pro Ser Leu Lys Glu Gln Ile Leu
               1695                1700                1705
   GAA CAT GAA AGC CTT GGC TTG CTG AGG GAT GCC ACT GCT TGT TAT GAC       5248
   Glu His Glu Ser Leu Gly Leu Leu Arg Asp Ala Thr Ala Cys Tyr Asp
           1710                1715                1720
   AGG GCT ATT CAG CTA GAA CCA GAC CAG ATC ATT CAT TAT CAT GGT GTA       5296
   Arg Ala Ile Gln Leu Glu Pro Asp Gln Ile Ile His Tyr His Gly Val
       1725                1730                1735
   GTA AAG TCC ATG TTA GGT CTT GGT CAG CTG TCT ACT GTT ATC ACT CAG 5344
   Val Lys Ser Met Leu Gly Leu Gly Gln Leu Ser Thr Val Ile Thr Gln
   1740 1745 1750 1755
   GTG AAT GGA GTG CAT GCT AAC AGG TCC GAG TGG ACA GAT GAA TTA AAC 5392
   Val Asn Gly Val His Ala Asn Arg Ser Glu Trp Thr Asp Glu Leu Asn
   1760 1765 1770
   ACG TAC AGA GTG GAA GCA GCT TGG AAA TTG TCA CAG TGG GAT TTG GTG 5440
   Thr Tyr Arg Val Glu Ala Ala Trp Lys Leu Ser Gln Trp Asp Leu Val
   1775 1780 1785
   GAA AAC TAT TTG GCA GCA GAT GGA AAA TCT ACA ACA TGG AGT GTC AGA 5488
   Glu Asn Tyr Leu Ala Ala Asp Gly Lys Ser Thr Thr Trp Ser Val Arg
   1790 1795 1800
   CTG GGA CAG CTA TTA TTA TCA GCC AAA AAA AGA GAT ATC ACA GCT TTT 5536
   Leu Gly Gln Leu Leu Leu Ser Ala Lys Lys Arg Asp Ile Thr Ala Phe
   1805 1810 1815
   TAT GAC TCA CTG AAA CTA GTG AGA GCA GAA CAA ATT GTA CCT CTT TCA 5584
   Tyr Asp Ser Leu Lys Leu Val Arg Ala Glu Gln Ile Val Pro Leu Ser
   1820 1825 1830 1835
   GCT GCA AGC TTT GAA AGA GGC TCC TAC CAA CGA GGA TAT GAA TAT ATT 5632
   Ala Ala Ser Phe Glu Arg Gly Ser Tyr Gln Arg Gly Tyr Glu Tyr Ile
   1840 1845 1850
   GTG AGA TTG CAC ATG TTA TGT GAG TTG GAG CAT AGC ATC AAA CCA CTT 5680
   Val Arg Leu His Met Leu Cys Glu Leu Glu His Ser Ile Lys Pro Leu
   1855 1860 1865
   TTC CAG CAT TCT CCA GGT GAC AGT TCT CAA GAA GAT TCT CTA AAC TGG 5728
   Phe Gln His Ser Pro Gly Asp Ser Ser Gln Glu Asp Ser Leu Asn Trp
   1870 1875 1880
   GTA GCT CGA CTA GAA ATG ACC CAG AAT TCC TAC AGA GCC AAG GAG CCT 5776
   Val Ala Arg Leu Glu Met Thr Gln Asn Ser Tyr Arg Ala Lys Glu Pro
   1885 1890 1895
   ATC CTG GCT CTC CGG AGG GCT TTA CTA AGC CTC AAC AAA AGA CCA GAT 5824
   Ile Leu Ala Leu Arg Arg Ala Leu Leu Ser Leu Asn Lys Arg Pro Asp
   1900 1905 1910 1915
   TAC AAT GAA ATG GTT GGA GAA TGC TGG CTG CAG AGT GCC AGG GTA GCT 5872
   Tyr Asn Glu Met Val Gly Glu Cys Trp Leu Gln Ser Ala Arg Val Ala
   1920 1925 1930
   AGA AAG GCT GGT CAC CAC CAG ACA GCC TAC AAT GCT CTC CTT AAT GCA 5920
   Arg Lys Ala Gly His His Gln Thr Ala Tyr Asn Ala Leu Leu Asn Ala
   1935 1940 1945
   GGG GAA TCA CGA CTC GCT GAA CTG TAC GTG GAA AGG GCA AAG TGG CTC 5968
   Gly Glu Ser Arg Leu Ala Glu Leu Tyr Val Glu Arg Ala Lys Trp Leu
   1950 1955 1960
   TGG TCC AAG GGT GAT GTT CAC CAG GCA CTA ATT GTT CTT CAA AAA GGT 6016
   Trp Ser Lys Gly Asp Val His Gln Ala Leu Ile Val Leu Gln Lys Gly
   1965 1970 1975
   GTT GAA TTA TGT TTT CCT GAA AAT GAA ACC CCA CCT GAG GGT AAG AAC 6064
   Val Glu Leu Cys Phe Pro Glu Asn Glu Thr Pro Pro Glu Gly Lys Asn
   1980 1985 1990 1995
   ATG TTA ATC CAT GGT CGA GCT ATG CTA CTA GTG GGC CGA TTT ATG GAA 6112
   Met Leu Ile His Gly Arg Ala Met Leu Leu Val Gly Arg Phe Met Glu
   2000 2005 2010
   GAA ACA GCT AAC TTT GAA AGC AAT GCA ATT ATG AAA AAA TAT AAG GAT 6160
   Glu Thr Ala Asn Phe Glu Ser Asn Ala Ile Met Lys Lys Tyr Lys Asp
   2015 2020 2025
   GTG ACC GCG TGC CTG CCA GAA TGG GAG GAT GGG CAT TTT TAC CTT GCC 6208
   Val Thr Ala Cys Leu Pro Glu Trp Glu Asp Gly His Phe Tyr Leu Ala
   2030 2035 2040
   AAG TAC TAT GAC AAA TTG ATG CCC ATG GTC ACA GAC AAC AAA ATG GAA 6256
   Lys Tyr Tyr Asp Lys Leu Met Pro Met Val Thr Asp Asn Lys Met Glu
   2045 2050 2055
   AAG CAA GGT GAT CTC ATC CGG TAT ATA GTT CTT CAT TTT GGC AGA TCT 6304
   Lys Gln Gly Asp Leu Ile Arg Tyr Ile Val Leu His Phe Gly Arg Ser
   2060 2065 2070 2075
   CTA CAA TAT GGA AAT CAG TTC ATA TAT CAG TCA ATG CCA CGA ATG TTA 6352
   Leu Gln Tyr Gly Asn Gln Phe Ile Tyr Gln Ser Met Pro Arg Met Leu
   2080 2085 2090
   ACT CTA TGG CTT GAT TAT GGT ACA AAG GCA TAT GAA TGG GAA AAA GCT 6400
   Thr Leu Trp Leu Asp Tyr Gly Thr Lys Ala Tyr Glu Trp Glu Lys Ala
   2095 2100 2105
   GGC CGC TCC GAT CGT GTA CAA ATG AGG AAT GAT TTG GGT AAA ATA AAC 6448
   Gly Arg Ser Asp Arg Val Gln Met Arg Asn Asp Leu Gly Lys Ile Asn
   2110 2115 2120
   AAG GTT ATC ACA GAG CAT ACA AAC TAT TTA GCT CCA TAT CAA TTT TTG 6496
   Lys Val Ile Thr Glu His Thr Asn Tyr Leu Ala Pro Tyr Gln Phe Leu
   2125 2130 2135
   ACT GCT TTT TCA CAA TTG ATC TCT CGA ATT TGT CAT TCT CAC GAT GAA 6544
   Thr Ala Phe Ser Gln Leu Ile Ser Arg Ile Cys His Ser His Asp Glu
   2140 2145 2150 2155
   GTT TTT GTT GTC TTG ATG GAA ATA ATA GCC AAA GTA TTT CTA GCC TAT 6592
   Val Phe Val Val Leu Met Glu Ile Ile Ala Lys Val Phe Leu Ala Tyr
   2160 2165 2170
   CCT CAA CAA GCA ATG TGG ATG ATG ACA GCT GTG TCA AAG TCA TCT TAT 6640
   Pro Gln Gln Ala Met Trp Met Met Thr Ala Val Ser Lys Ser Ser Tyr
   2175 2180 2185
   CCC ATG CGT GTG AAC AGA TGC AAG GAA ATC CTC AAT AAA GCT ATT CAT 6688
   Pro Met Arg Val Asn Arg Cys Lys Glu Ile Leu Asn Lys Ala Ile His
   2190 2195 2200
   ATG AAA AAA TCC TTA GAG AAG TTT GTT GGA GAT GCA ACT CGC CTA ACA 6736
   Met Lys Lys Ser Leu Glu Lys Phe Val Gly Asp Ala Thr Arg Leu Thr
   2205 2210 2215
   GAT AAG CTT CTA GAA TTG TGC AAT AAA CCG GTT GAT GGA AGT AGT TCC 6784
   Asp Lys Leu Leu Glu Leu Cys Asn Lys Pro Val Asp Gly Ser Ser Ser
   2220 2225 2230 2235
   ACA TTA AGC ATG AGC ACT CAT TTT AAA ATG CTT AAA AAG CTG GTA GAA 6832
   Thr Leu Ser Met Ser Thr His Phe Lys Met Leu Lys Lys Leu Val Glu
   2240 2245 2250
   GAA GCA ACA TTT AGT GAA ATC CTC ATT CCT CTA CAA TCA GTC ATG ATA 6880
   Glu Ala Thr Phe Ser Glu Ile Leu Ile Pro Leu Gln Ser Val Met Ile
   2255 2260 2265
   CCT ACA CTT CCA TCA ATT CTG GGT ACC CAT GCT AAC CAT GCT AGC CAT 6928
   Pro Thr Leu Pro Ser Ile Leu Gly Thr His Ala Asn His Ala Ser His
   2270 2275 2280
   GAA CCA TTT CCT GGA CAT TGG GCC TAT ATT GCA GGG TTT GAT GAT ATG 6976
   Glu Pro Phe Pro Gly His Trp Ala Tyr Ile Ala Gly Phe Asp Asp Met
   2285 2290 2295
   GTG GAA ATT CTT GCT TCT CTT CAG AAA CCA AAG AAG ATT TCT TTA AAA 7024
   Val Glu Ile Leu Ala Ser Leu Gln Lys Pro Lys Lys Ile Ser Leu Lys
   2300 2305 2310 2315
   GGC TCA GAT GGA AAG TTC TAC ATC ATG ATG TGT AAG CCA AAA GAT GAC 7072
   Gly Ser Asp Gly Lys Phe Tyr Ile Met Met Cys Lys Pro Lys Asp Asp
   2320 2325 2330
   CTG AGA AAG GAT TGT AGA CTA ATG GAA TTC AAT TCC TTG ATT AAT AAG 7120
   Leu Arg Lys Asp Cys Arg Leu Met Glu Phe Asn Ser Leu Ile Asn Lys
   2335 2340 2345
   TGC TTA AGA AAA GAT GCA GAG TCT CGT AGA AGA GAA CTT CAT ATT CGA 7168
   Cys Leu Arg Lys Asp Ala Glu Ser Arg Arg Arg Glu Leu His Ile Arg
   2350 2355 2360
   ACA TAT GCA GTT ATT CCA CTA AAT GAT GAA TGT GGG ATT ATT GAA TGG 7216
   Thr Tyr Ala Val Ile Pro Leu Asn Asp Glu Cys Gly Ile Ile Glu Trp
   2365 2370 2375
   GTG AAC AAC ACT GCT GGT TTG AGA CCT ATT CTG ACC AAA CTA TAT AAA 7264
   Val Asn Asn Thr Ala Gly Leu Arg Pro Ile Leu Thr Lys Leu Tyr Lys
   2380 2385 2390 2395
   GAA AAG GGA GTG TAT ATG ACA GGA AAA GAA CTT CGC CAG TGT ATG CTA 7312
   Glu Lys Gly Val Tyr Met Thr Gly Lys Glu Leu Arg Gln Cys Met Leu
   2400 2405 2410
   CCA AAG TCA GCA GCT TTA TCT GAA AAA CTC AAA GTA TTC CGA GAA TTT 7360
   Pro Lys Ser Ala Ala Leu Ser Glu Lys Leu Lys Val Phe Arg Glu Phe
   2415 2420 2425
   CTC CTG CCC AGG CAT CCT CCT ATT TTT CAT GAG TGG TTT CTG AGA ACA 7408
   Leu Leu Pro Arg His Pro Pro Ile Phe His Glu Trp Phe Leu Arg Thr
   2430 2435 2440
   TTC CCT GAT CCT ACA TCA TGG TAC AGT AGT AGA TCA GCT TAC TGC CGT 7456
   Phe Pro Asp Pro Thr Ser Trp Tyr Ser Ser Arg Ser Ala Tyr Cys Arg
   2445 2450 2455
   TCC ACT GCA GTA ATG TCA ATG GTT GGT TAT ATT CTG GGG CTT GGA GAC      7504
   Ser Thr Ala Val Met Ser Met Val Gly Tyr Ile Leu Gly Leu Gly Asp
   2460                2465                2470                2475
   CGT CAT GGT GAA AAT ATT CTC TTT GAT TCT TTG ACT GGT GAA TGC GTA      7552
   Arg His Gly Glu Asn Ile Leu Phe Asp Ser Leu Thr Gly Glu Cys Val
                   2480                2485                2490
   CAT GTA GAT TTC AAT TGT CTT TTC AAT AAG GGA GAA ACC TTT GAA GTT      7600
   His Val Asp Phe Asn Cys Leu Phe Asn Lys Gly Glu Thr Phe Glu Val
               2495                2500                2505
   CCA GAA ATT GTG CCA TTT CGC CTG ACT CAT AAT ATG GTT AAT GGA ATG      7648
   Pro Glu Ile Val Pro Phe Arg Leu Thr His Asn Met Val Asn Gly Met
           2510                2515                2520
   GGT CCT ATG GGA ACA GAG GGT CTT TTT CGA AGA GCA TGT GAA GTT ACA      7696
   Gly Pro Met Gly Thr Glu Gly Leu Phe Arg Arg Ala Cys Glu Val Thr
       2525                2530                2535
   ATG AGG CTG ATG CGT GAT CAG CGA GAG CCT TTA ATG AGT GTC TTA AAG      7744
   Met Arg Leu Met Arg Asp Gln Arg Glu Pro Leu Met Ser Val Leu Lys
   2540                2545                2550                2555
   ACT TTT CTA CAT GAT CCT CTT GTG GAA TGG AGT AAA CCA GTG AAA GGG      7792
   Thr Phe Leu His Asp Pro Leu Val Glu Trp Ser Lys Pro Val Lys Gly
                   2560                2565                2570
   CAT TCC AAA GCG CCA CTG AAT GAA ACT GGA GAA GTT GTC AAT GAA AAG      7840
   His Ser Lys Ala Pro Leu Asn Glu Thr Gly Glu Val Val Asn Glu Lys
               2575                2580                2585
   GCC AAG ACC CAT GTT CTT GAC ATT GAG CAG CGA CTA CAA GGT GTA ATC      7888
   Ala Lys Thr His Val Leu Asp Ile Glu Gln Arg Leu Gln Gly Val Ile
           2590                2595                2600
   AAG ACT CGA AAT AGA GTG ACA GGA CTG CCG TTA TCT ATT GAA GGA CAT      7936
   Lys Thr Arg Asn Arg Val Thr Gly Leu Pro Leu Ser Ile Glu Gly His
       2605                2610                2615
   GTG CAT TAC CTT ATA CAA GAA GCT ACT GAT GAA AAC TTA CTA TGC CAG      7984
   Val His Tyr Leu Ile Gln Glu Ala Thr Asp Glu Asn Leu Leu Cys Gln
   2620                2625                2630                2635
   ATG TAT CTT GGT TGG ACT CCA TAT ATG TGA AATGAAATTA TGTAAAAGAA         8034
   Met Tyr Leu Gly Trp Thr Pro Tyr Met  *
                   2640                2645
   TATGTTAATA ATCTAAAAGT AATGCATTTG GTATGAATCT GTGGTTGTAT CTGTTCAATT    8094
   CTAAAGTACA ACATAAATTT ACGTTCTCAG CAACTGTTAT TTCTCTCTGA TCATTAATTA    8154
   TATGTAAAAT AATATACATT CAGTTATTAA GAAATAAACT GCTTTCTTAA TAAAAAAAAA    8214
   AAAAAAAAAA AAAAAAAAAA AAAAA                                          8239
(2) INFORMATION FOR SEQ ID NO: 2:
   (i) SEQUENCE CHARACTERISTICS:
      (A) LENGTH: 2645 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Gly Glu His Gly Leu Glu Leu Ala Ser Met Ile Pro Ala Leu Arg
    1               5                  10                  15
   Glu Leu Gly Ser Ala Thr Pro Glu Glu Tyr Asn Thr Val Val Gln Lys
               20                  25                  30
   Pro Arg Gln Ile Leu Cys Gln Phe Ile Asp Arg Ile Leu Thr Asp Val
           35                  40                  45
   Asn Val Val Ala Val Glu Leu Val Lys Lys Thr Asp Ser Gln Pro Thr
       50                  55                  60
   Ser Val Met Leu Leu Asp Phe Ile Gln His Ile Met Lys Ser Ser Pro
   65                  70                  75                  80
   Leu Met Phe Val Asn Val Ser Gly Ser His Glu Arg Lys Gly Ser Cys
                   85                  90                  95
   Ile Glu Phe Ser Asn Trp Ile Ile Thr Arg Leu Leu Arg Ile Ala Ala
               100                 105                 110
   Thr Pro Ser Cys His Leu Leu His Lys Lys Ile Cys Glu Val Ile Cys
           115                 120                 125
   Ser Leu Leu Phe Leu Phe Lys Ser Lys Ser Pro Ala Ile Phe Gly Val
       130                 135                 140
   Leu Thr Lys Glu Leu Leu Gln Leu Phe Glu Asp Leu Val Tyr Leu His
   145                 150                 155                 160
   Arg Arg Asn Val Met Gly His Ala Val Glu Trp Pro Val Val Met Ser
                   165                 170                 175
   Arg Phe Leu Ser Gln Leu Asp Glu His Met Gly Tyr Leu Gln Ser Ala
               180                 185                 190
   Pro Leu Gln Leu Met Ser Met Gln Asn Leu Glu Phe Ile Glu Val Thr
           195                 200                 205
   Leu Leu Met Val Leu Thr Arg Ile Ile Ala Ile Val Phe Phe Arg Arg
       210                 215                 220
   Gln Glu Leu Leu Leu Trp Gln Ile Gly Cys Val Leu Leu Glu Tyr Gly
   225                 230                 235                 240
   Ser Pro Lys Ile Lys Ser Leu Ala Ile Ser Phe Leu Thr Glu Leu Phe
                   245                 250                 255
   Gln Leu Gly Gly Leu Pro Ala Gln Pro Ala Ser Thr Phe Phe Ser Ser
               260                 265                 270
   Phe Leu Glu Leu Leu Lys His Leu Val Glu Met Asp Thr Asp Gln Leu
   275 280 285
   Lys Leu Tyr Glu Glu Pro Leu Ser Lys Leu Ile Lys Thr Leu Phe Pro
   290 295 300
   Phe Glu Ala Glu Ala Tyr Arg Asn Ile Glu Pro Val Tyr Leu Asn Met
   305 310 315 320
   Leu Leu Glu Lys Leu Cys Val Met Phe Glu Asp Gly Val Leu Met Arg
   325 330 335
   Leu Lys Ser Asp Leu Leu Lys Ala Ala Leu Cys His Leu Leu Gln Tyr
   340 345 350
   Phe Leu Lys Phe Val Pro Ala Gly Tyr Glu Ser Ala Leu Gln Val Arg
   355 360 365
   Lys Val Tyr Val Arg Asn Ile Cys Lys Ala Leu Leu Asp Val Leu Gly
   370 375 380
   Ile Glu Val Asp Ala Glu Tyr Leu Leu Gly Pro Leu Tyr Ala Ala Leu
   385 390 395 400
   Lys Met Glu Ser Met Glu Ile Ile Glu Glu Ile Gln Cys Gln Thr Gln
   405 410 415
   Gln Glu Asn Leu Ser Ser Asn Ser Asp Gly Ile Ser Pro Lys Arg Arg
   420 425 430
   Arg Leu Ser Ser Ser Leu Asn Pro Ser Lys Arg Ala Pro Lys Gln Thr
   435 440 445
   Glu Glu Ile Lys His Val Asp Met Asn Gln Lys Ser Ile Leu Trp Ser
   450 455 460
   Ala Leu Lys Gln Lys Ala Glu Ser Leu Gln Ile Ser Leu Glu Tyr Ser
   465 470 475 480
   Gly Leu Lys Asn Pro Val Ile Glu Met Leu Glu Gly Ile Ala Val Val
   485 490 495
   Leu Gln Leu Thr Ala Leu Cys Thr Val His Cys Ser His Gln Asn Met
   500 505 510
   Asn Cys Arg Thr Phe Lys Asp Cys Gln His Lys Ser Lys Lys Lys Pro
   515 520 525
   Ser Val Val Ile Thr Trp Met Ser Leu Asp Phe Tyr Thr Lys Val Leu
   530 535 540
   Lys Ser Cys Arg Ser Leu Leu Glu Ser Val Gln Lys Leu Asp Leu Glu
   545 550 555 560
   Ala Thr Ile Asp Lys Val Val Lys Ile Tyr Asp Ala Leu Ile Tyr Met
   565 570 575
   Gln Val Asn Ser Ser Phe Glu Asp His Ile Leu Glu Asp Leu Cys Gly
   580 585 590
   Met Leu Ser Leu Pro Trp Ile Tyr Ser His Ser Asp Asp Gly Cys Leu
   595 600 605
   Lys Leu Thr Thr Phe Ala Ala Asn Leu Leu Thr Leu Ser Cys Arg Ile
   610 615 620
   Ser Asp Ser Tyr Ser Pro Gln Ala Gln Ser Arg Cys Val Phe Leu Leu
   625 630 635 640
   Thr Leu Phe Pro Arg Arg Ile Phe Leu Glu Trp Arg Thr Ala Val Tyr
   645 650 655
   Asn Trp Ala Leu Gln Ser Ser His Glu Val Ile Arg Ala Ser Cys Val
   660 665 670
   Ser Gly Phe Phe Ile Leu Leu Gln Gln Gln Asn Ser Cys Asn Arg Val
   675 680 685
   Pro Lys Ile Leu Ile Asp Lys Val Lys Asp Asp Ser Asp Ile Val Lys
   690 695 700
   Lys Glu Phe Ala Ser Ile Leu Gly Gln Leu Val Cys Thr Leu His Gly
   705 710 715 720
   Met Phe Tyr Leu Thr Ser Ser Leu Thr Glu Pro Phe Ser Glu His Gly
   725 730 735
   His Val Asp Leu Phe Cys Arg Asn Leu Lys Ala Thr Ser Gln His Glu
   740 745 750
   Cys Ser Ser Ser Gln Leu Lys Ala Ser Val Cys Lys Pro Phe Leu Phe
   755 760 765
   Leu Leu Lys Lys Lys Ile Pro Ser Pro Val Lys Leu Ala Phe Ile Asp
   770 775 780
   Asn Leu His His Leu Cys Lys His Leu Asp Phe Arg Glu Asp Glu Thr
   785 790 795 800
   Asp Val Lys Ala Val Leu Gly Thr Leu Leu Asn Leu Met Glu Asp Pro
   805 810 815
   Asp Lys Asp Val Arg Val Ala Phe Ser Gly Asn Ile Lys His Ile Leu
   820 825 830
   Glu Ser Leu Asp Ser Glu Asp Gly Phe Ile Lys Glu Leu Phe Val Leu
   835 840 845
   Arg Met Lys Glu Ala Tyr Thr His Ala Gln Ile Ser Arg Asn Asn Glu
   850 855 860
   Leu Lys Asp Thr Leu Ile Leu Thr Thr Gly Asp Ile Gly Arg Ala Ala
   865 870 875 880
   Lys Gly Asp Leu Val Pro Phe Ala Leu Leu His Leu Leu His Cys Leu
   885 890 895
   Leu Ser Lys Ser Ala Ser Val Ser Gly Ala Ala Tyr Thr Glu Ile Arg
   900 905 910
   Ala Leu Val Ala Ala Lys Ser Val Lys Leu Gln Ser Phe Phe Ser Gln
   915 920 925
   Tyr Lys Lys Pro Ile Cys Gln Phe Leu Val Glu Ser Leu His Ser Ser
   930 935 940
   Gln Met Thr Ala Leu Pro Asn Thr Pro Cys Gln Asn Ala Asp Val Arg
   945 950 955 960
   Lys Gln Asp Val Ala His Gln Arg Glu Met Ala Leu Asn Thr Leu Ser
   965 970 975
   Glu Ile Ala Asn Val Phe Asp Phe Pro Asp Leu Asn Arg Phe Leu Thr
   980 985 990
   Arg Thr Leu Gln Val Leu Leu Pro Asp Leu Ala Ala Lys Ala Ser Pro
   995 1000 1005
   Ala Ala Ser Ala Leu Ile Arg Thr Leu Gly Lys Gln Leu Asn Val Asn
   1010 1015 1020
   Arg Arg Glu Ile Leu Ile Asn Asn Phe Lys Tyr Ile Phe Ser His Leu
   1025 1030 1035 1040
   Val Cys Ser Cys Ser Lys Asp Glu Leu Glu Arg Ala Leu His Tyr Leu
   1045 1050 1055
   Lys Asn Glu Thr Glu Ile Glu Leu Gly Ser Leu Leu Arg Gln Asp Phe
   1060 1065 1070
   Gln Gly Leu His Asn Glu Leu Leu Leu Arg Ile Gly Glu His Tyr Gln
   1075 1080 1085
   Gln Val Phe Asn Gly Leu Ser Ile Leu Ala Ser Phe Ala Ser Ser Asp
   1090 1095 1100
   Asp Pro Tyr Gln Gly Pro Arg Asp Ile Ile Ser Pro Glu Leu Met Ala
   1105 1110 1115 1120
   Asp Tyr Leu Gln Pro Lys Leu Leu Gly Ile Leu Ala Phe Phe Asn Met
   1125 1130 1135
   Gln Leu Leu Ser Ser Ser Val Gly Ile Glu Asp Lys Lys Met Ala Leu
   1140 1145 1150
   Asn Ser Leu Met Ser Leu Met Lys Leu Met Gly Pro Lys His Val Ser
   1155 1160 1165
   Ser Val Arg Val Lys Met Met Thr Thr Leu Arg Thr Gly Leu Arg Phe
   1170 1175 1180
   Lys Asp Asp Phe Pro Glu Leu Cys Cys Arg Ala Trp Asp Cys Phe Val
   1185 1190 1195 1200
   Arg Cys Leu Asp His Ala Cys Leu Gly Ser Leu Leu Ser His Val Ile
   1205 1210 1215
   Val Ala Leu Leu Pro Leu Ile His Ile Gln Pro Lys Glu Thr Ala Ala
   1220 1225 1230
   Ile Phe His Tyr Leu Ile Ile Glu Asn Arg Asp Ala Val Gln Asp Phe
           1235                1240                1245
   Leu His Glu Ile Tyr Phe Leu Pro Asp His Pro Glu Leu Lys Lys Ile
       1250                1255                1260
   Lys Ala Val Leu Gln Glu Tyr Arg Lys Glu Thr Ser Glu Ser Thr Asp
   1265                1270                1275                1280
   Leu Gln Thr Thr Leu Gln Leu Ser Met Lys Ala Ile Gln His Glu Asn
                   1285                1290                1295
   Val Asp Val Arg Ile His Ala Leu Thr Ser Leu Lys Glu Thr Leu Tyr
               1300                1305                1310
   Lys Asn Gln Glu Lys Leu Ile Lys Tyr Ala Thr Asp Ser Glu Thr Val
           1315                1320                1325
   Glu Pro Ile Ile Ser Gln Leu Val Thr Val Leu Leu Lys Gly Cys Gln
       1330                1335                1340
   Asp Ala Asn Ser Gln Ala Arg Leu Leu Cys Gly Glu Cys Leu Gly Glu
   1345                1350                1355                1360
   Leu Gly Ala Ile Asp Pro Gly Arg Leu Asp Phe Ser Thr Thr Glu Thr
                   1365                1370                1375
   Gln Gly Lys Asp Phe Thr Phe Val Thr Gly Val Glu Asp Ser Ser Phe
               1380                1385                1390
   Ala Tyr Gly Leu Leu Met Glu Leu Thr Arg Ala Tyr Leu Ala Tyr Ala
           1395                1400                1405
   Asp Asn Ser Arg Ala Gln Asp Ser Ala Ala Tyr Ala Ile Gln Glu Leu
       1410                1415                1420
   Leu Ser Ile Tyr Asp Cys Arg Glu Met Glu Thr Asn Gly Pro Gly His
   1425                1430                1435                1440
   Gln Leu Trp Arg Arg Phe Pro Glu His Val Arg Glu Ile Leu Glu Pro
                   1445                1450                1455
   His Leu Asn Thr Arg Tyr Lys Ser Ser Gln Lys Ser Thr Asp Trp Ser
               1460                1465                1470
   Gly Val Lys Lys Pro Ile Tyr Leu Ser Lys Leu Gly Ser Asn Phe Ala
           1475                1480                1485
   Glu Trp Ser Ala Ser Trp Ala Gly Tyr Leu Ile Thr Lys Val Arg His
       1490                1495                1500
   Asp Leu Ala Ser Lys Ile Phe Thr Cys Cys Ser Ile Met Met Lys His
   1505                1510                1515                1520
   Asp Phe Lys Val Thr Ile Tyr Leu Leu Pro His Ile Leu Val Tyr Val
                   1525                1530                1535
   Leu Leu Gly Cys Asn Gln Glu Asp Gln Gln Glu Val Tyr Ala Glu Ile
               1540                1545                1550
   Met Ala Val Leu Lys His Asp Asp Gln His Thr Ile Asn Thr Gln Asp
   1555 1560 1565
   Ile Ala Ser Asp Leu Cys Gln Leu Ser Thr Gln Thr Val Phe Ser Met
   1570 1575 1580
   Leu Asp His Leu Thr Gln Trp Ala Arg His Lys Phe Gln Ala Leu Lys
   1585 1590 1595 1600
   Ala Glu Lys Cys Pro His Ser Lys Ser Asn Arg Asn Lys Val Asp Ser
   1605 1610 1615
   Met Val Ser Thr Val Asp Tyr Glu Asp Tyr Gln Ser Val Thr Arg Phe
   1620 1625 1630
   Leu Asp Leu Ile Pro Gln Asp Thr Leu Ala Val Ala Ser Phe Arg Ser
   1635 1640 1645
   Lys Ala Tyr Thr Arg Ala Val Met His Phe Glu Ser Phe Ile Thr Glu
   1650 1655 1660
   Lys Lys Gln Asn Ile Gln Glu His Leu Gly Phe Leu Gln Lys Leu Tyr
   1665 1670 1675 1680
   Ala Ala Met His Glu Pro Asp Gly Val Ala Gly Val Ser Ala Ile Arg
   1685 1690 1695
   Lys Ala Glu Pro Ser Leu Lys Glu Gln Ile Leu Glu His Glu Ser Leu
   1700 1705 1710
   Gly Leu Leu Arg Asp Ala Thr Ala Cys Tyr Asp Arg Ala Ile Gln Leu
   1715 1720 1725
   Glu Pro Asp Gln Ile Ile His Tyr His Gly Val Val Lys Ser Met Leu
   1730 1735 1740
   Gly Leu Gly Gln Leu Ser Thr Val Ile Thr Gln Val Asn Gly Val His
   1745 1750 1755 1760
   Ala Asn Arg Ser Glu Trp Thr Asp Glu Leu Asn Thr Tyr Arg Val Glu
   1765 1770 1775
   Ala Ala Trp Lys Leu Ser Gln Trp Asp Leu Val Glu Asn Tyr Leu Ala
   1780 1785 1790
   Ala Asp Gly Lys Ser Thr Thr Trp Ser Val Arg Leu Gly Gln Leu Leu
   1795 1800 1805
   Leu Ser Ala Lys Lys Arg Asp Ile Thr Ala Phe Tyr Asp Ser Leu Lys
   1810 1815 1820
   Leu Val Arg Ala Glu Gln Ile Val Pro Leu Ser Ala Ala Ser Phe Glu
   1825 1830 1835 1840
   Arg Gly Ser Tyr Gln Arg Gly Tyr Glu Tyr Ile Val Arg Leu His Met
   1845 1850 1855
   Leu Cys Glu Leu Glu His Ser Ile Lys Pro Leu Phe Gln His Ser Pro
   1860 1865 1870
   Gly Asp Ser Ser Gln Glu Asp Ser Leu Asn Trp Val Ala Arg Leu Glu
   1875 1880 1885
   Met Thr Gln Asn Ser Tyr Arg Ala Lys Glu Pro Ile Leu Ala Leu Arg
   1890 1895 1900
   Arg Ala Leu Leu Ser Leu Asn Lys Arg Pro Asp Tyr Asn Glu Met Val
   1905 1910 1915 1920
   Gly Glu Cys Trp Leu Gln Ser Ala Arg Val Ala Arg Lys Ala Gly His
   1925 1930 1935
   His Gln Thr Ala Tyr Asn Ala Leu Leu Asn Ala Gly Glu Ser Arg Leu
   1940 1945 1950
   Ala Glu Leu Tyr Val Glu Arg Ala Lys Trp Leu Trp Ser Lys Gly Asp
   1955 1960 1965
   Val His Gln Ala Leu Ile Val Leu Gln Lys Gly Val Glu Leu Cys Phe
   1970 1975 1980
   Pro Glu Asn Glu Thr Pro Pro Glu Gly Lys Asn Met Leu Ile His Gly
   1985 1990 1995 2000
   Arg Ala Met Leu Leu Val Gly Arg Phe Met Glu Glu Thr Ala Asn Phe
   2005 2010 2015
   Glu Ser Asn Ala Ile Met Lys Lys Tyr Lys Asp Val Thr Ala Cys Leu
   2020 2025 2030
   Pro Glu Trp Glu Asp Gly His Phe Tyr Leu Ala Lys Tyr Tyr Asp Lys
   2035 2040 2045
   Leu Met Pro Met Val Thr Asp Asn Lys Met Glu Lys Gln Gly Asp Leu
   2050 2055 2060
   Ile Arg Tyr Ile Val Leu His Phe Gly Arg Ser Leu Gln Tyr Gly Asn
   2065 2070 2075 2080
   Gln Phe Ile Tyr Gln Ser Met Pro Arg Met Leu Thr Leu Trp Leu Asp
   2085 2090 2095
   Tyr Gly Thr Lys Ala Tyr Glu Trp Glu Lys Ala Gly Arg Ser Asp Arg
   2100 2105 2110
   Val Gln Met Arg Asn Asp Leu Gly Lys Ile Asn Lys Val Ile Thr Glu
   2115 2120 2125
   His Thr Asn Tyr Leu Ala Pro Tyr Gln Phe Leu Thr Ala Phe Ser Gln
   2130 2135 2140
   Leu Ile Ser Arg Ile Cys His Ser His Asp Glu Val Phe Val Val Leu
   2145 2150 2155 2160
   Met Glu Ile Ile Ala Lys Val Phe Leu Ala Tyr Pro Gln Gln Ala Met
   2165 2170 2175
   Trp Met Met Thr Ala Val Ser Lys Ser Ser Tyr Pro Met Arg Val Asn
   2180 2185 2190
   Arg Cys Lys Glu Ile Leu Asn Lys Ala Ile His Met Lys Lys Ser Leu
   2195 2200 2205
   Glu Lys Phe Val Gly Asp Ala Thr Arg Leu Thr Asp Lys Leu Leu Glu
   2210 2215 2220
   Leu Cys Asn Lys Pro Val Asp Gly Ser Ser Ser Thr Leu Ser Met Ser
   2225 2230 2235 2240
   Thr His Phe Lys Met Leu Lys Lys Leu Val Glu Glu Ala Thr Phe Ser
   2245 2250 2255
   Glu Ile Leu Ile Pro Leu Gln Ser Val Met Ile Pro Thr Leu Pro Ser
   2260 2265 2270
   Ile Leu Gly Thr His Ala Asn His Ala Ser His Glu Pro Phe Pro Gly
   2275 2280 2285
   His Trp Ala Tyr Ile Ala Gly Phe Asp Asp Met Val Glu Ile Leu Ala
   2290 2295 2300
   Ser Leu Gln Lys Pro Lys Lys Ile Ser Leu Lys Gly Ser Asp Gly Lys
   2305 2310 2315 2320
   Phe Tyr Ile Met Met Cys Lys Pro Lys Asp Asp Leu Arg Lys Asp Cys
   2325 2330 2335
   Arg Leu Met Glu Phe Asn Ser Leu Ile Asn Lys Cys Leu Arg Lys Asp
   2340 2345 2350
   Ala Glu Ser Arg Arg Arg Glu Leu His Ile Arg Thr Tyr Ala Val Ile
   2355 2360 2365
   Pro Leu Asn Asp Glu Cys Gly Ile Ile Glu Trp Val Asn Asn Thr Ala
   2370 2375 2380
   Gly Leu Arg Pro Ile Leu Thr Lys Leu Tyr Lys Glu Lys Gly Val Tyr
   2385 2390 2395 2400
   Met Thr Gly Lys Glu Leu Arg Gln Cys Met Leu Pro Lys Ser Ala Ala
   2405 2410 2415
   Leu Ser Glu Lys Leu Lys Val Phe Arg Glu Phe Leu Leu Pro Arg His
   2420 2425 2430
   Pro Pro Ile Phe His Glu Trp Phe Leu Arg Thr Phe Pro Asp Pro Thr
   2435 2440 2445
   Ser Trp Tyr Ser Ser Arg Ser Ala Tyr Cys Arg Ser Thr Ala Val Met
   2450 2455 2460
   Ser Met Val Gly Tyr Ile Leu Gly Leu Gly Asp Arg His Gly Glu Asn
   2465 2470 2475 2480
   Ile Leu Phe Asp Ser Leu Thr Gly Glu Cys Val His Val Asp Phe Asn
   2485 2490 2495
   Cys Leu Phe Asn Lys Gly Glu Thr Phe Glu Val Pro Glu Ile Val Pro
   2500 2505 2510
   Phe Arg Leu Thr His Asn Met Val Asn Gly Met Gly Pro Met Gly Thr
   2515 2520 2525
   Glu Gly Leu Phe Arg Arg Ala Cys Glu Val Thr Met Arg Leu Met Arg
   2530 2535 2540
   Asp Gln Arg Glu Pro Leu Met Ser Val Leu Lys Thr Phe Leu His Asp
   2545 2550 2555 2560
   Pro Leu Val Glu Trp Ser Lys Pro Val Lys Gly His Ser Lys Ala Pro
   2565 2570 2575
   Leu Asn Glu Thr Gly Glu Val Val Asn Glu Lys Ala Lys Thr His Val
   2580 2585 2590
   Leu Asp Ile Glu Gln Arg Leu Gln Gly Val Ile Lys Thr Arg Asn Arg
   2595 2600 2605
   Val Thr Gly Leu Pro Leu Ser Ile Glu Gly His Val His Tyr Leu Ile
   2610 2615 2620
   Gln Glu Ala Thr Asp Glu Asn Leu Leu Cys Gln Met Tyr Leu Gly Trp
   2625 2630 2635 2640
   Thr Pro Tyr Met *
   2645
(2) INFORMATION FOR SEQ ID NO: 3:
   (i) SEQUENCE CHARACTERISTICS:
      (A) LENGTH: 8022 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: S. pombe
   (ix) FEATURE:
      (A) NAME/KEY: CDS
      (B) LOCATION:585..7745
   (xi) SEQUENCE DESCRIPTION: SEQ ID NO: 3:
   GGTACCAAGT AAAAACTGCT TAGTAAGTAT AAAACACAGA AGAATCCGCG ATCTAGTGAA    60
   CCAATGCCCT GCGTATGACG CTCCACTGAC GCTATAGTCA ATGAGAACTA GGATGTGCGA   120
   TTATAACTTA TCTTTTCAAT ATTTTCTTAT TATTTATTTA AGAAATAATT GAATTAAAAC   180
   TCATTTCTTC TTTTATTAGC CGTAAAATAG CTTATTTTCT CTCCTACTAC CTTTCAACAA   240
   TAACTTTTTT TTTTGTTTAT TGACCATTAT AATCACATCA AAAGTCAAAA AATTCAATCA   300
   TTATCAGAAA CATCCAGCCT AATATTACTT AAAAGTTAGT TTCCTCTGAA AATTCAGTAT   360
   CACAAAAGCT CGTTAATTAG CATCGCTCGA TACTTAGTGC ACCATGCATC TTCCTTTACC 420
   TCGTGAGTGG AAATCGATTT GATAATCGAT TGCCACTTTT CGCATAATTC TATTGAGATA 480
   TTTTATTACT TACAATCGTC TTTTATAAAT GCTCAAGACT TTGAACGCGC GTGTTGCGTT 540
   TTAAAAAGGC CTTTTTTTGA ATTGAATCAA TGGTTTGATA TAGT ATG AGC CAA CAC 596
   Met Ser Gln His
   1
   GCA AAA AGG AAA GCT GGG TCA CTC GAT CTT TCA CCC AGA GGC TTA GAT 644
   Ala Lys Arg Lys Ala Gly Ser Leu Asp Leu Ser Pro Arg Gly Leu Asp
   5 10 15 20
   GAC AGA CAG GCT TTC GGA CAG CTT TTG AAA GAA GTA TTA GCA TTA GAC 692
   Asp Arg Gln Ala Phe Gly Gln Leu Leu Lys Glu Val Leu Ala Leu Asp
   25 30 35
   AAA GAA CAT GAG TTA GGT AGA AGT AAT TCT TTA CCA TCT ATG ACC TCC 740
   Lys Glu His Glu Leu Gly Arg Ser Asn Ser Leu Pro Ser Met Thr Ser
   40 45 50
   GAG CTT GTT GAA GTT TTA ATT GAA GTT GGT CTT CTA GCT TTT AAA CAT 788
   Glu Leu Val Glu Val Leu Ile Glu Val Gly Leu Leu Ala Phe Lys His
   55 60 65
   GAT GAT TCA AAA TCT GAA TTT ATC TCT CCT AAG ATG CTA AAA GAA GCC 836
   Asp Asp Ser Lys Ser Glu Phe Ile Ser Pro Lys Met Leu Lys Glu Ala
   70 75 80
   CAT CTC TCT CTA CAA GCG TTA ATG CTA ATC TTA AAA AGG TCT CCG ACA 884
   His Leu Ser Leu Gln Ala Leu Met Leu Ile Leu Lys Arg Ser Pro Thr
   85 90 95 100
   GTT TTG CGG GAG ATT AAA TCA TCT GTT ACT CTT TTG GAT TGG ATT TTA 932
   Val Leu Arg Glu Ile Lys Ser Ser Val Thr Leu Leu Asp Trp Ile Leu
   105 110 115
   CCC AGG ACT ATA TCA TTG TTT GCT GAT ATT CGT TTT ATT AAG TTA TTT 980
   Pro Arg Thr Ile Ser Leu Phe Ala Asp Ile Arg Phe Ile Lys Leu Phe
   120 125 130
   GAC TCA TTA AAA GAG TTT CAT AAG CTA ATT TAT CAG CTA ATC AGT GAA 1028
   Asp Ser Leu Lys Glu Phe His Lys Leu Ile Tyr Gln Leu Ile Ser Glu
   135 140 145
   AAG TCA TTC CTA TGG GAC TTA TAT GCT TCG TTT ATG CGT TAT TGG AAA 1076
   Lys Ser Phe Leu Trp Asp Leu Tyr Ala Ser Phe Met Arg Tyr Trp Lys
   150 155 160
   TAT TAT ATT ACA AAC GTT TCT TCT ATA GTT CTC CAA ATC ACT AAT GCT 1124
   Tyr Tyr Ile Thr Asn Val Ser Ser Ile Val Leu Gln Ile Thr Asn Ala
   165 170 175 180
   ACA TTC CCT TAC AAG ATG CCC TCA CCC AAT TCT CAA CCA TTG CAG AGT 1172
   Thr Phe Pro Tyr Lys Met Pro Ser Pro Asn Ser Gln Pro Leu Gln Ser
   185 190 195
   ATC TCC CCA AAT TAT CCA ACC CAT CGA GAG GAC AAA TTT GAT TTA CTT    1220
   Ile Ser Pro Asn Tyr Pro Thr His Arg Glu Asp Lys Phe Asp Leu Leu
               200                 205                 210
   ATC ATT AAT ATA GAG GAG GCT TGT ACA TTT TTC TTT GAA AGT GCC CAT    1268
   Ile Ile Asn Ile Glu Glu Ala Cys Thr Phe Phe Phe Glu Ser Ala His
           215                 220                 225
   TTT TTT GCA CAA TGC TCA TAT TTA AAG AAA TCC AAT TTT CCT AGT CCA    1316
   Phe Phe Ala Gln Cys Ser Tyr Leu Lys Lys Ser Asn Phe Pro Ser Pro
       230                 235                 240
   CCT CTC TTT ACA GCG TGG ACT TGG ATC AAG CCA TGT TTT TTT AAT TTT    1364
   Pro Leu Phe Thr Ala Trp Thr Trp Ile Lys Pro Cys Phe Phe Asn Phe
   245                 250                 255                 260
   GTT ATT TTA TTA AAA CGA ATC AGC ATC GGA GAC TCA CAG CTC TTT CTA    1412
   Val Ile Leu Leu Lys Arg Ile Ser Ile Gly Asp Ser Gln Leu Phe Leu
                   265                 270                 275
   CAT TTG CAT TCA CGT ATA GTC CAA ACT TTA TGC TGT TTT TCC TTG AAT    1460
   His Leu His Ser Arg Ile Val Gln Thr Leu Cys Cys Phe Ser Leu Asn
               280                 285                 290
   TTT ATA TAT CAT GGC CTT CCC ATT TGT GAA AAA TCT AAA CAT ATT TTA    1508
   Phe Ile Tyr His Gly Leu Pro Ile Cys Glu Lys Ser Lys His Ile Leu
           295                 300                 305
   ATG TCC TCC ATC AAC TTA ACA TTG GGA TCA TTG AAG AAA ACT TAT ACA    1556
   Met Ser Ser Ile Asn Leu Thr Leu Gly Ser Leu Lys Lys Thr Tyr Thr
       310                 315                 320
   GTT GCT AAT ACT GCT ATA TCT CTT TTT TTT CTC TCT TTA TTT GTT TTA    1604
   Val Ala Asn Thr Ala Ile Ser Leu Phe Phe Leu Ser Leu Phe Val Leu
   325                 330                 335                 340
   CCC AAA ACT GTA GCT GGT CTA TTC TAT CCT TTT GGG GTT TCC TTA CTT    1652
   Pro Lys Thr Val Ala Gly Leu Phe Tyr Pro Phe Gly Val Ser Leu Leu
                   345                 350                 355
   TCT GAC TTC AAG GTA TTA GAG CAA CTT GAA CCA GAT TCT GAT CTC AAA    1700
   Ser Asp Phe Lys Val Leu Glu Gln Leu Glu Pro Asp Ser Asp Leu Lys
               360                 365                 370
   AAG GCA ATA ATA TTA TTT AAG TGC AGA TAC CAA AGT TCA GAA ATA GAT    1748
   Lys Ala Ile Ile Leu Phe Lys Cys Arg Tyr Gln Ser Ser Glu Ile Asp
           375                 380                 385
   CAA ACA ACT CTC CGT GCT TTT GGC GAA ATT TGT ACT GGT AAA CTT GAA    1796
   Gln Thr Thr Leu Arg Ala Phe Gly Glu Ile Cys Thr Gly Lys Leu Glu
       390                 395                 400
   AAC ACG TTG TTT TCT AAC TCT GAA TTA AAC CTT TTT CTT TTA CAT TAT    1844
   Asn Thr Leu Phe Ser Asn Ser Glu Leu Asn Leu Phe Leu Leu His Tyr
   405                 410                 415                 420
   CTT TCC TTG GAC AAT GAC TTG TCA AAT ATT CTT AAA GTG GAT TTC CAG    1892
   Leu Ser Leu Asp Asn Asp Leu Ser Asn Ile Leu Lys Val Asp Phe Gln
                   425                 430                 435
   AAT GGT CAT AAC ATA TGT ACA TTT GCA AAA TGG TGT ATA AAC AAC AAC 1940
   Asn Gly His Asn Ile Cys Thr Phe Ala Lys Trp Cys Ile Asn Asn Asn
   440 445 450
   TTA GAT GAA CCG TCT AAT TTA AAG CAC TTT CGT GAA ATG TTA GAT TAT 1988
   Leu Asp Glu Pro Ser Asn Leu Lys His Phe Arg Glu Met Leu Asp Tyr
   455 460 465
   TAT AGC TCT CAT AAT GTT ACA ATA AGT GAG GAC GAC CTG AAG AAC TTC 2036
   Tyr Ser Ser His Asn Val Thr Ile Ser Glu Asp Asp Leu Lys Asn Phe
   470 475 480
   TCT TTA GTT TTG TGT ACT CAT GTT GCA AAG GTG AAT GAG AAA ACA AAT 2084
   Ser Leu Val Leu Cys Thr His Val Ala Lys Val Asn Glu Lys Thr Asn
   485 490 495 500
   AGT ATT TTC CGC ACA TAT GAA GTA CAT GGT TGT GAA GTT TGT AAC TCA 2132
   Ser Ile Phe Arg Thr Tyr Glu Val His Gly Cys Glu Val Cys Asn Ser
   505 510 515
   TTT TGT TTA CTA TTT GAT GAG CGG TCG CCT TTT AAA ATT CCT TAT CAC 2180
   Phe Cys Leu Leu Phe Asp Glu Arg Ser Pro Phe Lys Ile Pro Tyr His
   520 525 530
   GAA TTG TTT TGT GCA TTG CTA AAA AAT CCC GAC ATA ATT TCC TCT TCT 2228
   Glu Leu Phe Cys Ala Leu Leu Lys Asn Pro Asp Ile Ile Ser Ser Ser
   535 540 545
   GTT AAA CAA TCA TTG TTG CTT GAT GGC TTT TTT CGG TGG AGC CAG CAT 2276
   Val Lys Gln Ser Leu Leu Leu Asp Gly Phe Phe Arg Trp Ser Gln His
   550 555 560
   TGC TCA AAC TTT AAT AAA GAA TCA ATG TTA AGT TTA AGA GAA TTT ATT 2324
   Cys Ser Asn Phe Asn Lys Glu Ser Met Leu Ser Leu Arg Glu Phe Ile
   565 570 575 580
   ATG AAA GCA TTA GCC AGT ACT TCA AGA TGT TTA CGT GTT GTT GCT GCA 2372
   Met Lys Ala Leu Ala Ser Thr Ser Arg Cys Leu Arg Val Val Ala Ala
   585 590 595
   AAA GTT TTG CCC ATT TTC ATT AAG GGA CCT AAT AAT CTT GAT ATA GTT 2420
   Lys Val Leu Pro Ile Phe Ile Lys Gly Pro Asn Asn Leu Asp Ile Val
   600 605 610
   GAA TTT CAC AAG GAA AGT AAA GCC TTG ATT TTT AAT ACG TTG AAA ATA 2468
   Glu Phe His Lys Glu Ser Lys Ala Leu Ile Phe Asn Thr Leu Lys Ile
   615 620 625
   TTG GCG GTG GAA AAT ACA GCT ATT TTA GAA ACG GTC ATT CTT TCC TGG 2516
   Leu Ala Val Glu Asn Thr Ala Ile Leu Glu Thr Val Ile Leu Ser Trp
   630 635 640
   ATC TCC TTA TCT AGA GTG GTA GAA GAA GAA GAA TTA CAT TTT GTA CTA 2564
   Ile Ser Leu Ser Arg Val Val Glu Glu Glu Glu Leu His Phe Val Leu
   645 650 655 660
   TTG GAA GTT ATA TCT TCT GTG ATA AAC AGC GGA ATA TTT TAT CAA GGC 2612
   Leu Glu Val Ile Ser Ser Val Ile Asn Ser Gly Ile Phe Tyr Gln Gly
   665 670 675
   ATT GGT CTC AGC GCT CTG CAA CAA ATT GCC TCG ACG CGT CAT ATA TCC      2660
   Ile Gly Leu Ser Ala Leu Gln Gln Ile Ala Ser Thr Arg His Ile Ser
               680                 685                 690
   GTT TGG CAA TTA CTT TCT CCA TAT TGG CCA ACA GTG TCC GTT GCG ATT      2708
   Val Trp Gln Leu Leu Ser Pro Tyr Trp Pro Thr Val Ser Val Ala Ile
           695                 700                 705
   GTC CAA GGT ATG GGT AAA AAA CCG AAC ATA GCC AGT TTA TTT GCT CAG      2756
   Val Gln Gly Met Gly Lys Lys Pro Asn Ile Ala Ser Leu Phe Ala Gln
       710                 715                 720
   CTT ATG AAT ATT TCC GAG GGC GAT TTT CTT ATT CGA ACA CAG GCG TAC      2804
   Leu Met Asn Ile Ser Glu Gly Asp Phe Leu Ile Arg Thr Gln Ala Tyr
   725                 730                 735                 740
   ACT TTA CCA TTC CTT GTA CTT ACT AAA AAC AAA GCG TTA ATA GTA CGT      2852
   Thr Leu Pro Phe Leu Val Leu Thr Lys Asn Lys Ala Leu Ile Val Arg
                   745                 750                 755
   ATA GCT GAA CTT TCA CAA AGT GAT GTT GCT ACT TTG TGC CTT ACC AAT      2900
   Ile Ala Glu Leu Ser Gln Ser Asp Val Ala Thr Leu Cys Leu Thr Asn
               760                 765                 770
   ATG CAT AAA ATC CTT GCT TCG CTA CTT ACT ACG GAT CAT CCT AAT TTG      2948
   Met His Lys Ile Leu Ala Ser Leu Leu Thr Thr Asp His Pro Asn Leu
           775                 780                 785
   GAA GAG AGT GTG ATG CTT CTT CTT TCA CTG GCC ACT TCT GAT TTT GAA      2996
   Glu Glu Ser Val Met Leu Leu Leu Ser Leu Ala Thr Ser Asp Phe Glu
       790                 795                 800
   AAA GTT GAT TTA ACG TCT TTG TTA CGC TCT GAT CCT ATT TCT ATT ACT      3044
   Lys Val Asp Leu Thr Ser Leu Leu Arg Ser Asp Pro Ile Ser Ile Thr
   805                 810                 815                 820
   GTG GAG TTG TTA CAG CTT TAT CAG AAT GAT GTT CCT CAT GAA AAA ATT      3092
   Val Glu Leu Leu Gln Leu Tyr Gln Asn Asp Val Pro His Glu Lys Ile
                   825                 830                 835
   GAA AAT GCT TTA AGA AAG GTA GCA ATG ATT GTC TCT CAA GTG GTT AAT      3140
   Glu Asn Ala Leu Arg Lys Val Ala Met Ile Val Ser Gln Val Val Asn
               840                 845                 850
   GAC GAA GAC TTG AGC AAT AAG GAA TTA CTT TAT GAT TTT TTT AAT AAT      3188
   Asp Glu Asp Leu Ser Asn Lys Glu Leu Leu Tyr Asp Phe Phe Asn Asn
           855                 860                 865
   CAC ATT TTG GGT ATC TTA GCA GAA TTT TCT AAT ATC CTT AAC GAC CTG      3236
   His Ile Leu Gly Ile Leu Ala Glu Phe Ser Asn Ile Leu Asn Asp Leu
       870                 875                 880
   AAA GGA AAG ACT TCA ATT AAT GAA AAG ATT AAG ACA ATT GTC GGC ATT      3284
   Lys Gly Lys Thr Ser Ile Asn Glu Lys Ile Lys Thr Ile Val Gly Ile
   885                 890                 895                 900
   GAA AAA ATG TTA TCT TTA TGT GGA GGT GCA GTC AAA CTT GGA TTA CCA      3332
   Glu Lys Met Leu Ser Leu Cys Gly Gly Ala Val Lys Leu Gly Leu Pro
                   905                 910                 915
   CAG ATA CTT TCT AAT TTA CAA AGT GCT TTT CAA AAT GAG CAC TTA AGG 3380
   Gln Ile Leu Ser Asn Leu Gln Ser Ala Phe Gln Asn Glu His Leu Arg
   920 925 930
   TTT TAT GCA ATC AAA GCT TGG TTC AGT TTG ATA TTA GCA ACC AAG GAG 3428
   Phe Tyr Ala Ile Lys Ala Trp Phe Ser Leu Ile Leu Ala Thr Lys Glu
   935 940 945
   CCC GAG TAT AGT TCA ATT GCT GGT TTA AGT CTT GTA ATT TTA CCT CCT 3476
   Pro Glu Tyr Ser Ser Ile Ala Gly Leu Ser Leu Val Ile Leu Pro Pro
   950 955 960
   TTA TTC CCT TAT TTA GAA CCA CAA GAA GCA GAG CTA GTA ATT CAA ATA 3524
   Leu Phe Pro Tyr Leu Glu Pro Gln Glu Ala Glu Leu Val Ile Gln Ile
   965 970 975 980
   TTT GAT TTT ATT TCT TCT GAC ACA CAC AAG TGC CTA CAA GGA TTA AAG 3572
   Phe Asp Phe Ile Ser Ser Asp Thr His Lys Cys Leu Gln Gly Leu Lys
   985 990 995
   TGG GCT ATC CCC ACC AGT CTG GAT TCA GCG TGC TTT AGC CTT AAG GCT 3620
   Trp Ala Ile Pro Thr Ser Leu Asp Ser Ala Cys Phe Ser Leu Lys Ala
   1000 1005 1010
   AAA GAA ATA TTC TGT TCG CTT CAA AAT GAA GAT TTT TAC TCT GAG CTT 3668
   Lys Glu Ile Phe Cys Ser Leu Gln Asn Glu Asp Phe Tyr Ser Glu Leu
   1015 1020 1025
   CAA AGT ATA ATT AAG TGT TTA ACT AAC GAA AAT GAG CCA GTT TGT TAT 3716
   Gln Ser Ile Ile Lys Cys Leu Thr Asn Glu Asn Glu Pro Val Cys Tyr
   1030 1035 1040
   TTA GGT TTA CAA AAA TTA GAA CTT TTT TTT CAA GCC AAG GTG GAC GAG 3764
   Leu Gly Leu Gln Lys Leu Glu Leu Phe Phe Gln Ala Lys Val Asp Glu
   1045 1050 1055 1060
   TTA CAT GAC ACA CTA AAT TTG GAC ATA TCC AAC GAA GTT CTG GAC CAA 3812
   Leu His Asp Thr Leu Asn Leu Asp Ile Ser Asn Glu Val Leu Asp Gln
   1065 1070 1075
   TTA CTA AGA TGC CTT TTA GAT TGT TGT GTA AAA TAT GCT TCA ACA AAT 3860
   Leu Leu Arg Cys Leu Leu Asp Cys Cys Val Lys Tyr Ala Ser Thr Asn
   1080 1085 1090
   ATG CAA ATA TCA TAT CTT GCT GCA AAA AAT CTT GGT GAA TTG GGT GCG 3908
   Met Gln Ile Ser Tyr Leu Ala Ala Lys Asn Leu Gly Glu Leu Gly Ala
   1095 1100 1105
   ATA GAT CCC AGC CGC GCC AAG GCT CAA CAT ATT ATT AAA GAA ACA GTT 3956
   Ile Asp Pro Ser Arg Ala Lys Ala Gln His Ile Ile Lys Glu Thr Val
   1110 1115 1120
   GTT CTT GAT AAC TTT GAA AAC GGA GAA GAA AGT TTG AAG TTT ATT CTA 4004
   Val Leu Asp Asn Phe Glu Asn Gly Glu Glu Ser Leu Lys Phe Ile Leu
   1125 1130 1135 1140
   GAT TTT ATG CAA TCG CAG TTA ATT CCA GCT TTC CTT GTT ACT ACT GAT 4052
   Asp Phe Met Gln Ser Gln Leu Ile Pro Ala Phe Leu Val Thr Thr Asp
   1145 1150 1155
   ACT AAA GCA CAA GGT TTT CTT GCC TAT GCT CTG CAA GAG TTT CTA AAG    4100
   Thr Lys Ala Gln Gly Phe Leu Ala Tyr Ala Leu Gln Glu Phe Leu Lys
               1160                1165                1170
   CTT GGT GGA TTC AAG TCC GCA GTG ATT AAT AAA AAA AAG GGA CTA ACT    4148
   Leu Gly Gly Phe Lys Ser Ala Val Ile Asn Lys Lys Lys Gly Leu Thr
           1175                1180                1185
   GTG GTA ACA GAA CAT TGG ATG TCT TTG CCT GAT TTA TCC AAA CGT GTG    4196
   Val Val Thr Glu His Trp Met Ser Leu Pro Asp Leu Ser Lys Arg Val
       1190                1195                1200
   CTT ATA CCA TTT TTA ACT TCC AAG TAT CAT TTA ACA CCA ATC CCC AAA    4244
   Leu Ile Pro Phe Leu Thr Ser Lys Tyr His Leu Thr Pro Ile Pro Lys
   1205                1210                1215                1220
   ATT GAC ATT CGG TAC CCT ATT TAT AAA GAA AAT GTT ACT ATT CAT ACT    4292
   Ile Asp Ile Arg Tyr Pro Ile Tyr Lys Glu Asn Val Thr Ile His Thr
                   1225                1230                1235
   TGG ATG CAG TTG TTT TCT CTT AAA TTG ATG GAG TAC GCC CAT TCG CAA    4340
   Trp Met Gln Leu Phe Ser Leu Lys Leu Met Glu Tyr Ala His Ser Gln
               1240                1245                1250
   AAC GCT GAA AAA ATA TTT GGT ATT TGT TCG AAA GTA GTG AAA GAC CAA    4388
   Asn Ala Glu Lys Ile Phe Gly Ile Cys Ser Lys Val Val Lys Asp Gln
           1255                1260                1265
   GAG GTT AAC ATT CCC TGT TTT CTT CTT CCC TTT CTT GTT TTA AAT GTT    4436
   Glu Val Asn Ile Pro Cys Phe Leu Leu Pro Phe Leu Val Leu Asn Val
       1270                1275                1280
   ATT TTA ACC GAG TCA GAA CTG GAA GTT AAT AAA GTC ATT GAA GAA TTC    4484
   Ile Leu Thr Glu Ser Glu Leu Glu Val Asn Lys Val Ile Glu Glu Phe
   1285                1290                1295                1300
   CAG CTT GTT ATT AAT CAA CCG GGA CCT GAT GGA TTA AAT TCC GTG GGG    4532
   Gln Leu Val Ile Asn Gln Pro Gly Pro Asp Gly Leu Asn Ser Val Gly
                   1305                1310                1315
   CAA CAA AGA TAC ACC TCA TTT GTA GAT GTA TTT TTT AAG ATT GTG GAT    4580
   Gln Gln Arg Tyr Thr Ser Phe Val Asp Val Phe Phe Lys Ile Val Asp
               1320                1325                1330
   TAC CTT AAC AAA TGG CTT CGC ATG CGA AAG AAG AGG AAT TGG GAT AGA    4628
   Tyr Leu Asn Lys Trp Leu Arg Met Arg Lys Lys Arg Asn Trp Asp Arg
           1335                1340                1345
   CGT TCT GCC ATT GCA AGG AAA GAG AAC CGT TAT ATG TCG GTG GAA GAT    4676
   Arg Ser Ala Ile Ala Arg Lys Glu Asn Arg Tyr Met Ser Val Glu Asp
       1350                1355                1360
   GCT ACC TCT CGA GAA TCA TCG ATC TCA AAA GTT GAG TCA TTT CTT TCT    4724
   Ala Thr Ser Arg Glu Ser Ser Ile Ser Lys Val Glu Ser Phe Leu Ser
   1365                1370                1375                1380
   CGA TTT CCT TCA AAA ACA TTA GGT ATT GTC TCT TTA AAT TGT GGA TTT    4772
   Arg Phe Pro Ser Lys Thr Leu Gly Ile Val Ser Leu Asn Cys Gly Phe
                   1385                1390                1395
   CAT GCT CGT GCA TTG TTT TAT TGG GAG CAA CAC ATA CGT AAT GCT ACA      4820
   His Ala Arg Ala Leu Phe Tyr Trp Glu Gln His Ile Arg Asn Ala Thr
               1400                1405                1410
   GCT CCA TAT GCA GCT TTA GAG TCC GAT TAT AGA GTT TTG CAG GAA ATA      4868
   Ala Pro Tyr Ala Ala Leu Glu Ser Asp Tyr Arg Val Leu Gln Glu Ile
           1415                1420                1425
   TAT GCT GGA ATT GAT GAT CCA GAT GAA ATC GAA GCA GTG TCT TTA AAT      4916
   Tyr Ala Gly Ile Asp Asp Pro Asp Glu Ile Glu Ala Val Ser Leu Asn
       1430                1435                1440
   TTC CAT GAT TAC TCG TTT GAT CAA CAA CTC CTT TTA CAT GAA AAT TCA      4964
   Phe His Asp Tyr Ser Phe Asp Gln Gln Leu Leu Leu His Glu Asn Ser
   1445                1450                1455                1460
   GGA ACA TGG GAC TCG GCT TTG AGT TGT TAC GAA ATT ATT ATT CAA AAG      5012
   Gly Thr Trp Asp Ser Ala Leu Ser Cys Tyr Glu Ile Ile Ile Gln Lys
                   1465                1470                1475
   GAT CCT GAA AAT AAA AAG GCG AAA ATC GGT TTG CTT AAC AGC ATG CTG      5060
   Asp Pro Glu Asn Lys Lys Ala Lys Ile Gly Leu Leu Asn Ser Met Leu
               1480                1485                1490
   CAA TCG GGG CAT TAT GAA TCT CTT GTT TTG AGT TTA GAT TCT TTT ATA      5108
   Gln Ser Gly His Tyr Glu Ser Leu Val Leu Ser Leu Asp Ser Phe Ile
           1495                1500                1505
   ATC AAT GAC AAC CAC GAG TAT TCG AAG ATG TTA AAT TTG GGT ATT GAA      5156
   Ile Asn Asp Asn His Glu Tyr Ser Lys Met Leu Asn Leu Gly Ile Glu
       1510                1515                1520
   GCT TCA TGG CGT TCG CTA TCT ATT GAT TCG TTA AAA AAG TGT CTT TCA      5204
   Ala Ser Trp Arg Ser Leu Ser Ile Asp Ser Leu Lys Lys Cys Leu Ser
   1525                1530                1535                1540
   AAA AGC AAC TTG GAA TCT TTC GAA GCT AAA TTG GGT AGC ATA TTT TAC      5252
   Lys Ser Asn Leu Glu Ser Phe Glu Ala Lys Leu Gly Ser Ile Phe Tyr
                   1545                1550                1555
   CAA TAC CTA CGG AAG GAT TCT TTT GCT GAA TTG ACG GAG CGG CTG CAA      5300
   Gln Tyr Leu Arg Lys Asp Ser Phe Ala Glu Leu Thr Glu Arg Leu Gln
               1560                1565                1570
   CCC TTG TAC GTT GAT GCT GCT ACA GCA ATT GCA AAC ACA GGC GCC CAT      5348
   Pro Leu Tyr Val Asp Ala Ala Thr Ala Ile Ala Asn Thr Gly Ala His
           1575                1580                1585
   TCA GCC TAT GAT TGT TAT GAT ATT TTA TCT AAG CTG CAC GCA ATT AAT      5396
   Ser Ala Tyr Asp Cys Tyr Asp Ile Leu Ser Lys Leu His Ala Ile Asn
       1590                1595                1600
   GAC TTT AGT AGG ATT GCT GAA ACT GAC GGA ATT GTT TCC GAC AAT CTT      5444
   Asp Phe Ser Arg Ile Ala Glu Thr Asp Gly Ile Val Ser Asp Asn Leu
   1605                1610                1615                1620
   GAT ATT GTT CTT CGC CGT CGG CTT AGC CAA GTA GCT CCG TAC GGT AAA      5492
   Asp Ile Val Leu Arg Arg Arg Leu Ser Gln Val Ala Pro Tyr Gly Lys
                   1625                1630                1635
   TTC AAG CAC CAA ATC CTG TCC ACT CAC TTA GTT GGC TAT GAA AAA TTT 5540
   Phe Lys His Gln Ile Leu Ser Thr His Leu Val Gly Tyr Glu Lys Phe
   1640 1645 1650
   GAA AAC ACG AAG AAA ACT GCT GAA ATA TAT CTC GAG ATT GCA AGA ATA 5588
   Glu Asn Thr Lys Lys Thr Ala Glu Ile Tyr Leu Glu Ile Ala Arg Ile
   1655 1660 1665
   TCT CGA AAA AAT GGT CAA TTT CAA AGA GCC TTC AAT GCC ATC CTC AAA 5636
   Ser Arg Lys Asn Gly Gln Phe Gln Arg Ala Phe Asn Ala Ile Leu Lys
   1670 1675 1680
   GCA ATG GAT TTA GAT AAA CCG CTA GCA ACA ATA GAG CAC GCA CAA TGG 5684
   Ala Met Asp Leu Asp Lys Pro Leu Ala Thr Ile Glu His Ala Gln Trp
   1685 1690 1695 1700
   TGG TGG CAT CAA GGG CAA CAT CGT AAA GCT ATT TCT GAA TTG AAT TTT 5732
   Trp Trp His Gln Gly Gln His Arg Lys Ala Ile Ser Glu Leu Asn Phe
   1705 1710 1715
   TCG CTT AAT AAC AAC ATG TTT GAT TTG GTT GAT GAG CAT GAA GAA AGA 5780
   Ser Leu Asn Asn Asn Met Phe Asp Leu Val Asp Glu His Glu Glu Arg
   1720 1725 1730
   CCT AAA AAT CGT AAA GAA ACT TTA GGA AAT CCA CTT AAA GGA AAA GTG 5828
   Pro Lys Asn Arg Lys Glu Thr Leu Gly Asn Pro Leu Lys Gly Lys Val
   1735 1740 1745
   TTC TTG AAA CTT ACA AAA TGG CTC GGA AAA GCT GGC CAA CTG GGA TTG 5876
   Phe Leu Lys Leu Thr Lys Trp Leu Gly Lys Ala Gly Gln Leu Gly Leu
   1750 1755 1760
   AAG GAT TTG GAG ACG TAT TAT CAT AAA GCG GTA GAG ATT TAC TCA GAA 5924
   Lys Asp Leu Glu Thr Tyr Tyr His Lys Ala Val Glu Ile Tyr Ser Glu
   1765 1770 1775 1780
   TGT GAG AAT ACG CAT TAT TAT CTT GGC CAT CAT CGA GTT TTA ATG TAT 5972
   Cys Glu Asn Thr His Tyr Tyr Leu Gly His His Arg Val Leu Met Tyr
   1785 1790 1795
   GAA GAA GAA CAA AAG CTC CCA GTT AAT GAA CAG AGC GAA CGA TTT TTA 6020
   Glu Glu Glu Gln Lys Leu Pro Val Asn Glu Gln Ser Glu Arg Phe Leu
   1800 1805 1810
   AGT GGT GAG TTA GTA ACT CGC ATA ATT AAC GAA TTT GGT CGA TCT TTG 6068
   Ser Gly Glu Leu Val Thr Arg Ile Ile Asn Glu Phe Gly Arg Ser Leu
   1815 1820 1825
   TAC TAT GGT ACA AAT CAT ATA TAT GAA AGT ATG CCA AAA TTG CTC ACA 6116
   Tyr Tyr Gly Thr Asn His Ile Tyr Glu Ser Met Pro Lys Leu Leu Thr
   1830 1835 1840
   CTG TGG CTT GAT TTT GGG GCC GAA GAA CTT CGC TTA TCT AAA GAT GAC 6164
   Leu Trp Leu Asp Phe Gly Ala Glu Glu Leu Arg Leu Ser Lys Asp Asp
   1845 1850 1855 1860
   GGC GAA AAG TAC TTT CGT GAA CAC ATT ATC TCT TCG AGA AAA AAA TCT 6212
   Gly Glu Lys Tyr Phe Arg Glu His Ile Ile Ser Ser Arg Lys Lys Ser
   1865 1870 1875
   TTG GAA CTT ATG AAT TCG AAT GTT TGT CGC CTT TCT ATG AAA ATT CCT 6260
   Leu Glu Leu Met Asn Ser Asn Val Cys Arg Leu Ser Met Lys Ile Pro
   1880 1885 1890
   CAA TAC TTT TTT CTG GTT GCA TTA TCC CAA ATG ATA TCC AGA GTA TGC 6308
   Gln Tyr Phe Phe Leu Val Ala Leu Ser Gln Met Ile Ser Arg Val Cys
   1895 1900 1905
   CAT CCA AAT AAT AAA GTT TAT AAA ATT TTG GAA CAT ATA ATT GCA AAC 6356
   His Pro Asn Asn Lys Val Tyr Lys Ile Leu Glu His Ile Ile Ala Asn
   1910 1915 1920
   GTT GTA GCA TCT TAT CCT GGG GAG ACG CTA TGG CAA TTA ATG GCA ACA 6404
   Val Val Ala Ser Tyr Pro Gly Glu Thr Leu Trp Gln Leu Met Ala Thr
   1925 1930 1935 1940
   ATA AAA TCG ACT TCT CAA AAG CGC TCG CTT CGT GGA AAA AGC ATT TTA 6452
   Ile Lys Ser Thr Ser Gln Lys Arg Ser Leu Arg Gly Lys Ser Ile Leu
   1945 1950 1955
   AAT GTT TTA CAT TCT AGG AAG CTT TCT ATG TCT TCC AAA GTT GAT ATA 6500
   Asn Val Leu His Ser Arg Lys Leu Ser Met Ser Ser Lys Val Asp Ile
   1960 1965 1970
   AAA GCA CTC AGT CAA TCT GCA ATT CTC ATT ACT GAA AAG TTA ATC AAT 6548
   Lys Ala Leu Ser Gln Ser Ala Ile Leu Ile Thr Glu Lys Leu Ile Asn
   1975 1980 1985
   TTG TGC AAT ACA AGG ATT AAC AGT AAA TCT GTA AAA ATG AGC TTA AAG 6596
   Leu Cys Asn Thr Arg Ile Asn Ser Lys Ser Val Lys Met Ser Leu Lys
   1990 1995 2000
   GAT CAT TTT CGG CTT TCT TTT GAT GAT CCG GTA GAT TTA GTC ATT CCT 6644
   Asp His Phe Arg Leu Ser Phe Asp Asp Pro Val Asp Leu Val Ile Pro
   2005 2010 2015 2020
   GCT AAA TCA TTT TTA GAC ATT ACT TTA CCA GCT AAA GAT GCT AAC AGA 6692
   Ala Lys Ser Phe Leu Asp Ile Thr Leu Pro Ala Lys Asp Ala Asn Arg
   2025 2030 2035
   GCT AGT CAT TAT CCA TTT CCA AAA ACT CAG CCT ACT CTG TTG AAA TTT 6740
   Ala Ser His Tyr Pro Phe Pro Lys Thr Gln Pro Thr Leu Leu Lys Phe
   2040 2045 2050
   GAG GAT GAG GTG GAT ATA ATG AAC TCT CTT CAA AAA CCA AGA AAA GTG 6788
   Glu Asp Glu Val Asp Ile Met Asn Ser Leu Gln Lys Pro Arg Lys Val
   2055 2060 2065
   TAC GTT AGA GGT ACG GAT GGC AAC TTA TAC CCA TTC TTG TGC AAA CCC 6836
   Tyr Val Arg Gly Thr Asp Gly Asn Leu Tyr Pro Phe Leu Cys Lys Pro
   2070 2075 2080
   AAA GAT GAT CTT CGT AAG GAT GCT AGA TTG ATG GAA TTT AAT AAT CTT 6884
   Lys Asp Asp Leu Arg Lys Asp Ala Arg Leu Met Glu Phe Asn Asn Leu
   2085 2090 2095 2100
   ATT TGT AAA ATA TTG AGG AAA GAT CAA GAA GCG AAC AGA AGG AAC TTG 6932
   Ile Cys Lys Ile Leu Arg Lys Asp Gln Glu Ala Asn Arg Arg Asn Leu
   2105 2110 2115
   TGT ATT AGA ACT TAT GTT GTT ATT CCT TTA AAT GAA GAA TGC GGA TTT 6980
   Cys Ile Arg Thr Tyr Val Val Ile Pro Leu Asn Glu Glu Cys Gly Phe
   2120 2125 2130
   ATC GAA TGG GTA AAT CAT ACT CGT CCA TTT AGA GAA ATT TTG TTA AAA 7028
   Ile Glu Trp Val Asn His Thr Arg Pro Phe Arg Glu Ile Leu Leu Lys
   2135 2140 2145
   AGC TAT AGA CAG AAA AAC ATT CCC ATA TCA TAT CAA GAA ATC AAA GTT 7076
   Ser Tyr Arg Gln Lys Asn Ile Pro Ile Ser Tyr Gln Glu Ile Lys Val
   2150 2155 2160
   GAT TTA GAC TTT GCA CTG CGA AGT CCT AAC CCT GGT GAT ATA TTT GAA 7124
   Asp Leu Asp Phe Ala Leu Arg Ser Pro Asn Pro Gly Asp Ile Phe Glu
   2165 2170 2175 2180
   AAG AAA ATC TTA CCG AAA TTT CCT CCA GTT TTT TAT GAG TGG TTT GTT 7172
   Lys Lys Ile Leu Pro Lys Phe Pro Pro Val Phe Tyr Glu Trp Phe Val
   2185 2190 2195
   GAA TCT TTC CCA GAA CCA AAT AAT TGG GTT ACT AGT AGA CAA AAC TAT 7220
   Glu Ser Phe Pro Glu Pro Asn Asn Trp Val Thr Ser Arg Gln Asn Tyr
   2200 2205 2210
   TGC CGA ACT TTA GCA GTA ATG TCA ATA GTT GGC TAC GTT TTG GGT TTG 7268
   Cys Arg Thr Leu Ala Val Met Ser Ile Val Gly Tyr Val Leu Gly Leu
   2215 2220 2225
   GGA GAT CGC CAT GGC GAA AAC ATA TTG TTT GAT GAA TTT ACA GGT GAA 7316
   Gly Asp Arg His Gly Glu Asn Ile Leu Phe Asp Glu Phe Thr Gly Glu
   2230 2235 2240
   GCT ATC CAT GTC GAT TTC AAC TGT CTT TTT GAT AAA GGT CTT ACT TTT 7364
   Ala Ile His Val Asp Phe Asn Cys Leu Phe Asp Lys Gly Leu Thr Phe
   2245 2250 2255 2260
   GAA AAA CCT GAA AAG GTG CCG TTC AGA TTA ACT CAT AAT ATG GTA GAT 7412
   Glu Lys Pro Glu Lys Val Pro Phe Arg Leu Thr His Asn Met Val Asp
   2265 2270 2275
   GCA ATG GGT CCG ACA GGT TAT GAA GGG GGT TTC AGG AAA GCT AGC GAA 7460
   Ala Met Gly Pro Thr Gly Tyr Glu Gly Gly Phe Arg Lys Ala Ser Glu
   2280 2285 2290
   ATA ACG ATG CGG CTT CTT CGC TCA AAC CAA GAT ACA TTG ATG AGC GTA 7508
   Ile Thr Met Arg Leu Leu Arg Ser Asn Gln Asp Thr Leu Met Ser Val
   2295 2300 2305
   CTA GAG TCT TTC CTA CAT GAT CCT TTA GTC GAG TGG AAT AGA AAG AAG 7556
   Leu Glu Ser Phe Leu His Asp Pro Leu Val Glu Trp Asn Arg Lys Lys
   2310 2315 2320
   TCG TCA AGC AAG TAC CCG AAT AAT GAA GCA AAT GAA GTT TTG GAT ATA 7604
   Ser Ser Ser Lys Tyr Pro Asn Asn Glu Ala Asn Glu Val Leu Asp Ile
   2325 2330 2335 2340
   ATT CGC AAA AAA TTT CAA GGC TTT ATG CCA GGG GAG ACG ATA CCT TTA 7652
   Ile Arg Lys Lys Phe Gln Gly Phe Met Pro Gly Glu Thr Ile Pro Leu
   2345 2350 2355
   TCT ATT GAA GGG CAA ATT CAA GAA TTG ATC AAA TCT GCT GTC AAC CCA 7700
   Ser Ile Glu Gly Gln Ile Gln Glu Leu Ile Lys Ser Ala Val Asn Pro
   2360 2365 2370
   AAA AAC CTG GTA GAA ATG TAC ATT GGT TGG GCT GCT TAT TTC TAG 7745
   Lys Asn Leu Val Glu Met Tyr Ile Gly Trp Ala Ala Tyr Phe *
   2375 2380 2385
   CATTTTACTA ACAAAAATTT CAATGAACAA GCTACCCATT ATTAAACTTA TGATTTGAAT 7805
   CGAAGATATT TTATTTATTA ATCCGATGAA GAATTCTCGC TGAGTTGTTC AATTTCTTGT 7865
   AATTTTCCTT CCATTTCTAA ATCGTCGATT CGCTTAAATA GGGCACTGGC TTTTTGTGCA 7925
   TTTTTCTCTC GTAAAGCAGC TTCTGATTGA AAAAAAGCTA TATCTGTTTC TGAGTCATCA 7985
   TCCGAATCAA CAATATATTT TGCAGATCGA CCTGCAG 8022
(2) INFORMATION FOR SEQ ID NO: 4:
   (i) SEQUENCE CHARACTERISTICS:
      (A) LENGTH: 2387 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Ser Gln His Ala Lys Arg Lys Ala Gly Ser Leu Asp Leu Ser Pro
    1               5                  10                  15
   Arg Gly Leu Asp Asp Arg Gln Ala Phe Gly Gln Leu Leu Lys Glu Val
               20                  25                  30
   Leu Ala Leu Asp Lys Glu His Glu Leu Gly Arg Ser Asn Ser Leu Pro
           35                  40                  45
   Ser Met Thr Ser Glu Leu Val Glu Val Leu Ile Glu Val Gly Leu Leu
       50                  55                  60
   Ala Phe Lys His Asp Asp Ser Lys Ser Glu Phe Ile Ser Pro Lys Met
   65                  70                  75                  80
   Leu Lys Glu Ala His Leu Ser Leu Gln Ala Leu Met Leu Ile Leu Lys
                   85                  90                  95
   Arg Ser Pro Thr Val Leu Arg Glu Ile Lys Ser Ser Val Thr Leu Leu
               100                 105                 110
   Asp Trp Ile Leu Pro Arg Thr Ile Ser Leu Phe Ala Asp Ile Arg Phe
           115                 120                 125
   Ile Lys Leu Phe Asp Ser Leu Lys Glu Phe His Lys Leu Ile Tyr Gln
       130                 135                 140
   Leu Ile Ser Glu Lys Ser Phe Leu Trp Asp Leu Tyr Ala Ser Phe Met
   145                 150                 155                 160
   Arg Tyr Trp Lys Tyr Tyr Ile Thr Asn Val Ser Ser Ile Val Leu Gln
   165 170 175
   Ile Thr Asn Ala Thr Phe Pro Tyr Lys Met Pro Ser Pro Asn Ser Gln
   180 185 190
   Pro Leu Gln Ser Ile Ser Pro Asn Tyr Pro Thr His Arg Glu Asp Lys
   195 200 205
   Phe Asp Leu Leu Ile Ile Asn Ile Glu Glu Ala Cys Thr Phe Phe Phe
   210 215 220
   Glu Ser Ala His Phe Phe Ala Gln Cys Ser Tyr Leu Lys Lys Ser Asn
   225 230 235 240
   Phe Pro Ser Pro Pro Leu Phe Thr Ala Trp Thr Trp Ile Lys Pro Cys
   245 250 255
   Phe Phe Asn Phe Val Ile Leu Leu Lys Arg Ile Ser Ile Gly Asp Ser
   260 265 270
   Gln Leu Phe Leu His Leu His Ser Arg Ile Val Gln Thr Leu Cys Cys
   275 280 285
   Phe Ser Leu Asn Phe Ile Tyr His Gly Leu Pro Ile Cys Glu Lys Ser
   290 295 300
   Lys His Ile Leu Met Ser Ser Ile Asn Leu Thr Leu Gly Ser Leu Lys
   305 310 315 320
   Lys Thr Tyr Thr Val Ala Asn Thr Ala Ile Ser Leu Phe Phe Leu Ser
   325 330 335
   Leu Phe Val Leu Pro Lys Thr Val Ala Gly Leu Phe Tyr Pro Phe Gly
   340 345 350
   Val Ser Leu Leu Ser Asp Phe Lys Val Leu Glu Gln Leu Glu Pro Asp
   355 360 365
   Ser Asp Leu Lys Lys Ala Ile Ile Leu Phe Lys Cys Arg Tyr Gln Ser
   370 375 380
   Ser Glu Ile Asp Gln Thr Thr Leu Arg Ala Phe Gly Glu Ile Cys Thr
   385 390 395 400
   Gly Lys Leu Glu Asn Thr Leu Phe Ser Asn Ser Glu Leu Asn Leu Phe
   405 410 415
   Leu Leu His Tyr Leu Ser Leu Asp Asn Asp Leu Ser Asn Ile Leu Lys
   420 425 430
   Val Asp Phe Gln Asn Gly His Asn Ile Cys Thr Phe Ala Lys Trp Cys
   435 440 445
   Ile Asn Asn Asn Leu Asp Glu Pro Ser Asn Leu Lys His Phe Arg Glu
   450 455 460
   Met Leu Asp Tyr Tyr Ser Ser His Asn Val Thr Ile Ser Glu Asp Asp
   465 470 475 480
   Leu Lys Asn Phe Ser Leu Val Leu Cys Thr His Val Ala Lys Val Asn
   485 490 495
   Glu Lys Thr Asn Ser Ile Phe Arg Thr Tyr Glu Val His Gly Cys Glu
   500 505 510
   Val Cys Asn Ser Phe Cys Leu Leu Phe Asp Glu Arg Ser Pro Phe Lys
   515 520 525
   Ile Pro Tyr His Glu Leu Phe Cys Ala Leu Leu Lys Asn Pro Asp Ile
   530 535 540
   Ile Ser Ser Ser Val Lys Gln Ser Leu Leu Leu Asp Gly Phe Phe Arg
   545 550 555 560
   Trp Ser Gln His Cys Ser Asn Phe Asn Lys Glu Ser Met Leu Ser Leu
   565 570 575
   Arg Glu Phe Ile Met Lys Ala Leu Ala Ser Thr Ser Arg Cys Leu Arg
   580 585 590
   Val Val Ala Ala Lys Val Leu Pro Ile Phe Ile Lys Gly Pro Asn Asn
   595 600 605
   Leu Asp Ile Val Glu Phe His Lys Glu Ser Lys Ala Leu Ile Phe Asn
   610 615 620
   Thr Leu Lys Ile Leu Ala Val Glu Asn Thr Ala Ile Leu Glu Thr Val
   625 630 635 640
   Ile Leu Ser Trp Ile Ser Leu Ser Arg Val Val Glu Glu Glu Glu Leu
   645 650 655
   His Phe Val Leu Leu Glu Val Ile Ser Ser Val Ile Asn Ser Gly Ile
   660 665 670
   Phe Tyr Gln Gly Ile Gly Leu Ser Ala Leu Gln Gln Ile Ala Ser Thr
   675 680 685
   Arg His Ile Ser Val Trp Gln Leu Leu Ser Pro Tyr Trp Pro Thr Val
   690 695 700
   Ser Val Ala Ile Val Gln Gly Met Gly Lys Lys Pro Asn Ile Ala Ser
   705 710 715 720
   Leu Phe Ala Gln Leu Met Asn Ile Ser Glu Gly Asp Phe Leu Ile Arg
   725 730 735
   Thr Gln Ala Tyr Thr Leu Pro Phe Leu Val Leu Thr Lys Asn Lys Ala
   740 745 750
   Leu Ile Val Arg Ile Ala Glu Leu Ser Gln Ser Asp Val Ala Thr Leu
   755 760 765
   Cys Leu Thr Asn Met His Lys Ile Leu Ala Ser Leu Leu Thr Thr Asp
   770 775 780
   His Pro Asn Leu Glu Glu Ser Val Met Leu Leu Leu Ser Leu Ala Thr
   785 790 795 800
   Ser Asp Phe Glu Lys Val Asp Leu Thr Ser Leu Leu Arg Ser Asp Pro
   805 810 815
   Ile Ser Ile Thr Val Glu Leu Leu Gln Leu Tyr Gln Asn Asp Val Pro
   820 825 830
   His Glu Lys Ile Glu Asn Ala Leu Arg Lys Val Ala Met Ile Val Ser
   835 840 845
   Gln Val Val Asn Asp Glu Asp Leu Ser Asn Lys Glu Leu Leu Tyr Asp
   850 855 860
   Phe Phe Asn Asn His Ile Leu Gly Ile Leu Ala Glu Phe Ser Asn Ile
   865 870 875 880
   Leu Asn Asp Leu Lys Gly Lys Thr Ser Ile Asn Glu Lys Ile Lys Thr
   885 890 895
   Ile Val Gly Ile Glu Lys Met Leu Ser Leu Cys Gly Gly Ala Val Lys
   900 905 910
   Leu Gly Leu Pro Gln Ile Leu Ser Asn Leu Gln Ser Ala Phe Gln Asn
   915 920 925
   Glu His Leu Arg Phe Tyr Ala Ile Lys Ala Trp Phe Ser Leu Ile Leu
   930 935 940
   Ala Thr Lys Glu Pro Glu Tyr Ser Ser Ile Ala Gly Leu Ser Leu Val
   945 950 955 960
   Ile Leu Pro Pro Leu Phe Pro Tyr Leu Glu Pro Gln Glu Ala Glu Leu
   965 970 975
   Val Ile Gln Ile Phe Asp Phe Ile Ser Ser Asp Thr His Lys Cys Leu
   980 985 990
   Gln Gly Leu Lys Trp Ala Ile Pro Thr Ser Leu Asp Ser Ala Cys Phe
   995 1000 1005
   Ser Leu Lys Ala Lys Glu Ile Phe Cys Ser Leu Gln Asn Glu Asp Phe
   1010 1015 1020
   Tyr Ser Glu Leu Gln Ser Ile Ile Lys Cys Leu Thr Asn Glu Asn Glu
   1025 1030 1035 1040
   Pro Val Cys Tyr Leu Gly Leu Gln Lys Leu Glu Leu Phe Phe Gln Ala
   1045 1050 1055
   Lys Val Asp Glu Leu His Asp Thr Leu Asn Leu Asp Ile Ser Asn Glu
   1060 1065 1070
   Val Leu Asp Gln Leu Leu Arg Cys Leu Leu Asp Cys Cys Val Lys Tyr
   1075 1080 1085
   Ala Ser Thr Asn Met Gln Ile Ser Tyr Leu Ala Ala Lys Asn Leu Gly
   1090 1095 1100
   Glu Leu Gly Ala Ile Asp Pro Ser Arg Ala Lys Ala Gln His Ile Ile
   1105 1110 1115 1120
   Lys Glu Thr Val Val Leu Asp Asn Phe Glu Asn Gly Glu Glu Ser Leu
   1125 1130 1135
   Lys Phe Ile Leu Asp Phe Met Gln Ser Gln Leu Ile Pro Ala Phe Leu
   1140 1145 1150
   Val Thr Thr Asp Thr Lys Ala Gln Gly Phe Leu Ala Tyr Ala Leu Gln
   1155 1160 1165
   Glu Phe Leu Lys Leu Gly Gly Phe Lys Ser Ala Val Ile Asn Lys Lys
   1170 1175 1180
   Lys Gly Leu Thr Val Val Thr Glu His Trp Met Ser Leu Pro Asp Leu
   1185 1190 1195 1200
   Ser Lys Arg Val Leu Ile Pro Phe Leu Thr Ser Lys Tyr His Leu Thr
   1205 1210 1215
   Pro Ile Pro Lys Ile Asp Ile Arg Tyr Pro Ile Tyr Lys Glu Asn Val
   1220 1225 1230
   Thr Ile His Thr Trp Met Gln Leu Phe Ser Leu Lys Leu Met Glu Tyr
   1235 1240 1245
   Ala His Ser Gln Asn Ala Glu Lys Ile Phe Gly Ile Cys Ser Lys Val
   1250 1255 1260
   Val Lys Asp Gln Glu Val Asn Ile Pro Cys Phe Leu Leu Pro Phe Leu
   1265 1270 1275 1280
   Val Leu Asn Val Ile Leu Thr Glu Ser Glu Leu Glu Val Asn Lys Val
   1285 1290 1295
   Ile Glu Glu Phe Gln Leu Val Ile Asn Gln Pro Gly Pro Asp Gly Leu
   1300 1305 1310
   Asn Ser Val Gly Gln Gln Arg Tyr Thr Ser Phe Val Asp Val Phe Phe
   1315 1320 1325
   Lys Ile Val Asp Tyr Leu Asn Lys Trp Leu Arg Met Arg Lys Lys Arg
   1330 1335 1340
   Asn Trp Asp Arg Arg Ser Ala Ile Ala Arg Lys Glu Asn Arg Tyr Met
   1345 1350 1355 1360
   Ser Val Glu Asp Ala Thr Ser Arg Glu Ser Ser Ile Ser Lys Val Glu
   1365 1370 1375
   Ser Phe Leu Ser Arg Phe Pro Ser Lys Thr Leu Gly Ile Val Ser Leu
   1380 1385 1390
   Asn Cys Gly Phe His Ala Arg Ala Leu Phe Tyr Trp Glu Gln His Ile
   1395 1400 1405
   Arg Asn Ala Thr Ala Pro Tyr Ala Ala Leu Glu Ser Asp Tyr Arg Val
   1410 1415 1420
   Leu Gln Glu Ile Tyr Ala Gly Ile Asp Asp Pro Asp Glu Ile Glu Ala
   1425 1430 1435 1440
   Val Ser Leu Asn Phe His Asp Tyr Ser Phe Asp Gln Gln Leu Leu Leu
   1445 1450 1455
   His Glu Asn Ser Gly Thr Trp Asp Ser Ala Leu Ser Cys Tyr Glu Ile
   1460 1465 1470
   Ile Ile Gln Lys Asp Pro Glu Asn Lys Lys Ala Lys Ile Gly Leu Leu
   1475 1480 1485
   Asn Ser Met Leu Gln Ser Gly His Tyr Glu Ser Leu Val Leu Ser Leu
   1490 1495 1500
   Asp Ser Phe Ile Ile Asn Asp Asn His Glu Tyr Ser Lys Met Leu Asn
   1505 1510 1515 1520
   Leu Gly Ile Glu Ala Ser Trp Arg Ser Leu Ser Ile Asp Ser Leu Lys
   1525 1530 1535
   Lys Cys Leu Ser Lys Ser Asn Leu Glu Ser Phe Glu Ala Lys Leu Gly
   1540 1545 1550
   Ser Ile Phe Tyr Gln Tyr Leu Arg Lys Asp Ser Phe Ala Glu Leu Thr
   1555 1560 1565
   Glu Arg Leu Gln Pro Leu Tyr Val Asp Ala Ala Thr Ala Ile Ala Asn
   1570 1575 1580
   Thr Gly Ala His Ser Ala Tyr Asp Cys Tyr Asp Ile Leu Ser Lys Leu
   1585 1590 1595 1600
   His Ala Ile Asn Asp Phe Ser Arg Ile Ala Glu Thr Asp Gly Ile Val
   1605 1610 1615
   Ser Asp Asn Leu Asp Ile Val Leu Arg Arg Arg Leu Ser Gln Val Ala
   1620 1625 1630
   Pro Tyr Gly Lys Phe Lys His Gln Ile Leu Ser Thr His Leu Val Gly
   1635 1640 1645
   Tyr Glu Lys Phe Glu Asn Thr Lys Lys Thr Ala Glu Ile Tyr Leu Glu
   1650 1655 1660
   Ile Ala Arg Ile Ser Arg Lys Asn Gly Gln Phe Gln Arg Ala Phe Asn
   1665 1670 1675 1680
   Ala Ile Leu Lys Ala Met Asp Leu Asp Lys Pro Leu Ala Thr Ile Glu
   1685 1690 1695
   His Ala Gln Trp Trp Trp His Gln Gly Gln His Arg Lys Ala Ile Ser
   1700 1705 1710
   Glu Leu Asn Phe Ser Leu Asn Asn Asn Met Phe Asp Leu Val Asp Glu
   1715 1720 1725
   His Glu Glu Arg Pro Lys Asn Arg Lys Glu Thr Leu Gly Asn Pro Leu
   1730 1735 1740
   Lys Gly Lys Val Phe Leu Lys Leu Thr Lys Trp Leu Gly Lys Ala Gly
   1745 1750 1755 1760
   Gln Leu Gly Leu Lys Asp Leu Glu Thr Tyr Tyr His Lys Ala Val Glu
   1765 1770 1775
   Ile Tyr Ser Glu Cys Glu Asn Thr His Tyr Tyr Leu Gly His His Arg
   1780 1785 1790
   Val Leu Met Tyr Glu Glu Glu Gln Lys Leu Pro Val Asn Glu Gln Ser
   1795 1800 1805
   Glu Arg Phe Leu Ser Gly Glu Leu Val Thr Arg Ile Ile Asn Glu Phe
   1810 1815 1820
   Gly Arg Ser Leu Tyr Tyr Gly Thr Asn His Ile Tyr Glu Ser Met Pro
   1825 1830 1835 1840
   Lys Leu Leu Thr Leu Trp Leu Asp Phe Gly Ala Glu Glu Leu Arg Leu
   1845 1850 1855
   Ser Lys Asp Asp Gly Glu Lys Tyr Phe Arg Glu His Ile Ile Ser Ser
   1860 1865 1870
   Arg Lys Lys Ser Leu Glu Leu Met Asn Ser Asn Val Cys Arg Leu Ser
   1875 1880 1885
   Met Lys Ile Pro Gln Tyr Phe Phe Leu Val Ala Leu Ser Gln Met Ile
   1890 1895 1900
   Ser Arg Val Cys His Pro Asn Asn Lys Val Tyr Lys Ile Leu Glu His
   1905 1910 1915 1920
   Ile Ile Ala Asn Val Val Ala Ser Tyr Pro Gly Glu Thr Leu Trp Gln
   1925 1930 1935
   Leu Met Ala Thr Ile Lys Ser Thr Ser Gln Lys Arg Ser Leu Arg Gly
   1940 1945 1950
   Lys Ser Ile Leu Asn Val Leu His Ser Arg Lys Leu Ser Met Ser Ser
   1955 1960 1965
   Lys Val Asp Ile Lys Ala Leu Ser Gln Ser Ala Ile Leu Ile Thr Glu
   1970 1975 1980
   Lys Leu Ile Asn Leu Cys Asn Thr Arg Ile Asn Ser Lys Ser Val Lys
   1985 1990 1995 2000
   Met Ser Leu Lys Asp His Phe Arg Leu Ser Phe Asp Asp Pro Val Asp
   2005 2010 2015
   Leu Val Ile Pro Ala Lys Ser Phe Leu Asp Ile Thr Leu Pro Ala Lys
   2020 2025 2030
   Asp Ala Asn Arg Ala Ser His Tyr Pro Phe Pro Lys Thr Gln Pro Thr
   2035 2040 2045
   Leu Leu Lys Phe Glu Asp Glu Val Asp Ile Met Asn Ser Leu Gln Lys
   2050 2055 2060
   Pro Arg Lys Val Tyr Val Arg Gly Thr Asp Gly Asn Leu Tyr Pro Phe
   2065 2070 2075 2080
   Leu Cys Lys Pro Lys Asp Asp Leu Arg Lys Asp Ala Arg Leu Met Glu
                   2085                2090                2095
   Phe Asn Asn Leu Ile Cys Lys Ile Leu Arg Lys Asp Gln Glu Ala Asn
               2100                2105                2110
   Arg Arg Asn Leu Cys Ile Arg Thr Tyr Val Val Ile Pro Leu Asn Glu
           2115                2120                2125
   Glu Cys Gly Phe Ile Glu Trp Val Asn His Thr Arg Pro Phe Arg Glu
       2130                2135                2140
   Ile Leu Leu Lys Ser Tyr Arg Gln Lys Asn Ile Pro Ile Ser Tyr Gln
   2145                2150                2155                2160
   Glu Ile Lys Val Asp Leu Asp Phe Ala Leu Arg Ser Pro Asn Pro Gly
                   2165                2170                2175
   Asp Ile Phe Glu Lys Lys Ile Leu Pro Lys Phe Pro Pro Val Phe Tyr
               2180                2185                2190
   Glu Trp Phe Val Glu Ser Phe Pro Glu Pro Asn Asn Trp Val Thr Ser
           2195                2200                2205
   Arg Gln Asn Tyr Cys Arg Thr Leu Ala Val Met Ser Ile Val Gly Tyr
       2210                2215                2220
   Val Leu Gly Leu Gly Asp Arg His Gly Glu Asn Ile Leu Phe Asp Glu
   2225                2230                2235                2240
   Phe Thr Gly Glu Ala Ile His Val Asp Phe Asn Cys Leu Phe Asp Lys
                   2245                2250                2255
   Gly Leu Thr Phe Glu Lys Pro Glu Lys Val Pro Phe Arg Leu Thr His
               2260                2265                2270
   Asn Met Val Asp Ala Met Gly Pro Thr Gly Tyr Glu Gly Gly Phe Arg
           2275                2280                2285
   Lys Ala Ser Glu Ile Thr Met Arg Leu Leu Arg Ser Asn Gln Asp Thr
       2290                2295                2300
   Leu Met Ser Val Leu Glu Ser Phe Leu His Asp Pro Leu Val Glu Trp
   2305                2310                2315                2320
   Asn Arg Lys Lys Ser Ser Ser Lys Tyr Pro Asn Asn Glu Ala Asn Glu
                   2325                2330                2335
   Val Leu Asp Ile Ile Arg Lys Lys Phe Gln Gly Phe Met Pro Gly Glu
               2340                2345                2350
   Thr Ile Pro Leu Ser Ile Glu Gly Gln Ile Gln Glu Leu Ile Lys Ser
           2355                2360                2365
   Ala Val Asn Pro Lys Asn Leu Val Glu Met Tyr Ile Gly Trp Ala Ala
       2370                2375                2380
   Tyr Phe  *
   2385